# EUROPEAN PATENT SPECIFICATION

(11) **EP 1 248 803 B1**
(45) Date of publication and mention of the grant of the patent: **05.05.2010**
(21) Application number: 01942367.2
(22) Date of filing: 12.01.2001
(51) Int. Cl.: C07K 14/71, C07K 14/47, C12N 15/63, C07K 19/00, C07K 16/18, A01K 67/027, G01N 33/53, A61P 25/00

(54) **NOGO RECEPTOR-MEDIATED BLOCKADE OF AXONAL GROWTH**
NOGO REZEPTOR-VERMITTELTE BLOCKADE DES AXONALEN WACHSTUMS
BLOCAGE DE LA CROISSANCE AXONALE A MEDIATION ASSUREE PAR LE RECEPTEUR DE NOGO

(30) Priority: 12.01.2000 US 175707 P; 26.05.2000 US 207366 P; 29.09.2000 US 236378 P
(43) Date of publication of application: 16.10.2002
(60) Divisional application: 09170776.0
(73) Proprietor: Yale University, New Haven, CT 06511 (US)
(72) Inventor: STRITTMATTER, Stephen, M., New Haven, CT 06520 (US)
(74) Representative: Adams, Harvey Vaughan John
(86) International application number: PCT/US2001/001041
(87) International publication number: WO 2001/051520

(56) References cited:
- WO-A-00/31235
- WO-A-00/32221
- WO-A-00/37638
- WO-A-00/53756
- WO-A-00/53758
- WO-A-00/58473
- WO-A-00/70050
- WO-A-00/73452
- WO-A-01/09162
- WO-A-99/46281
- WO-A-99/66041
- HU P. ET AL.: "Homo sapiens Chromosome 22q11 PAC Clone p215k21 Distal To DGCR Region" EMBL DATABASE ENTRY AC006549; ACCESSION NO. AC006549, 11 February 1999 (1999-02-11), XP002171167
- SPILLMANN ET AL: "Identification and Characterization of a Bovine Neurite Growth Inhibitor (bNI-220)" JOURNAL OF BIOLOGICAL CHEMISTRY, vol. 273, no. 30, 24 July 1998 (1998-07-24), pages 19283-19293, XP002162896 ISSN: 0021-9258
- FOURNIER A. ET AL.: "Identification of a receptor mediating Nogo-66 inhibition of axonal regeneration" NATURE, vol. 409, no. 6818, 18 January 2001 (2001-01-18), pages 341-346, XP000926532
- HUBER A.B. ET AL.: "Nogo-A, a Potent Inhibitor of Neurite Outgrowth and Regeneration" BIOLOGICAL CHEMISTRY, vol. 381, May 2000 (2000-05), pages 407-419, XP000926531

### SEQUENCE LISTING

<110> Strittmatter, Stephen M.
<120> Nogo Receptor-Mediated Blockade of Axonal Growth
<130> 44574-5073-WO
<140>
   <141>
<150> US 60/175,707
   <151> 2000-01-12
<150> US 60/207,366
   <151> 2000-05-26
<150> US 60/236,378
   <151> 2000-09-29
<160> 20
<170> PatentIn Ver. 2.1
<210> 1
   <211> 1719
   <212> DNA
   <213> Homo sapiens
<220>
   <221> CDS
   <222> (166)..(1584)
   <223> Predicted human Nogo receptor gene
<400> 1
   agcccagcca gagccgggcg gagcggagcg cgccgagcct cgtcccgcgg ccgggccggg 60
   gccgggccgt agcggcggcg cctggatgcg gacccggccg cggggagacg ggcgcccgcc 120
   ccgaaacgac tttcagtccc cgacgcgccc cgcccaaccc ctacg atg aag agg gcg 177
                                                    Met Lys Arg Ala
                                                    1
   tcc gct gga ggg agc cgg ctg ctg gca tgg gtg ctg tgg ctg cag gcc   225
   Ser Ala Gly Gly Ser Arg Leu Leu Ala Trp Val Leu Trp Leu Gln Ala
   5                   10                  15                  20
   tgg cag gtg gca gcc cca tgc cca ggt gcc tgc gta tgc tac aat gag   273
   Trp Gln Val Ala Ala Pro Cys Pro Gly Ala Cys Val Cys Tyr Asn Glu
                   25                  30                  35
   ccc aag gtg acg aca agc tgc ccc cag cag ggc ctg cag gct gtg ccc   321
   Pro Lys Val Thr Thr Ser Cys Pro Gln Gln Gly Leu Gln Ala Val Pro
               40                  45                  50
   gtg ggc atc cct gct gcc agc cag cgc atc ttc ctg cac ggc aac cgc   369
   Val Gly Ile Pro Ala Ala Ser Gln Arg Ile Phe Leu His Gly Asn Arg
           55                  60                  65
   atc tcg cat gtg cca gct gcc agc ttc cgt gcc tgc cgc aac ctc acc     417
   Ile Ser His Val Pro Ala Ala Ser Phe Arg Ala Cys Arg Asn Leu Thr
       70                  75                  80
   atc ctg tgg ctg cac tcg aat gtg ctg gcc cga att gat gcg gct gcc     465
   Ile Leu Trp Leu His Ser Asn Val Leu Ala Arg Ile Asp Ala Ala Ala
   85                  90                  95                  100
   ttc act ggc ctg gcc ctc ctg gag cag ctg gac ctc agc gat aat gca     513
   Phe Thr Gly Leu Ala Leu Leu Glu Gln Leu Asp Leu Ser Asp Asn Ala
                   105                 110                 115
   cag ctc cgg tct gtg gac cct gcc aca ttc cac ggc ctg ggc cgc cta     561
   Gln Leu Arg Ser Val Asp Pro Ala Thr Phe His Gly Leu Gly Arg Leu
               120                 125                 130
   cac acg ctg cac ctg gac cgc tgc ggc ctg cag gag ctg ggc ccg ggg     609
   His Thr Leu His Leu Asp Arg Cys Gly Leu Gln Glu Leu Gly Pro Gly
           135                 140                 145
   ctg ttc cgc ggc ctg gct gcc ctg cag tac ctc tac ctg cag gac aac     657
   Leu Phe Arg Gly Leu Ala Ala Leu Gln Tyr Leu Tyr Leu Gln Asp Asn
       150                 155                 160
   gcg ctg cag gca ctg cct gat gac acc ttc cgc gac ctg ggc aac ctc     705
   Ala Leu Gln Ala Leu Pro Asp Asp Thr Phe Arg Asp Leu Gly Asn Leu
   165                 170                 175                 180
   aca cac ctc ttc ctg cac ggc aac cgc atc tcc agc gtg ccc gag cgc     753
   Thr His Leu Phe Leu His Gly Asn Arg Ile Ser Ser Val Pro Glu Arg
                   185                 190                 195
   gcc ttc cgt ggg ctg cac agc ctc gac cgt ctc cta ctg cac cag aac     801
   Ala Phe Arg Gly Leu His Ser Leu Asp Arg Leu Leu Leu His Gln Asn
               200                 205                 210
   cgc gtg gcc cat gtg cac ccg cat gcc ttc cgt gac ctt ggc cgc ctc     849
   Arg Val Ala His Val His Pro His Ala Phe Arg Asp Leu Gly Arg Leu
           215                 220                 225
   atg aca ctc tat ctg ttt gcc aac aat cta tca gcg ctg ccc act gag     897
   Met Thr Leu Tyr Leu Phe Ala Asn Asn Leu Ser Ala Leu Pro Thr Glu
       230                 235                 240
   gcc ctg gcc ccc ctg cgt gcc ctg cag tac ctg agg ctc aac gac aac     945
   Ala Leu Ala Pro Leu Arg Ala Leu Gln Tyr Leu Arg Leu Asn Asp Asn
   245                 250                 255                 260
   ccc tgg gtg tgt gac tgc cgg gca cgc cca ctc tgg gcc tgg ctg cag     993
   Pro Trp Val Cys Asp Cys Arg Ala Arg Pro Leu Trp Ala Trp Leu Gln
                   265                 270                 275
   aag ttc cgc ggc tcc tcc tcc gag gtg ccc tgc agc ctc ccg caa cgc     1041
   Lys Phe Arg Gly Ser Ser Ser Glu Val Pro Cys Ser Leu Pro Gln Arg
               280                 285                 290
   ctg gct ggc cgt gac ctc aaa cgc cta gct gcc aat gac ctg cag ggc    1089
   Leu Ala Gly Arg Asp Leu Lys Arg Leu Ala Ala Asn Asp Leu Gln Gly
           295                 300                 305
   tgc gct gtg gcc acc ggc cct tac cat ccc atc tgg acc ggc agg gcc    1137
   Cys Ala Val Ala Thr Gly Pro Tyr His Pro Ile Trp Thr Gly Arg Ala
       310                 315                 320
   acc gat gag gag ccg ctg ggg ctt ccc aag tgc tgc cag cca gat gcc    1185
   Thr Asp Glu Glu Pro Leu Gly Leu Pro Lys Cys Cys Gln Pro Asp Ala
   325                 330                 335                 340
   gct gac aag gcc tca gta ctg gag cct gga aga cca gct tcg gca ggc    1233
   Ala Asp Lys Ala Ser Val Leu Glu Pro Gly Arg Pro Ala Ser Ala Gly
                   345                 350                 355
   aat gcg ctg aag gga cgc gtg ccg ccc ggt gac agc ccg ccg ggc aac    1281
   Asn Ala Leu Lys Gly Arg Val Pro Pro Gly Asp Ser Pro Pro Gly Asn
               360                 365                 370
   ggc tct ggc cca cgg cac atc aat gac tca ccc ttt ggg act ctg cct    1329
   Gly Ser Gly Pro Arg His Ile Asn Asp Ser Pro Phe Gly Thr Leu Pro
           375                 380                 385
   ggc tct gct gag ccc ccg ctc act gca gtg cgg ccc gag ggc tcc gag    1377
   Gly Ser Ala Glu Pro Pro Leu Thr Ala Val Arg Pro Glu Gly Ser Glu
       390                 395                 400
   cca cca ggg ttc ccc acc tcg ggc cct cgc cgg agg cca ggc tgt tca    1425
   Pro Pro Gly Phe Pro Thr Ser Gly Pro Arg Arg Arg Pro Gly Cys Ser
   405                 410                 415                 420
   cgc aag aac cgc acc cgc agc cac tgc cgt ctg ggc cag gca ggc agc    1473
   Arg Lys Asn Arg Thr Arg Ser His Cys Arg Leu Gly Gln Ala Gly Ser
                   425                 430                 435
   ggg ggt ggc ggg act ggt gac tca gaa ggc tca ggt gcc cta ccc agc    1521
   Gly Gly Gly Gly Thr Gly Asp Ser Glu Gly Ser Gly Ala Leu Pro Ser
               440                 445                 450
   ctc acc tgc agc ctc acc ccc ctg ggc ctg gcg ctg gtg ctg tgg aca    1569
   Leu Thr Cys Ser Leu Thr Pro Leu Gly Leu Ala Leu Val Leu Trp Thr
           455                 460                 465
   gtg ctt ggg ccc tgc tgacccccag cggacacaag agcgtgctca gcagccaggt   1624
   Val Leu Gly Pro Cys
       470
   gtgtgtacat acggggtctc tctccacgcc gccaagccag ccgggcggcc gacccgtggg 1684
   gcaggccagg ccaggtcctc cctgatggac gcctg                            1719
<210> 2
   <211> 473
   <212> PRT
   <213> Homo sapiens
<400> 2
   Met Lys Arg Ala Ser Ala Gly Gly Ser Arg Leu Leu Ala Trp Val Leu
    1               5                  10                  15
   Trp Leu Gln Ala Trp Gln Val Ala Ala Pro Cys Pro Gly Ala Cys Val
               20                  25                  30
   Cys Tyr Asn Glu Pro Lys Val Thr Thr Ser Cys Pro Gln Gln Gly Leu
           35                  40                  45
   Gln Ala Val Pro Val Gly Ile Pro Ala Ala Ser Gln Arg Ile Phe Leu
       50                  55                  60
   His Gly Asn Arg Ile Ser His Val Pro Ala Ala Ser Phe Arg Ala Cys
   65                  70                  75                  80
   Arg Asn Leu Thr Ile Leu Trp Leu His Ser Asn Val Leu Ala Arg Ile
                   85                  90                  95
   Asp Ala Ala Ala Phe Thr Gly Leu Ala Leu Leu Glu Gln Leu Asp Leu
               100                 105                 110
   Ser Asp Asn Ala Gln Leu Arg Ser Val Asp Pro Ala Thr Phe His Gly
           115                 120                 125
   Leu Gly Arg Leu His Thr Leu His Leu Asp Arg Cys Gly Leu Gln Glu
       130                 135                 140
   Leu Gly Pro Gly Leu Phe Arg Gly Leu Ala Ala Leu Gln Tyr Leu Tyr
   145                 150                 155                 160
   Leu Gln Asp Asn Ala Leu Gln Ala Leu Pro Asp Asp Thr Phe Arg Asp
                   165                 170                 175
   Leu Gly Asn Leu Thr His Leu Phe Leu His Gly Asn Arg Ile Ser Ser
               180                 185                 190
   Val Pro Glu Arg Ala Phe Arg Gly Leu His Ser Leu Asp Arg Leu Leu
           195                 200                 205
   Leu His Gln Asn Arg Val Ala His Val His Pro His Ala Phe Arg Asp
       210                 215                 220
   Leu Gly Arg Leu Met Thr Leu Tyr Leu Phe Ala Asn Asn Leu Ser Ala
   225                 230                 235                 240
   Leu Pro Thr Glu Ala Leu Ala Pro Leu Arg Ala Leu Gln Tyr Leu Arg
                   245                 250                 255
   Leu Asn Asp Asn Pro Trp Val Cys Asp Cys Arg Ala Arg Pro Leu Trp
               260                 265                 270
   Ala Trp Leu Gln Lys Phe Arg Gly Ser Ser Ser Glu Val Pro Cys Ser
   275 280 285
   Leu Pro Gln Arg Leu Ala Gly Arg Asp Leu Lys Arg Leu Ala Ala Asn
   290 295 300
   Asp Leu Gln Gly Cys Ala Val Ala Thr Gly Pro Tyr His Pro Ile Trp
   305 310 315 320
   Thr Gly Arg Ala Thr Asp Glu Glu Pro Leu Gly Leu Pro Lys Cys Cys
   325 330 335
   Gln Pro Asp Ala Ala Asp Lys Ala Ser Val Leu Glu Pro Gly Arg Pro
   340 345 350
   Ala Ser Ala Gly Asn Ala Leu Lys Gly Arg Val Pro Pro Gly Asp Ser
   355 360 365
   Pro Pro Gly Asn Gly Ser Gly Pro Arg His Ile Asn Asp Ser Pro Phe
   370 375 380
   Gly Thr Leu Pro Gly Ser Ala Glu Pro Pro Leu Thr Ala Val Arg Pro
   385 390 395 400
   Glu Gly Ser Glu Pro Pro Gly Phe Pro Thr Ser Gly Pro Arg Arg Arg
   405 410 415
   Pro Gly Cys Ser Arg Lys Asn Arg Thr Arg Ser His Cys Arg Leu Gly
   420 425 430
   Gln Ala Gly Ser Gly Gly Gly Gly Thr Gly Asp Ser Glu Gly Ser Gly
   435 440 445
   Ala Leu Pro Ser Leu Thr Cys Ser Leu Thr Pro Leu Gly Leu Ala Leu
   450 455 460
   Val Leu Trp Thr Val Leu Gly Pro Cys
   465 470
<210> 3
   <211> 1866
   <212> DNA
   <213> Mus musculus
<220>
   <221> CDS
   <222> (178)..(1596)
   <223> Mouse Nogo receptor cDNA
<400> 3
   agccgcagcc cgcgagccca gcccggcccg gtagagcgga gcgccggagc ctcgtcccgc 60
   ggccgggccg ggaccgggcc ggagcagcgg cgcctggatg cggacccggc cgcgcgcaga 120
   cgggcgcccg ccccgaagcc gcttccagtg cccgacgcgc cccgctcgac cccgaag     177
   atg aag agg gcg tcc tcc gga gga agc agg ctg ctg gca tgg gtg tta    225
   Met Lys Arg Ala Ser Ser Gly Gly Ser Arg Leu Leu Ala Trp Val Leu
    1               5                  10                  15
   tgg cta cag gcc tgg agg gta gca aca cca tgc cct ggt gct tgt gtg    273
   Trp Leu Gln Ala Trp Arg Val Ala Thr Pro Cys Pro Gly Ala Cys Val
               20                  25                  30
   tgc tac aat gag ccc aag gta aca aca agc tgc ccc cag cag ggt ctg    321
   Cys Tyr Asn Glu Pro Lys Val Thr Thr Ser Cys Pro Gln Gln Gly Leu
           35                  40                  45
   cag gct gtg ccc act ggc atc cca gcc tct agc cag cga atc ttc ctg    369
   Gln Ala Val Pro Thr Gly Ile Pro Ala Ser Ser Gln Arg Ile Phe Leu
       50                  55                  60
   cat ggc aac cga atc tct cac gtg cca gct gcg agc ttc cag tca tgc    417
   His Gly Asn Arg Ile Ser His Val Pro Ala Ala Ser Phe Gln Ser Cys
   65                  70                  75                  80
   cga aat ctc act atc ctg tgg ctg cac tct aat gcg ctg gct cgg atc    465
   Arg Asn Leu Thr Ile Leu Trp Leu His Ser Asn Ala Leu Ala Arg Ile
                   85                  90                  95
   gat gct gct gcc ttc act ggt ctg acc ctc ctg gag caa cta gat ctt    513
   Asp Ala Ala Ala Phe Thr Gly Leu Thr Leu Leu Glu Gln Leu Asp Leu
               100                 105                 110
   agt gat aat gca cag ctt cat gtc gtg gac cct acc acg ttc cac ggc    561
   Ser Asp Asn Ala Gln Leu His Val Val Asp Pro Thr Thr Phe His Gly
           115                 120                 125
   ctg ggc cac ctg cac aca ctg cac cta gac cga tgt ggc ctg cgg gag    609
   Leu Gly His Leu His Thr Leu His Leu Asp Arg Cys Gly Leu Arg Glu
       130                 135                 140
   ctg ggt ccc ggc cta ttc cgt gga cta gca gct ctg cag tac ctc tac    657
   Leu Gly Pro Gly Leu Phe Arg Gly Leu Ala Ala Leu Gln Tyr Leu Tyr
   145                 150                 155                 160
   cta caa gac aac aat ctg cag gca ctc cct gac aac acc ttt cga gac    705
   Leu Gln Asp Asn Asn Leu Gln Ala Leu Pro Asp Asn Thr Phe Arg Asp
                   165                 170                 175
   ctg ggc aac ctc acg cat ctc ttt ctg cat ggc aac cgt atc ccc agt    753
   Leu Gly Asn Leu Thr His Leu Phe Leu His Gly Asn Arg Ile Pro Ser
               180                 185                 190
   gtg cct gag cac gct ttc cgt ggc ctg cac agt ctt gac cgc ctc ctc    801
   Val Pro Glu His Ala Phe Arg Gly Leu His Ser Leu Asp Arg Leu Leu
           195                 200                 205
   ttg cac cag aac cat gtg gct cgt gtg cac cca cat gcc ttc cgg gac    849
   Leu His Gln Asn His Val Ala Arg Val His Pro His Ala Phe Arg Asp
   210                 215                 220
   ctt ggc cgc ctc atg acc ctc tac ctg ttt gcc aac aac ctc tcc atg    897
   Leu Gly Arg Leu Met Thr Leu Tyr Leu Phe Ala Asn Asn Leu Ser Met
   225                 230                 235                 240
   ctg cct gca gag gtc cta atg ccc ctg agg tct ctg cag tac ctg cga    945
   Leu Pro Ala Glu Val Leu Met Pro Leu Arg Ser Leu Gln Tyr Leu Arg
                   245                 250                 255
   ctc aat gac aac ccc tgg gtg tgt gac tgc cgg gca cgt cca ctc tgg    993
   Leu Asn Asp Asn Pro Trp Val Cys Asp Cys Arg Ala Arg Pro Leu Trp
               260                 265                 270
   gcc tgg ctg cag aag ttc cga ggt tcc tca tca gag gtg ccc tgc aac    1041
   Ala Trp Leu Gln Lys Phe Arg Gly Ser Ser Ser Glu Val Pro Cys Asn
           275                 280                 285
   ctg ccc caa cgc ctg gca gac cgt gat ctt aag cgc ctc gct gcc agt    1089
   Leu Pro Gln Arg Leu Ala Asp Arg Asp Leu Lys Arg Leu Ala Ala Ser
       290                 295                 300
   gac cta gag ggc tgt gct gtg gct tca gga ccc ttc cgt ccc atc cag    1137
   Asp Leu Glu Gly Cys Ala Val Ala Ser Gly Pro Phe Arg Pro Ile Gln
   305                 310                 315                 320
   acc agt cag ctc act gat gag gag ctg ctg agc ctc ccc aag tgc tgc    1185
   Thr Ser Gln Leu Thr Asp Glu Glu Leu Leu Ser Leu Pro Lys Cys Cys
                   325                 330                 335
   cag cca gat gct gca gac aaa gcc tca gta ctg gaa ccc ggg agg cca    1233
   Gln Pro Asp Ala Ala Asp Lys Ala Ser Val Leu Glu Pro Gly Arg Pro
               340                 345                 350
   gct tct gcc gga aac gcc ctc aag gga cgt gtg cct ccc ggt gac act    1281
   Ala Ser Ala Gly Asn Ala Leu Lys Gly Arg Val Pro Pro Gly Asp Thr
           355                 360                 365
   cca cca ggc aat ggc tca ggc cct cgg cac atc aat gac tct cca ttt    1329
   Pro Pro Gly Asn Gly Ser Gly Pro Arg His Ile Asn Asp Ser Pro Phe
       370                 375                 380
   gga act ttg ccc agc tct gca gag ccc cca ctg act gcc ctg cgg cct    1377
   Gly Thr Leu Pro Ser Ser Ala Glu Pro Pro Leu Thr Ala Leu Arg Pro
   385                 390                 395                 400
   ggg ggt tcc gag cca cca gga ctt ccc acc act ggt ccc cgc agg agg    1425
   Gly Gly Ser Glu Pro Pro Gly Leu Pro Thr Thr Gly Pro Arg Arg Arg
                   405                 410                 415
   cca ggt tgt tcc cgg aag aat cgc acc cgc agc cac tgc cgt ctg ggc    1473
   Pro Gly Cys Ser Arg Lys Asn Arg Thr Arg Ser His Cys Arg Leu Gly
               420                 425                 430
   cag gcg gga agt ggg gcc agt gga aca ggg gac gca gag ggt tca ggg    1521
   Gln Ala Gly Ser Gly Ala Ser Gly Thr Gly Asp Ala Glu Gly Ser Gly
           435                 440                 445
   gct ctg cct gct ctg gcc tgc agc ctt gct cct ctg ggc ctt gca ctg    1569
   Ala Leu Pro Ala Leu Ala Cys Ser Leu Ala Pro Leu Gly Leu Ala Leu
       450                 455                 460
   gta ctt tgg aca gtg ctt ggg ccc tgc tgaccagcca ccagccacca          1616
   Val Leu Trp Thr Val Leu Gly Pro Cys
   465                 470
   ggtgtgtgta catatggggt ctccctccac gccgccagcc agagccaggg acaggctctg 1676
   aggggcaggc caggccctcc ctgacagatg cctccccacc agcccacccc catctccacc 1736
   ccatcatgtt tacagggttc cgggggtggc ggttggttca caaccccaac ttccacccgg 1796
   atcgcggcat atagacatat gaaatttatt ttacttgcgt aaaatatcgg atgacgtgga 1856
   ataaacagct                                                         1866
<210> 4
   <211> 473
   <212> PRT
   <213> Mus musculus
<400> 4
   Met Lys Arg Ala Ser Ser Gly Gly Ser Arg Leu Leu Ala Trp Val Leu
   1               5                   10                  15
   Trp Leu Gln Ala Trp Arg Val Ala Thr Pro Cys Pro Gly Ala Cys Val
               20                  25                  30
   Cys Tyr Asn Glu Pro Lys Val Thr Thr Ser Cys Pro Gln Gln Gly Leu
           35                  40                  45
   Gln Ala Val Pro Thr Gly Ile Pro Ala Ser Ser Gln Arg Ile Phe Leu
       50                  55                  60
   His Gly Asn Arg Ile Ser His Val Pro Ala Ala Ser Phe Gln Ser Cys
   65                  70                  75                  80
   Arg Asn Leu Thr Ile Leu Trp Leu His Ser Asn Ala Leu Ala Arg Ile
                   85                  90                  95
   Asp Ala Ala Ala Phe Thr Gly Leu Thr Leu Leu Glu Gln Leu Asp Leu
               100                 105                 110
   Ser Asp Asn Ala Gln Leu His Val Val Asp Pro Thr Thr Phe His Gly
           115                 120                 125
   Leu Gly His Leu His Thr Leu His Leu Asp Arg Cys Gly Leu Arg Glu
       130                 135                 140
   Leu Gly Pro Gly Leu Phe Arg Gly Leu Ala Ala Leu Gln Tyr Leu Tyr
   145                 150                 155                 160
   Leu Gln Asp Asn Asn Leu Gln Ala Leu Pro Asp Asn Thr Phe Arg Asp
                   165                 170                 175
   Leu Gly Asn Leu Thr His Leu Phe Leu His Gly Asn Arg Ile Pro Ser
               180                 185                 190
   Val Pro Glu His Ala Phe Arg Gly Leu His Ser Leu Asp Arg Leu Leu
           195                 200                 205
   Leu His Gln Asn His Val Ala Arg Val His Pro His Ala Phe Arg Asp
       210                 215                 220
   Leu Gly Arg Leu Met Thr Leu Tyr Leu Phe Ala Asn Asn Leu Ser Met
   225                 230                 235                 240
   Leu Pro Ala Glu Val Leu Met Pro Leu Arg Ser Leu Gln Tyr Leu Arg
                   245                 250                 255
   Leu Asn Asp Asn Pro Trp Val Cys Asp Cys Arg Ala Arg Pro Leu Trp
               260                 265                 270
   Ala Trp Leu Gln Lys Phe Arg Gly Ser Ser Ser Glu Val Pro Cys Asn
           275                 280                 285
   Leu Pro Gln Arg Leu Ala Asp Arg Asp Leu Lys Arg Leu Ala Ala Ser
       290                 295                 300
   Asp Leu Glu Gly Cys Ala Val Ala Ser Gly Pro Phe Arg Pro Ile Gln
   305                 310                 315                 320
   Thr Ser Gln Leu Thr Asp Glu Glu Leu Leu Ser Leu Pro Lys Cys Cys
                   325                 330                 335
   Gln Pro Asp Ala Ala Asp Lys Ala Ser Val Leu Glu Pro Gly Arg Pro
               340                 345                 350
   Ala Ser Ala Gly Asn Ala Leu Lys Gly Arg Val Pro Pro Gly Asp Thr
           355                 360                 365
   Pro Pro Gly Asn Gly Ser Gly Pro Arg His Ile Asn Asp Ser Pro Phe
       370                 375                 380
   Gly Thr Leu Pro Ser Ser Ala Glu Pro Pro Leu Thr Ala Leu Arg Pro
   385                 390                 395                 400
   Gly Gly Ser Glu Pro Pro Gly Leu Pro Thr Thr Gly Pro Arg Arg Arg
                   405                 410                 415
   Pro Gly Cys Ser Arg Lys Asn Arg Thr Arg Ser His Cys Arg Leu Gly
               420                 425                 430
   Gln Ala Gly Ser Gly Ala Ser Gly Thr Gly Asp Ala Glu Gly Ser Gly
           435                 440                 445
   Ala Leu Pro Ala Leu Ala Cys Ser Leu Ala Pro Leu Gly Leu Ala Leu
       450                 455                 460
   Val Leu Trp Thr Val Leu Gly Pro Cys
   465                 470
<210> 5
   <211> 4053
   <212> DNA
   <213> Homo sapiens
<220>
   <221> CDS
   <222> (135)..(3710)
   <223> Human mRNA for Nogo protein (KIAA0886, GenBank Accession No. AB020693)
<400> 5
   caccacagta ggtccctcgg ctcagtcggc ccagcccctc tcagtcctcc ccaaccccca 60
   caaccgcccg cggctctgag acgcggcccc ggcggcggcg gcagcagctg cagcatcatc 120
   tccaccctcc agcc atg gaa gac ctg gac cag tct cct ctg gtc tcg tcc    170
                   Met Glu Asp Leu Asp Gln Ser Pro Leu Val Ser Ser
                   1               5                   10
   tcg gac agc cca ccc cgg ccg cag ccc gcg ttc aag tac cag ttc gtg    218
   Ser Asp Ser Pro Pro Arg Pro Gln Pro Ala Phe Lys Tyr Gln Phe Val
           15                  20                  25
   agg gag ccc gag gac gag gag gaa gaa gag gag gag gaa gag gag gac    266
   Arg Glu Pro Glu Asp Glu Glu Glu Glu Glu Glu Glu Glu Glu Glu Asp
       30                  35                  40
   gag gac gaa gac ctg gag gag ctg gag gtg ctg gag agg aag ccc gcc    314
   Glu Asp Glu Asp Leu Glu Glu Leu Glu Val Leu Glu Arg Lys Pro Ala
   45                  50                  55                  60
   gcc ggg ctg tcc gcg gcc cca gtg ccc acc gcc cct gcc gcc ggc gcg    362
   Ala Gly Leu Ser Ala Ala Pro Val Pro Thr Ala Pro Ala Ala Gly Ala
                   65                  70                  75
   ccc ctg atg gac ttc gga aat gac ttc gtg ccg ccg gcg ccc cgg gga    410
   Pro Leu Met Asp Phe Gly Asn Asp Phe Val Pro Pro Ala Pro Arg Gly
               80                  85                  90
   ccc ctg ccg gcc gct ccc ccc gtc gcc ccg gag cgg cag ccg tct tgg    458
   Pro Leu Pro Ala Ala Pro Pro Val Ala Pro Glu Arg Gln Pro Ser Trp
           95                  100                 105
   gac ccg agc ccg gtg tcg tcg acc gtg ccc gcg cca tcc ccg ctg tct    506
   Asp Pro Ser Pro Val Ser Ser Thr Val Pro Ala Pro Ser Pro Leu Ser
       110                 115                 120
   gct gcc gca gtc tcg ccc tcc aag ctc cct gag gac gac gag cct ccg    554
   Ala Ala Ala Val Ser Pro Ser Lys Leu Pro Glu Asp Asp Glu Pro Pro
   125                 130                 135                 140
   gcc cgg cct ccc cct cct ccc ccg gcc agc gtg agc ccc cag gca gag    602
   Ala Arg Pro Pro Pro Pro Pro Pro Ala Ser Val Ser Pro Gln Ala Glu
                   145                     150                 155
   ccc gtg tgg acc ccg cca gcc ccg gct ccc gcc gcg ccc ccc tcc acc    650
   Pro Val Trp Thr Pro Pro Ala Pro Ala Pro Ala Ala Pro Pro Ser Thr
               160                 165                 170
   ccg gcc gcg ccc aag cgc agg ggc tcc tcg ggc tca gtg gat gag acc    698
   Pro Ala Ala Pro Lys Arg Arg Gly Ser Ser Gly Ser Val Asp Glu Thr
           175                 180                 185
   ctt ttt gct ctt cct gct gca tct gag cct gtg ata cgc tcc tct gca    746
   Leu Phe Ala Leu Pro Ala Ala Ser Glu Pro Val Ile Arg Ser Ser Ala
       190                 195                 200
   gaa aat atg gac ttg aag gag cag cca ggt aac act att tcg gct ggt    794
   Glu Asn Met Asp Leu Lys Glu Gln Pro Gly Asn Thr Ile Ser Ala Gly
   205                 210                 215                 220
   caa gag gat ttc cca tct gtc ctg ctt gaa act gct gct tct ctt cct    842
   Gln Glu Asp Phe Pro Ser Val Leu Leu Glu Thr Ala Ala Ser Leu Pro
                   225                 230                 235
   tct ctg tct cct ctc tca gcc gct tct ttc aaa gaa cat gaa tac ctt    890
   Ser Leu Ser Pro Leu Ser Ala Ala Ser Phe Lys Glu His Glu Tyr Leu
               240                 245                 250
   ggt aat ttg tca aca gta tta ccc act gaa gga aca ctt caa gaa aat    938
   Gly Asn Leu Ser Thr Val Leu Pro Thr Glu Gly Thr Leu Gln Glu Asn
           255                 260                 265
   gtc agt gaa gct tct aaa gag gtc tca gag aag gca aaa act cta ctc    986
   Val Ser Glu Ala Ser Lys Glu Val Ser Glu Lys Ala Lys Thr Leu Leu
       270                 275                 280
   ata gat aga gat tta aca gag ttt tca gaa tta gaa tac tca gaa atg    1034
   Ile Asp Arg Asp Leu Thr Glu Phe Ser Glu Leu Glu Tyr Ser Glu Met
   285                 290                 295                 300
   gga tca tcg ttc agt gtc tct cca aaa gca gaa tct gcc gta ata gta    1082
   Gly Ser Ser Phe Ser Val Ser Pro Lys Ala Glu Ser Ala Val Ile Val
                   305                 310                 315
   gca aat cct agg gaa gaa ata atc gtg aaa aat aaa gat gaa gaa gag    1130
   Ala Asn Pro Arg Glu Glu Ile Ile Val Lys Asn Lys Asp Glu Glu Glu
               320                 325                 330
   aag tta gtt agt aat aac atc ctt cat aat caa caa gag tta cct aca    1178
   Lys Leu Val Ser Asn Asn Ile Leu His Asn Gln Gln Glu Leu Pro Thr
           335                 340                 345
   gct ctt act aaa ttg gtt aaa gag gat gaa gtt gtg tct tca gaa aaa    1226
   Ala Leu Thr Lys Leu Val Lys Glu Asp Glu Val Val Ser Ser Glu Lys
       350                 355                 360
   gca aaa gac agt ttt aat gaa aag aga gtt gca gtg gaa gct cct atg     1274
   Ala Lys Asp Ser Phe Asn Glu Lys Arg Val Ala Val Glu Ala Pro Met
   365                 370                 375                 380
   agg gag gaa tat gca gac ttc aaa cca ttt gag cga gta tgg gaa gtg     1322
   Arg Glu Glu Tyr Ala Asp Phe Lys Pro Phe Glu Arg Val Trp Glu Val
                   385                 390                 395
   aaa gat agt aag gaa gat agt gat atg ttg gct gct gga ggt aaa atc     1370
   Lys Asp Ser Lys Glu Asp Ser Asp Met Leu Ala Ala Gly Gly Lys Ile
               400                 405                 410
   gag agc aac ttg gaa agt aaa gtg gat aaa aaa tgt ttt gca gat agc     1418
   Glu Ser Asn Leu Glu Ser Lys Val Asp Lys Lys Cys Phe Ala Asp Ser
           415                 420                 425
   ctt gag caa act aat cac gaa aaa gat agt gag agt agt aat gat gat     1466
   Leu Glu Gln Thr Asn His Glu Lys Asp Ser Glu Ser Ser Asn Asp Asp
       430                 435                 440
   act tct ttc ccc agt acg cca gaa ggt ata aag gat cgt tca gga gca     1514
   Thr Ser Phe Pro Ser Thr Pro Glu Gly Ile Lys Asp Arg Ser Gly Ala
   445                 450                 455                 460
   tat atc aca tgt gct ccc ttt aac cca gca gca act gag agc att gca     1562
   Tyr Ile Thr Cys Ala Pro Phe Asn Pro Ala Ala Thr Glu Ser Ile Ala
                   465                 470                 475
   aca aac att ttt cct ttg tta gga gat cct act tca gaa aat aag acc     1610
   Thr Asn Ile Phe Pro Leu Leu Gly Asp Pro Thr Ser Glu Asn Lys Thr
               480                 485                 490
   gat gaa aaa aaa ata gaa gaa aag aag gcc caa ata gta aca gag aag     1658
   Asp Glu Lys Lys Ile Glu Glu Lys Lys Ala Gln Ile Val Thr Glu Lys
           495                 500                 505
   aat act agc acc aaa aca tca aac cct ttt ctt gta gca gca cag gat     1706
   Asn Thr Ser Thr Lys Thr Ser Asn Pro Phe Leu Val Ala Ala Gln Asp
       510                 515                 520
   tct gag aca gat tat gtc aca aca gat aat tta aca aag gtg act gag     1754
   Ser Glu Thr Asp Tyr Val Thr Thr Asp Asn Leu Thr Lys Val Thr Glu
   525                 530                 535                 540
   gaa gtc gtg gca aac atg cct gaa ggc ctg act cca gat tta gta cag     1802
   Glu Val Val Ala Asn Met Pro Glu Gly Leu Thr Pro Asp Leu Val Gln
                   545                 550                 555
   gaa gca tgt gaa agt gaa ttg aat gaa gtt act ggt aca aag att gct     1850
   Glu Ala Cys Glu Ser Glu Leu Asn Glu Val Thr Gly Thr Lys Ile Ala
               560                 565                 570
   tat gaa aca aaa atg gac ttg gtt caa aca tca gaa gtt atg caa gag     1898
   Tyr Glu Thr Lys Met Asp Leu Val Gln Thr Ser Glu Val Met Gln Glu
           575                 580                 585
   tca ctc tat cct gca gca cag ctt tgc cca tca ttt gaa gag tca gaa      1946
   Ser Leu Tyr Pro Ala Ala Gln Leu Cys Pro Ser Phe Glu Glu Ser Glu
       590                 595                 600
   gct act cct tca cca gtt ttg cct gac att gtt atg gaa gca cca ttg      1994
   Ala Thr Pro Ser Pro Val Leu Pro Asp Ile Val Met Glu Ala Pro Leu
   605                 610                 615                 620
   aat tct gca gtt cct agt gct ggt gct tcc gtg ata cag ccc agc tca      2042
   Asn Ser Ala Val Pro Ser Ala Gly Ala Ser Val Ile Gln Pro Ser Ser
                   625                 630                 635
   tca cca tta gaa gct tct tca gtt aat tat gaa agc ata aaa cat gag      2090
   Ser Pro Leu Glu Ala Ser Ser Val Asn Tyr Glu Ser Ile Lys His Glu
               640                 645                 650
   cct gaa aac ccc cca cca tat gaa gag gcc atg agt gta tca cta aaa      2138
   Pro Glu Asn Pro Pro Pro Tyr Glu Glu Ala Met Ser Val Ser Leu Lys
           655                 660                 665
   aaa gta tca gga ata aag gaa gaa att aaa gag cct gaa aat att aat      2186
   Lys Val Ser Gly Ile Lys Glu Glu Ile Lys Glu Pro Glu Asn Ile Asn
       670                 675                 680
   gca gct ctt caa gaa aca gaa gct cct tat ata tct att gca tgt gat      2234
   Ala Ala Leu Gln Glu Thr Glu Ala Pro Tyr Ile Ser Ile Ala Cys Asp
   685                 690                 695                 700
   tta att aaa gaa aca aag ctt tct gct gaa cca gct ccg gat ttc tct      2282
   Leu Ile Lys Glu Thr Lys Leu Ser Ala Glu Pro Ala Pro Asp Phe Ser
                   705                 710                 715
   gat tat tca gaa atg gca aaa gtt gaa cag cca gtg cct gat cat tct      2330
   Asp Tyr Ser Glu Met Ala Lys Val Glu Gln Pro Val Pro Asp His Ser
               720                 725                 730
   gag cta gtt gaa gat tcc tca cct gat tct gaa cca gtt gac tta ttt      2378
   Glu Leu Val Glu Asp Ser Ser Pro Asp Ser Glu Pro Val Asp Leu Phe
           735                 740                 745
   agt gat gat tca ata cct gac gtt cca caa aaa caa gat gaa act gtg      2426
   Ser Asp Asp Ser Ile Pro Asp Val Pro Gln Lys Gln Asp Glu Thr Val
       750                 755                 760
   atg ctt gtg aaa gaa agt ctc act gag act tca ttt gag tca atg ata      2474
   Met Leu Val Lys Glu Ser Leu Thr Glu Thr Ser Phe Glu Ser Met Ile
   765                 770                 775                 780
   gaa tat gaa aat aag gaa aaa ctc agt gct ttg cca cct gag gga gga      2522
   Glu Tyr Glu Asn Lys Glu Lys Leu Ser Ala Leu Pro Pro Glu Gly Gly
                   785                 790                 795
   aag cca tat ttg gaa tct ttt aag ctc agt tta gat aac aca aaa gat      2570
   Lys Pro Tyr Leu Glu Ser Phe Lys Leu Ser Leu Asp Asn Thr Lys Asp
               800                 805                 810
   acc ctg tta cct gat gaa gtt tca aca ttg agc aaa aag gag aaa att      2618
   Thr Leu Leu Pro Asp Glu Val Ser Thr Leu Ser Lys Lys Glu Lys Ile
           815                 820                 825
   cct ttg cag atg gag gag ctc agt act gca gtt tat tca aat gat gac      2666
   Pro Leu Gln Met Glu Glu Leu Ser Thr Ala Val Tyr Ser Asn Asp Asp
       830                 835                 840
   tta ttt att tct aag gaa gca cag ata aga gaa act gaa acg ttt tca      2714
   Leu Phe Ile Ser Lys Glu Ala Gln Ile Arg Glu Thr Glu Thr Phe Ser
   845                 850                 855                 860
   gat tca tct cca att gaa att ata gat gag ttc cct aca ttg atc agt      2762
   Asp Ser Ser Pro Ile Glu Ile Ile Asp Glu Phe Pro Thr Leu Ile Ser
                   865                 870                 875
   tct aaa act gat tca ttt tct aaa tta gcc agg gaa tat act gac cta      2810
   Ser Lys Thr Asp Ser Phe Ser Lys Leu Ala Arg Glu Tyr Thr Asp Leu
               880                 885                 890
   gaa gta tcc cac aaa agt gaa att gct aat gcc ccg gat gga gct ggg      2858
   Glu Val Ser His Lys Ser Glu Ile Ala Asn Ala Pro Asp Gly Ala Gly
           895                 900                 905
   tca ttg cct tgc aca gaa ttg ccc cat gac ctt tct ttg aag aac ata      2906
   Ser Leu Pro Cys Thr Glu Leu Pro His Asp Leu Ser Leu Lys Asn Ile
       910                 915                 920
   caa ccc aaa gtt gaa gag aaa atc agt ttc tca gat gac ttt tct aaa      2954
   Gln Pro Lys Val Glu Glu Lys Ile Ser Phe Ser Asp Asp Phe Ser Lys
   925                 930                 935                 940
   aat ggg tct gct aca tca aag gtg ctc tta ttg cct cca gat gtt tct      3002
   Asn Gly Ser Ala Thr Ser Lys Val Leu Leu Leu Pro Pro Asp Val Ser
                   945                 950                 955
   gct ttg gcc act caa gca gag ata gag agc ata gtt aaa ccc aaa gtt      3050
   Ala Leu Ala Thr Gln Ala Glu Ile Glu Ser Ile Val Lys Pro Lys Val
               960                 965                 970
   ctt gtg aaa gaa gct gag aaa aaa ctt cct tcc gat aca gaa aaa gag      3098
   Leu Val Lys Glu Ala Glu Lys Lys Leu Pro Ser Asp Thr Glu Lys Glu
           975                 980                 985
   gac aga tca cca tct gct ata ttt tca gca gag ctg agt aaa act tca      3146
   Asp Arg Ser Pro Ser Ala Ile Phe Ser Ala Glu Leu Ser Lys Thr Ser
       990                 995                 1000
   gtt gtt gac ctc ctg tac tgg aga gac att aag aag act gga gtg gtg      3194
   Val Val Asp Leu Leu Tyr Trp Arg Asp Ile Lys Lys Thr Gly Val Val
   1005                1010                1015                1020
   ttt ggt gcc agc cta ttc ctg ctg ctt tca ttg aca gta ttc agc att      3242
   Phe Gly Ala Ser Leu Phe Leu Leu Leu Ser Leu Thr Val Phe Ser Ile
                   1025                1030                1035
   gtg agc gta aca gcc tac att gcc ttg gcc ctg ctc tct gtg acc atc    3290
   Val Ser Val Thr Ala Tyr Ile Ala Leu Ala Leu Leu Ser Val Thr Ile
             1040                1045                1050
   agc ttt agg ata tac aag ggt gtg atc caa gct atc cag aaa tca gat    3338
   Ser Phe Arg Ile Tyr Lys Gly Val Ile Gln Ala Ile Gln Lys Ser Asp
         1055                1060                1065
   gaa ggc cac cca ttc agg gca tat ctg gaa tct gaa gtt gct ata tct    3386
   Glu Gly His Pro Phe Arg Ala Tyr Leu Glu Ser Glu Val Ala Ile Ser
     1070                1075                1080
   gag gag ttg gtt cag aag tac agt aat tct gct ctt ggt cat gtg aac    3434
   Glu Glu Leu Val Gln Lys Tyr Ser Asn Ser Ala Leu Gly His Val Asn
   1085                1090                1095                1100
   tgc acg ata aag gaa ctc agg cgc ctc ttc tta gtt gat gat tta gtt    3482
   Cys Thr Ile Lys Glu Leu Arg Arg Leu Phe Leu Val Asp Asp Leu Val
                 1105                1110                1115
   gat tct ctg aag ttt gca gtg ttg atg tgg gta ttt acc tat gtt ggt    3530
   Asp Ser Leu Lys Phe Ala Val Leu Met Trp Val Phe Thr Tyr Val Gly
             1120                1125                1130
   gcc ttg ttt aat ggt ctg aca cta ctg att ttg gct ctc att tca ctc    3578
   Ala Leu Phe Asn Gly Leu Thr Leu Leu Ile Leu Ala Leu Ile Ser Leu
         1135                1140                1145
   ttc agt gtt cct gtt att tat gaa cgg cat cag gca cag ata gat cat    3626
   Phe Ser Val Pro Val Ile Tyr Glu Arg His Gln Ala Gln Ile Asp His
     1150                1155                1160
   tat cta gga ctt gca aat aag aat gtt aaa gat gct atg gct aaa atc    3674
   Tyr Leu Gly Leu Ala Asn Lys Asn Val Lys Asp Ala Met Ala Lys Ile
   1165                1170                1175                1180
   caa gca aaa atc cct gga ttg aag cgc aaa gct gaa tgaaaacgcc         3720
   Gln Ala Lys Ile Pro Gly Leu Lys Arg Lys Ala Glu
                 1185                1190
   caaaataatt agtaggagtt catctttaaa ggggatattc atttgattat acgggggagg 3780
   gtcagggaag aacgaacctt gacgttgcag tgcagtttca cagatcgttg ttagatcttt 3840
   atttttagcc atgcactgtt gtgaggaaaa attacctgtc ttgactgcca tgtgttcatc 3900
   atcttaagta ttgtaagctg ctatgtatgg atttaaaccg taatcatatc tttttcctat 3960
   ctgaggcact ggtggaataa aaaacctgta tattttactt tgttgcagat agtcttgccg 4020
   catcttggca agttgcagag atggtggagc tag                              4053
<210> 6
   <211> 1192
   <212> PRT
   <213> Homo sapiens
<400> 6
   Met Glu Asp Leu Asp Gln Ser Pro Leu Val Ser Ser Ser Asp Ser Pro
   1               5                   10                  15
   Pro Arg Pro Gln Pro Ala Phe Lys Tyr Gln Phe Val Arg Glu Pro Glu
               20                  25                  30
   Asp Glu Glu Glu Glu Glu Glu Glu Glu Glu Glu Asp Glu Asp Glu Asp
           35                  40                  45
   Leu Glu Glu Leu Glu Val Leu Glu Arg Lys Pro Ala Ala Gly Leu Ser
       50                  55                  60
   Ala Ala Pro Val Pro Thr Ala Pro Ala Ala Gly Ala Pro Leu Met Asp
   65                  70                  75                  80
   Phe Gly Asn Asp Phe Val Pro Pro Ala Pro Arg Gly Pro Leu Pro Ala
                   85                  90                  95
   Ala Pro Pro Val Ala Pro Glu Arg Gln Pro Ser Trp Asp Pro Ser Pro
               100                 105                 110
   Val Ser Ser Thr Val Pro Ala Pro Ser Pro Leu Ser Ala Ala Ala Val
           115                 120                 125
   Ser Pro Ser Lys Leu Pro Glu Asp Asp Glu Pro Pro Ala Arg Pro Pro
       130                 135                 140
   Pro Pro Pro Pro Ala Ser Val Ser Pro Gln Ala Glu Pro Val Trp Thr
   145                 150                 155                 160
   Pro Pro Ala Pro Ala Pro Ala Ala Pro Pro Ser Thr Pro Ala Ala Pro
                   165                 170                 175
   Lys Arg Arg Gly Ser Ser Gly Ser Val Asp Glu Thr Leu Phe Ala Leu
               180                 185                 190
   Pro Ala Ala Ser Glu Pro Val Ile Arg Ser Ser Ala Glu Asn Met Asp
           195                 200                 205
   Leu Lys Glu Gln Pro Gly Asn Thr Ile Ser Ala Gly Gln Glu Asp Phe
       210                 215                 220
   Pro Ser Val Leu Leu Glu Thr Ala Ala Ser Leu Pro Ser Leu Ser Pro
   225                 230                 235                 240
   Leu Ser Ala Ala Ser Phe Lys Glu His Glu Tyr Leu Gly Asn Leu Ser
                   245                 250                 255
   Thr Val Leu Pro Thr Glu Gly Thr Leu Gln Glu Asn Val Ser Glu Ala
               260                 265                 270
   Ser Lys Glu Val Ser Glu Lys Ala Lys Thr Leu Leu Ile Asp Arg Asp
           275                 280                 285
   Leu Thr Glu Phe Ser Glu Leu Glu Tyr Ser Glu Met Gly Ser Ser Phe
   290 295 300
   Ser Val Ser Pro Lys Ala Glu Ser Ala Val Ile Val Ala Asn Pro Arg
   305 310 315 320
   Glu Glu Ile Ile Val Lys Asn Lys Asp Glu Glu Glu Lys Leu Val Ser
   325 330 335
   Asn Asn Ile Leu His Asn Gln Gln Glu Leu Pro Thr Ala Leu Thr Lys
   340 345 350
   Leu Val Lys Glu Asp Glu Val Val Ser Ser Glu Lys Ala Lys Asp Ser
   355 360 365
   Phe Asn Glu Lys Arg Val Ala Val Glu Ala Pro Met Arg Glu Glu Tyr
   370 375 380
   Ala Asp Phe Lys Pro Phe Glu Arg Val Trp Glu Val Lys Asp Ser Lys
   385 390 395 400
   Glu Asp Ser Asp Met Leu Ala Ala Gly Gly Lys Ile Glu Ser Asn Leu
   405 410 415
   Glu Ser Lys Val Asp Lys Lys Cys Phe Ala Asp Ser Leu Glu Gln Thr
   420 425 430
   Asn His Glu Lys Asp Ser Glu Ser Ser Asn Asp Asp Thr Ser Phe Pro
   435 440 445
   Ser Thr Pro Glu Gly Ile Lys Asp Arg Ser Gly Ala Tyr Ile Thr Cys
   450 455 460
   Ala Pro Phe Asn Pro Ala Ala Thr Glu Ser Ile Ala Thr Asn Ile Phe
   465 470 475 480
   Pro Leu Leu Gly Asp Pro Thr Ser Glu Asn Lys Thr Asp Glu Lys Lys
   485 490 495
   Ile Glu Glu Lys Lys Ala Gln Ile Val Thr Glu Lys Asn Thr Ser Thr
   500 505 510
   Lys Thr Ser Asn Pro Phe Leu Val Ala Ala Gln Asp Ser Glu Thr Asp
   515 520 525
   Tyr Val Thr Thr Asp Asn Leu Thr Lys Val Thr Glu Glu Val Val Ala
   530 535 540
   Asn Met Pro Glu Gly Leu Thr Pro Asp Leu Val Gln Glu Ala Cys Glu
   545 550 555 560
   Ser Glu Leu Asn Glu Val Thr Gly Thr Lys Ile Ala Tyr Glu Thr Lys
   565 570 575
   Met Asp Leu Val Gln Thr Ser Glu Val Met Gln Glu Ser Leu Tyr Pro
   580 585 590
   Ala Ala Gln Leu Cys Pro Ser Phe Glu Glu Ser Glu Ala Thr Pro Ser
   595 600 605
   Pro Val Leu Pro Asp Ile Val Met Glu Ala Pro Leu Asn Ser Ala Val
   610 615 620
   Pro Ser Ala Gly Ala Ser Val Ile Gln Pro Ser Ser Ser Pro Leu Glu
   625 630 635 640
   Ala Ser Ser Val Asn Tyr Glu Ser Ile Lys His Glu Pro Glu Asn Pro
   645 650 655
   Pro Pro Tyr Glu Glu Ala Met Ser Val Ser Leu Lys Lys Val Ser Gly
   660 665 670
   Ile Lys Glu Glu Ile Lys Glu Pro Glu Asn Ile Asn Ala Ala Leu Gln
   675 680 685
   Glu Thr Glu Ala Pro Tyr Ile Ser Ile Ala Cys Asp Leu Ile Lys Glu
   690 695 700
   Thr Lys Leu Ser Ala Glu Pro Ala Pro Asp Phe Ser Asp Tyr Ser Glu
   705 710 715 720
   Met Ala Lys Val Glu Gln Pro Val Pro Asp His Ser Glu Leu Val Glu
   725 730 735
   Asp Ser Ser Pro Asp Ser Glu Pro Val Asp Leu Phe Ser Asp Asp Ser
   740 745 750
   Ile Pro Asp Val Pro Gln Lys Gln Asp Glu Thr Val Met Leu Val Lys
   755 760 765
   Glu Ser Leu Thr Glu Thr Ser Phe Glu Ser Met Ile Glu Tyr Glu Asn
   770 775 780
   Lys Glu Lys Leu Ser Ala Leu Pro Pro Glu Gly Gly Lys Pro Tyr Leu
   785 790 795 800
   Glu Ser Phe Lys Leu Ser Leu Asp Asn Thr Lys Asp Thr Leu Leu Pro
   805 810 815
   Asp Glu Val Ser Thr Leu Ser Lys Lys Glu Lys Ile Pro Leu Gln Met
   820 825 830
   Glu Glu Leu Ser Thr Ala Val Tyr Ser Asn Asp Asp Leu Phe Ile Ser
   835 840 845
   Lys Glu Ala Gln Ile Arg Glu Thr Glu Thr Phe Ser Asp Ser Ser Pro
   850 855 860
   Ile Glu Ile Ile Asp Glu Phe Pro Thr Leu Ile Ser Ser Lys Thr Asp
   865 870 875 880
   Ser Phe Ser Lys Leu Ala Arg Glu Tyr Thr Asp Leu Glu Val Ser His
   885 890 895
   Lys Ser Glu Ile Ala Asn Ala Pro Asp Gly Ala Gly Ser Leu Pro Cys
               900                 905                 910
   Thr Glu Leu Pro His Asp Leu Ser Leu Lys Asn Ile Gln Pro Lys Val
           915                 920                 925
   Glu Glu Lys Ile Ser Phe Ser Asp Asp Phe Ser Lys Asn Gly Ser Ala
       930                 935                 940
   Thr Ser Lys Val Leu Leu Leu Pro Pro Asp Val Ser Ala Leu Ala Thr
   945                 950                 955                 960
   Gln Ala Glu Ile Glu Ser Ile Val Lys Pro Lys Val Leu Val Lys Glu
                   965                 970                 975
   Ala Glu Lys Lys Leu Pro Ser Asp Thr Glu Lys Glu Asp Arg Ser Pro
               980                 985                 990
   Ser Ala Ile Phe Ser Ala Glu Leu Ser Lys Thr Ser Val Val Asp Leu
           995                 1000                1005
   Leu Tyr Trp Arg Asp Ile Lys Lys Thr Gly Val Val Phe Gly Ala Ser
       1010                1015                1020
   Leu Phe Leu Leu Leu Ser Leu Thr Val Phe Ser Ile Val Ser Val Thr
   1025                1030                1035                1040
   Ala Tyr Ile Ala Leu Ala Leu Leu Ser Val Thr Ile Ser Phe Arg Ile
                   1045                1050                1055
   Tyr Lys Gly Val Ile Gln Ala Ile Gln Lys Ser Asp Glu Gly His Pro
               1060                1065                1070
   Phe Arg Ala Tyr Leu Glu Ser Glu Val Ala Ile Ser Glu Glu Leu Val
           1075                1080                1085
   Gln Lys Tyr Ser Asn Ser Ala Leu Gly His Val Asn Cys Thr Ile Lys
       1090                1095                1100
   Glu Leu Arg Arg Leu Phe Leu Val Asp Asp Leu Val Asp Ser Leu Lys
   1105                1110                1115                1120
   Phe Ala Val Leu Met Trp Val Phe Thr Tyr Val Gly Ala Leu Phe Asn
                   1125                1130                1135
   Gly Leu Thr Leu Leu Ile Leu Ala Leu Ile Ser Leu Phe Ser Val Pro
               1140                1145                1150
   Val Ile Tyr Glu Arg His Gln Ala Gln Ile Asp His Tyr Leu Gly Leu
           1155                1160                1165
   Ala Asn Lys Asn Val Lys Asp Ala Met Ala Lys Ile Gln Ala Lys Ile
       1170                1175                1180
   Pro Gly Leu Lys Arg Lys Ala Glu
   1185                1190
<210> 7
   <211> 75
   <212> DNA
   <213> Artificial Sequence
<220>
   <223> Description of Artificial Sequence: cDNA encoding receptor binding inhibitor Pep1
<400> 7
   tttaggatat acaagggtgt gatccaagct atccagaaat cagatgaagg ccacccattc 60
   agggcatatc tggaa                                                  75
<210> 8
   <211> 40
   <212> PRT
   <213> Artificial Sequence
<220>
   <223> Description of Artificial Sequence: Pep1- Nogo protein inhibitor
<400> 8
   Arg Ile Tyr Lys Gly Val Ile Gln Ala Ile Gln Lys Ser Asp Glu Gly
    1               5                  10                  15
   His Pro Phe Arg Ala Tyr Leu Glu Ser Glu Val Ala Ile Ser Glu Glu
               20                  25                  30
   Leu Val Gln Lys Tyr Ser Asn Ser
           35                  40
<210> 9
   <211> 75
   <212> DNA
   <213> Artificial Sequence
<220>
   <223> Description of Artificial Sequence: cDNA encoding receptor binding inhibitor Pep2
<400> 9
   atccagaaat cagatgaagg ccacccattc agggcatatc tggaatctga agttgctata 60
   tctgaggagt tggtt                                                  75
<210> 10
   <211> 25
   <212> PRT
   <213> Artificial Sequence
<220>
   <223> Description of Artificial Sequence: Pep2- Nogo protein inhibitor
<400> 10
   Ile Gln Lys Ser Asp Glu Gly His Pro Phe Arg Ala Tyr Leu Glu Ser
   1               5                   10                  15
   Glu Val Ala Ile Ser Glu Glu Leu Val
               20                  25
<210> 11
   <211> 75
   <212> PRT
   <213> Artificial Sequence
<220>
   <223> Description of Artificial Sequence: cDNA encoding receptor binding inhibitor Pep3
<400> 11
   Ala Gly Gly Gly Cys Ala Thr Ala Thr Cys Thr Gly Gly Ala Ala Thr
   1               5                   10                  15
   Cys Thr Gly Ala Ala Gly Thr Thr Gly Cys Thr Ala Thr Ala Thr Cys
               20                  25                  30
   Thr Gly Ala Gly Gly Ala Gly Thr Thr Gly Gly Thr Thr Cys Ala Gly
           35                  40                  45
   Ala Ala Gly Thr Ala Cys Ala Gly Thr Ala Ala Thr Thr Cys Thr Gly
       50                  55                  60
   Cys Thr Cys Thr Thr Gly Gly Thr Cys Ala Thr
   65                  70                  75
<210> 12
   <211> 25
   <212> PRT
   <213> Artificial Sequence
<220>
   <223> Description of Artificial Sequence: Pep3- Nogo protein inhibitor
<400> 12
   Arg Ala Tyr Leu Glu Ser Glu Val Ala Ile Ser Glu Glu Leu Val Gln
   1               5                   10                  15
   Lys Tyr Ser Asn Ser Ala Leu Gly His
               20                  25
<210> 13
   <211> 75
   <212> DNA
   <213> Artificial Sequence
<220>
   <223> Description of Artificial Sequence: cDNA encoding receptor binding inhibitor Pep4
<400> 13
   tctgaggagt tggttcagaa gtacagtaat tctgctcttg gtcatgtgaa ctgcacgata 60
   aaggaactca ggcgc                                                  75
<210> 14
   <211> 25
   <212> PRT
   <213> Artificial Sequence
<220>
   <223> Description of Artificial Sequence: Pep4- Nogo protein inhibitor
<400> 14
   Ser Glu Glu Leu Val Gln Lys Tyr Ser Asn Ser Ala Leu Gly His Val
    1               5                  10                  15
   Asn Cys Thr Ile Lys Glu Leu Arg Arg
               20                  25
<210> 15
   <211> 75
   <212> DNA
   <213> Artificial Sequence
<220>
   <223> Description of Artificial Sequence: cDNA encoding receptor binding inhibitor Pep5
<400> 15
   gctcttggtc atgtgaactg cacgataaag gaactcaggc gcctcttctt agttgatgat 60
   ttagttgatt ctctg                                                  75
<210> 16
   <211> 25
   <212> PRT
   <213> Artificial Sequence
<220>
   <223> Description of Artificial Sequence: Pep5- Nogo protein inhibitor
<400> 16
   Ala Leu Gly His Val Asn Cys Thr Ile Lys Glu Leu Arg Arg Leu Phe
    1               5                  10                  15
   Leu Val Asp Asp Leu Val Asp Ser Leu
               20                  25
<210> 17
   <211> 120
   <212> DNA
   <213> Artificial Sequence
<220>
   <223> Description of Artificial Sequence: cDNA encoding receptor binding inhibitor Pep2-41
<400> 17
   aggatataca agggtgtgat ccaagctatc cagaaatcag atgaaggcca cccattcagg 60
   gcatatctgg aatctgaagt tgctatatct gaggagttgg ttcagaagta cagtaattct 120
<210> 18
   <211> 40
   <212> PRT
   <213> Artificial Sequence
<220>
   <223> Description of Artificial Sequence: Pep2-41- Nogo protein inhibitor
<400> 18
   Arg Ile Tyr Lys Gly Val Ile Gln Ala Ile Gln Lys Ser Asp Glu Gly
   1               5                   10                  15
   His Pro Phe Arg Ala Tyr Leu Glu Ser Glu Val Ala Ile Ser Glu Glu
               20                  25                  30
   Leu Val Gln Lys Tyr Ser Asn Ser
           35                  40
<210> 19
   <211> 198
   <212> DNA
   <213> Homo sapiens
<220>
   <221> CDS
   <222> (1) .. (198)
   <223> Full receptor binding region of Nogo gene
<400> 19
   ttt agg ata tac aag ggt gtg atc caa gct atc cag aaa tca gat gaa    48
   Phe Arg Ile Tyr Lys Gly Val Ile Gln Ala Ile Gln Lys Ser Asp Glu
   1               5                   10                  15
   ggc cac cca ttc agg gca tat ctg gaa tct gaa gtt gct ata tct gag    96
   Gly His Pro Phe Arg Ala Tyr Leu Glu Ser Glu Val Ala Ile Ser Glu
               20                  25                  30
   gag ttg gtt cag aag tac agt aat tct gct ctt ggt cat gtg aac tgc    144
   Glu Leu Val Gln Lys Tyr Ser Asn Ser Ala Leu Gly His Val Asn Cys
           35                  40                  45
   acg ata aag gaa ctc agg cgc ctc ttc tta gtt gat gat tta gtt gat    192
   Thr Ile Lys Glu Leu Arg Arg Leu Phe Leu Val Asp Asp Leu Val Asp
       50                  55                  60
   tct ctg                                                            198
   Ser Leu
   65
<210> 20
   <211> 66
   <212> PRT
   <213> Homo sapiens
<400> 20
   Phe Arg Ile Tyr Lys Gly Val Ile Gln Ala Ile Gln Lys Ser Asp Glu
   1               5                   10                  15
   Gly His Pro Phe Arg Ala Tyr Leu Glu Ser Glu Val Ala Ile Ser Glu
               20                  25                  30
   Glu Leu Val Gln Lys Tyr Ser Asn Ser Ala Leu Gly His Val Asn Cys
           35                  40                  45
   Thr Ile Lys Glu Leu Arg Arg Leu Phe Leu Val Asp Asp Leu Val Asp
       50                  55                  60
   Ser Leu
   65

## Description

### CROSS-REFERENCE TO RELATED APPLICATIONS

This application is related to U.S. Provisional Patent Applications 60/175,707 filed January 12, 2000; 60/207,366 filed May 26, 2000 and 60/236,378 filed September 29, 2000.

### U.S. GOVERNMENT SUPPORT

This invention was partially made with government support under National Institute of Health Grant 5-R01-NS33020.

### FIELD OF THE INVENTION

This disclosure relates specifically to novel human and murine genes which encode a receptor for the Nogo protein, this receptor being capable of regulating axonal growth. These Nogo receptor genes are selectively expressed in axons and dendrites of neurons in the central nervous system during axonal growth. The disclosure also relates to compositions and methods for the selective blockade of Nogo receptor-mediated inhibition of axonal growth by blocking the interaction of Nogo with the Nogo receptor. The blockade of the interaction of Nogo with its receptor results in a blockade of the inhibitory effects of Nogo on axonal growth causing a subsequent increase in axonal growth.

### BACKGROUND OF THE INVENTION

Axons and dendrites of neurons are long cellular extensions from neurons. At the distal tip of an extending axon or neurite is a specialized region, known as the growth cone. Growth cones are responsible for sensing the local environment and moving toward the neuron's target cell. Growth cones are hand shaped, with several long filopodia that differentially adhere to surfaces in the embryo. Growth cones can be sensitive to several guidance cues, for example, surface adhesiveness, growth factors, neurotransmitters and electric fields. The guidance of growth at the cone depends on various classes of adhesion molecules, intercellular signals, as well as factors which stimulate and inhibit growth cones. The growth cone located at the end of a growing neurite advances at various rates, but typically at the speed of one to two millimeters per day. The cone consists of a broad and flat expansion, with numerous long microspikes or filopodia that extend like spikes. These filopodia are continually active. While some filopodia retract back into the growth cone, others continue to elongate through the substratum. The elongations between different filopodia form lamellipodia.

The growth cone can explore the area that is ahead of it and on either side with its lamellipodia and filopodia. When an elongation comes in contact with a surface that is unfavorable, it withdraws. When an elongation comes into contact with a favorable surface, it continues to extend and can manipulate the growth cone moving in that direction. Hence, the growth cone can be guided by small variations in surface properties of the substrata. When the growth cone reaches an appropriate target cell a synaptic connection is created.

Damaged neurons do not regenerate in the central nervous system (CNS) following injury due to trauma and disease. The absence of axon regeneration following injury can be attributed to the presence of axon growth inhibitors. These inhibitors are predominantly associated with myelin and constitute an important barrier to regeneration. Axon growth inhibitors are present in CNS-derived myelin and the plasma membrane of oligodendrocytes, which synthesize myelin in the CNS (Schwab et al., (1993) Ann. Rev. Neurosci. 16, 565-595).

CNS myelin is an elaborate extension of the oligodendrocyte cell membrane. A single oligodendrocyte myelinates as many as thirty different CNS axonal segments. Oligodendrocyte membrane extensions wrap around the axons in a concentric fashion to form the myelin sheath. Tightly compacted mature myelin consists of parallel layers of bimolecular lipids apposed to layers of hydrated protein. Active myelin synthesis starts *in utero* and continues for the first two years of human life. Slower synthesis continues through childhood and adolescence while turnover of mature myelin continues at a slower rate throughout adult life. Both developing and mature forms of myelin are susceptible to injury from disease or physical trauma resulting in degradation of the myelin surrounding axons.

Myelin-associated inhibitors appear to be a primary contributor to the failure of CNS axon regeneration *in vivo* after an interruption of axonal continuity, while other non-myelin associated axon growth inhibitors in the CNS may play a lesser role. These inhibitors block axonal regeneration following neuronal injury due to trauma, stroke, or viral infection.

Numerous myelin-derived axon growth inhibitors have been characterized (see, for review, David et al., (1999) WO995394547; Bandman et al., (1999) U.S. Patent 5,858,708; Schwab, (1996) Neurochem. Res. 21, 755-761). Several components of CNS white matter, NI35, NI250 (Nogo) and Myelin-associated glycoprotein (MAG), which have inhibitory activity for axonal extension, have been also been described (Schwab et al., (1990) WO9005191; Schwab et al., (1997) U.S. Patent 5,684,133). In particular, Nogo is a 250 kDa myelin-associated axon growth inhibitor which has been cloned and characterized (Nagase et al., (1998) DNA Res. 5, 355-364; Schwab, (1990) Exp. Neurol. 109,2-5). The Nogo cDNA was first identified through random analysis of brain cDNA and had no suggested function (Nagase et al., (1998) DNA Res. 5, 355-364).

Schwab and colleagues published the sequence of six peptides randomly derived from a proteolytic digest of presumed bovine NI250 (Nogo) protein (Spillmann et al., (1998) J. Biol. Chem. 273, 19283-19293). A probable full-length cDNA sequence for this protein was recently deposited in the GenBank. This 4.1 kilobase human cDNA clone, KIAA0886, is derived from the Kazusa DNA Research Institute effort to sequence random high molecular weight brain-derived cDNA (Nagase et al., (1998) DNA Res. 31, 355-364). This novel cDNA clone encodes a 135 kDa protein that includes all six of the peptide sequences derived from bovine Nogo.

The human Nogo-A sequence shares high homology over its carboxyl third with the Reticulon (Rtn) protein family. Rtn1 has also been termed neuro-endocrine specific protein (NSP) because it is expressed exclusively in neuro-endocrine cells (Van de Velde et al., (1994) J. Cell. Sci. 107, 2403-2416). All Rtn proteins share a 200 amino acid residue region of sequence similarity at the carboxyl terminus of the protein (Van de Velde et al., (1994) J. Cell. Sci. 107,2403-2416; Roebroek et al., (1996) Genomics 32, 191-199; Roebroek et al., (1998) Genomics 51, 98-106; Moreira et al., (1999) Genomics 58,73-81*;* Morris et al., (1991) Biochim. Biophys. Acta 1450, 68-76). Related sequences have been recognized in the fly and worm genomes (Moreira et al., (1999) Genomics 58, 73-81). This region is approximately 70% identical across the Rtn family. Amino terminal regions are not related to one another and are derived from various alternative RNA splicing events.

From analysis of sequences deposited in the GenBank and by homology with published Rtn1 isoforms, three forms of the Nogo protein are predicted (Nogo-A, Nogo-B, Nogo-C). Nogo-B of 37 kDa might possibly correspond to NI35, and explain the antigenic relatedness of the NI35 and NI250 (Nogo-A) axon outgrowth inhibiting activity. Nogo-C-Myc exhibits an electrophoretic mobility of 25 kDa by SDS-PAGE and has been described previously as Rtn4 and vp2015. The ability of Nogo-A protein to inhibit axonal regeneration has been recognized only recently (GrandPré et al., (2000) Nature 403, 439-444; Chen et al., (2000) Nature 403, 434-439; Prinjha et al., (2000) Nature 403, 483-484).

The absence of re-extension of axons across lesions in the CNS following injury has been attributed as a cause of the permanent deleterious effects associated with trauma, stroke and demyelinating disorders. Modulation of NI250 has been described as a means for treatment of regeneration for neurons damaged by trauma, infarction and degenerative disorders of the CNS (Schwab et al., (1994) W09417831; Tatagiba et al., (1997) Neurosurgery 40,541-546) as well as malignant tumors in the CNS such as glioblastoma (Schwab et al., (1993) U.S. Patent 5,250,414; Schwab et al., (2000) U.S. Patent 6,025,333).

Antibodies which recognize NI250 have been reported to be useful in the diagnosis and treatment of nerve damage resulting from trauma, infarction and degenerative disorders of the CNS (Schnell & Schwab, (1990) Nature 343,269-272; Schwab et al., (1997) U.S. Patent 5,684,133). In axons which become myelinated, there is a correlation with the development of myelin and the appearance ofNogo. After Nogo is blocked by antibodies, neurons can again extend across lesions caused by nerve damage (Varga et al., (1995) Proc. Natl. Acad. Sci. USA 92, 10959-10963).

The mechanism of action whereby Nogo inhibits axonal growth has not yet been elucidated. Identification and characterization of this mechanism of action and the biochemical pathways associated with the effects of Nogo would be useful in treatment of disease states associated with axonal injury and axonal demyelination.

### SUMMARY OF THE INVENTION

The present disclosure is based on the discovery of Nogo receptor proteins and biologically active Nogo protein (ligand) fragments. The disclosure provides an isolated nucleic acid molecule selected from the group consisting of an isolated nucleic acid molecule that encodes the amino acid sequence of SEQ ID NO: 2, 4, 8, 10,12,14,16,18 or 20; an isolated nucleic acid molecule that encodes a fragment of at least six, *e.g*., ten, fifteen, twenty, twenty-five, thirty, forty, fifty, sixty or seventy amino acids of SEQ ID NO: 2,4,8,10,12,14,16,18 or 20; an isolated nucleic acid molecule which hybridizes to a nucleic acid molecule comprising the complement of SEQ ID NO: 1, 3, 7, 9, 11, 13, 15, 17 or 19 under high stringency conditions; and an isolated nucleic acid molecule with at least seventy-five, *e.g*., eighty, eighty-five, ninety or ninety-five percent amino acid sequence identity to SEQ ID NO: 1, 3, 7, 9, 11, 13, 15, 17 or 19. In a preferred instance, the disclosure includes an isolated nucleic acid molecule comprising nucleotides 166 to 1584 of SEQ ID NO: 1 or nucleotides 178 to 1596 of SEQ ID NO: 3.

The present disclosure further includes the nucleic acid molecules operably linked to one or more expression control elements, including vectors comprising the isolated nucleic acid molecules. The disclosure further includes host cells transformed to contain the nucleic acid molecules of the disclosure and methods for producing a protein comprising the step of culturing a host cell transformed with a nucleic acid molecule of the disclosure under conditions in which the protein is expressed.

The present disclosure includes an isolated polypeptide selected from the group consisting of an isolated polypeptide comprising the amino acid sequence of SEQ ID NO: 2, 4, 8, 10, 12, 14, 16, 18 or 20; an isolated polypeptide comprising a fragment of at least six, *e.g*., ten, fifteen, twenty, twenty-five, thirty, forty, fifty, sixty or seventy amino acids of SEQ ID NO: 2, 4, 8, 10, 12, 14, 16, 18 or 20; an isolated polypeptide comprising the amino acid sequence of SEQ ID NO: 2, 4, 8, 10, 12, 14, 16, 18 or 20 comprising at least one, *e.g*., five, ten; fifteen or twenty conservative amino acid substitutions; an isolated polypeptide comprising the amino acid sequence of SEQ ID NO: 2, 4, 8, 10, 12, 14, 16, 18 or 20 comprising one, *e.g*., five, ten, fifteen or twenty naturally occurring amino acid sequence substitutions; and an isolated polypeptide with at least seventy-five, *e.g*., eighty, eighty-five, ninety or ninety-five percent amino acid sequence identity to SEQ ID NO: 2, 4, 8, 10, 12, 14, 16, 18 or 20. The disclosure also includes chimeric polypeptides comprising the amino acid sequence of SEQ ID NO: 2, 4, 8, 10, 12, 14, 16, 18 or 20.

The disclosure further provides antibodies that bind to a Nogo protein and antibodies which bind to a Nogo receptor protein. The antibodies can be monoclonal or polyclonal antibodies. In addition, the antibody may be humanized. The disclosure also includes antibody fragments which display antigen binding activity.

The disclosure includes a method of identifying an agent which modulates Nogo protein or Nogo receptor protein expression comprising the steps of providing a cell expressing a Nogo protein or Nogo receptor protein; contacting the cell with a candidate agent; and detecting an increase or decrease in the level of Nogo protein or Nogo receptor protein expression in the presence of the candidate agent relative to the level of Nogo protein or Nogo receptor protein expression in the absence of the candidate agent.

The disclosure also includes a method of identifying an agent which modulates at least one activity of a Nogo protein or Nogo receptor protein comprising the steps of providing a cell expressing a Nogo protein or Nogo receptor protein; contacting the cell with a candidate agent; and detecting an increase or decrease in the level of Nogo protein or Nogo receptor protein activity in the presence of the candidate agent relative to the level of Nogo protein or Nogo receptor protein activity in the absence of the candidate agent. In one instance of the disclosure, the activity is growth cone movement. In another instance, the agent is selected from the group consisting of a Nogo protein fragment, anti-Nogo antibody and anti-Nogo receptor antibody.

The disclosure further includes a method of identifying a binding partner for a Nogo receptor protein comprising the steps of providing a Nogo receptor protein; contacting the Nogo receptor with a candidate binding partner; and detecting binding of the candidate binding partner to the Nogo receptor protein. In one instance, the binding partner is selected from the group consisting of a Nogo protein fragment, an anti-Nogo antibody, an anti-Nogo receptor antibody fragment; and a humanized anti-Nogo receptor antibody.

The disclosure encompasses a method of treating a central nervous system disorder in a mammal comprising the step of administering an effective amount of an agent which modulates the expression of a Nogo protein or Nogo receptor protein. In some instances of the disclosure the expression is decreased, while in other embodiments, it is increased.

The disclosure further encompasses a method of treating a central nervous system disorder in a mammal comprising the step of administering an effective amount of an agent which modulates the activity of a Nogo protein or Nogo receptor protein. The activity may be either increased or decreased. If the activity is decreased, the agent can be *e.g*., a polypeptide comprising the amino acid sequence of SEQ ID NO: 8, 10, 12, 18 or 20; a Nogo receptor protein fragment; a soluble Nogo receptor protein fragment; or an anti-Nogo receptor antibody or active fragment thereof. If the activity is increased the agent is a polypeptide selected from the group consisting of SEQ ID NO: 14 and 16.

A soluble Nogo receptor protein can comprise a fragment of at least six, *e.g*., ten, fifteen, twenty, twenty-five, thirty, forty, fifty, sixty or seventy amino acids of SEQ ID NO: 2 or 4; the amino acid sequence of SEQ ID NO: 2, 4, 8, 10, 12, 14, 16, 18 or 20; the amino acid sequence of SEQ ID NO: 2, 4, 8, 10, 12, 14, 16, 18 or 20 comprising at least one, *e.g*., five, ten, fifteen or twenty conservative amino acid substitutions; the amino acid sequence of SEQ ID NO: 2, 4, 8, 10, 12, 14, 16, 18 or 20 comprising one, *e.g*., five, ten, fifteen or twenty naturally occurring amino acid sequence substitutions.

In some instances, the central nervous system disorder is a result of cranial or cerebral trauma, spinal cord injury, stroke or a demyelinating disease. Examples of demyelinating diseases are multiple sclerosis, monophasic demyelination, encephalomyelitis, multifocal leukoencephalopathy, panencephalitis, Marchiafava-Bignami disease, pontine myelinolysis, adrenoleukodystrophy, Pelizaeus-Merzbacher disease, Spongy degeneration, Alexander's disease, Canavan's disease, metachromatic leukodystrophy and Krabbe's disease.

The disclosure further encompasses an isolated peptide that specifically binds to a Nogo receptor protein. The specific binding of the peptide to the Nogo receptor protein preferably has at least one of the following effects: inhibition of binding of a Nogo protein to the Nogo receptor protein, blockade of No go-mediated inhibition of axonal growth, modulation of Nogo protein expression, or modulation of Nogo receptor protein expression. In some instances, the isolated peptide comprises the amino acid sequence of SEQ ID NO: 8, 10, 12, 14, 16, 18 or 20, or one of the foregoing with one or more, *e.g*., five, ten, fifteen or twenty consecutive amino acid substitutions or naturally occurring amino acid substitutions.

Based on the disclosure that is contained herein, the present invention provides an isolated nucleic acid molecule selected from the group consisting of:
(a) an isolated nucleic acid molecule that encodes a fragment of SEQ ID NO:2;
(b) an isolated nucleic acid molecule which hybridises to a nucleic acid molecule comprising the complement of SEQ ID NO: 1 under high stringency conditions; and
(c) an isolated nucleic acid molecule with at least seventy-five percent sequence homology to SEQ ID NO: 1;
wherein said isolated nucleic acid molecule encodes a polypeptide which blocks the inhibition of axonal growth that is mediated by the NOGO-receptor of SEQ ID NO:2.

The invention also provides a vector comprising said isolated nucleic acid molecule, a host cell transformed to contain said isolated nucleic acid molecule or a host cell containing said vector, a method of producing a polypeptide comprising the step of culturing said host cell under conditions in which the polypeptide encoded by said nucleic acid molecule is expressed, and an isolated polypeptide produced by said method.

The invention also provides an isolated polypeptide selected from the group consisting of:
(a) an isolated polypeptide comprising a fragment of at least 30 amino acids of SEQ ID NO:2; and
(b) an isolated polypeptide with at least seventy-five percent amino acid sequence homology to SEQ ID NO:2;
wherein said polypeptide blocks the inhibition of axonal growth that is mediated by the NOGO-receptor of SEQ ID NO:2.

In another aspect, the invention provides an antibody or antigen-binding fragment thereof that specifically binds to said polypeptide wherein said antibody or antigen-binding fragment inhibits the binding between the NOGO-receptor of SEQ ID NO:2 and the NOGO polypeptide of SEQ ID NO:6 and wherein said antibody or antigen-binding fragment blocks the inhibition of axonal growth that is mediated by the NOGO-receptor of SEQ ID NO:2.

In a further aspect, the invention provides a composition comprising a polypeptide, antibody or antigen-binding fragment of the invention, and a pharmaceutically acceptable carrier.

In still further aspects, the invention provides for the use of a polypeptide, antibody or antigen-binding fragment of the invention for the preparation of a medicament for promoting axonal growth or axonal regeneration, and for the use of a polynucleotide, polypeptide, antibody or antigen-binding fragment of the invention for the preparation of a medicament for the treatment of a central nervous system (CNS) disease, disorder, or injury, in a mammal.

The CNS disease, disorder, or injury may be selected from the group consisting of cranial or cerebral trauma, spinal cord injury, stroke or a demyelinating disease.

The demyelinating disease may be selected from the group consisting of multiple sclerosis, monophasic demyelination, encephalomyelitis, multifocal leukoencephalopathy, panencephalitis, Marchiafava-Bignami disease, pontine myelinolysis, adrenoleukodystrophy, Pelizaeus-Merzbacher disease, Spongy degeneration, Alexander's disease, Canavan's disease, metachromatic leukodystrophy, and Krabbe's disease.

The invention further provides an *in vitro* method of promoting axonal growth or axonal regeneration comprising contacting a neuron with a polypeptide, antibody or antigen-binding fragment of the invention.

### DESCRIPTION OF THE FIGURES

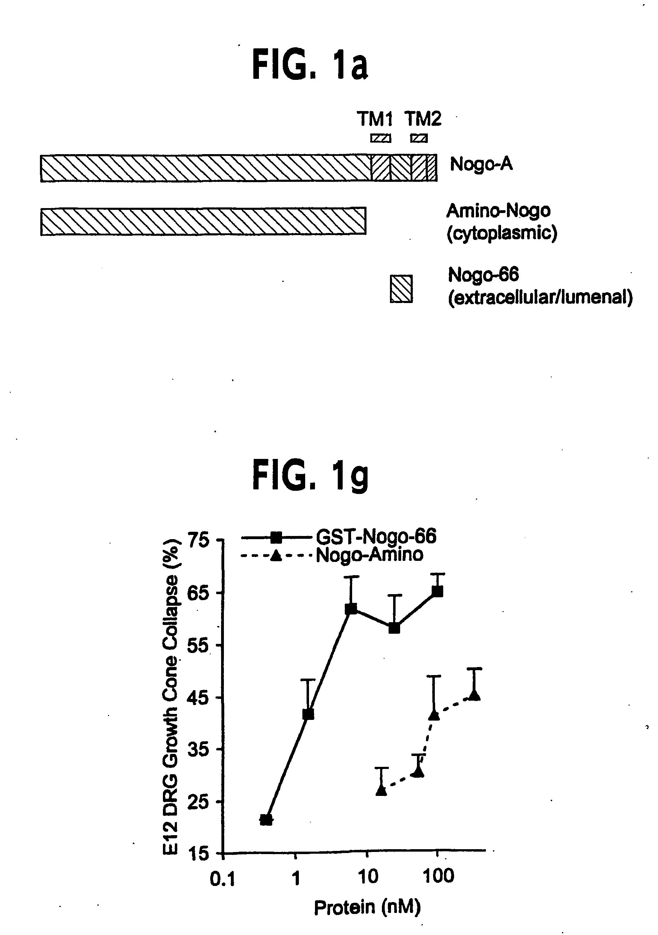

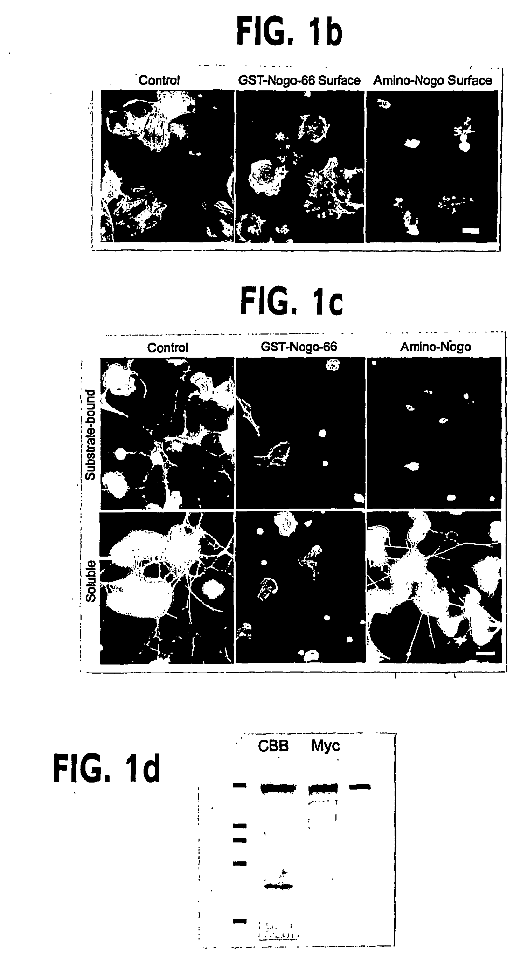

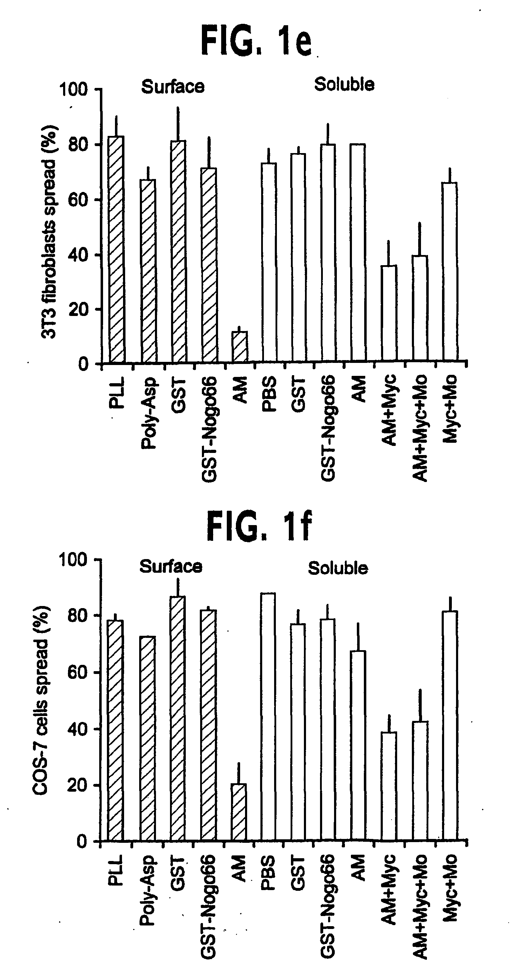

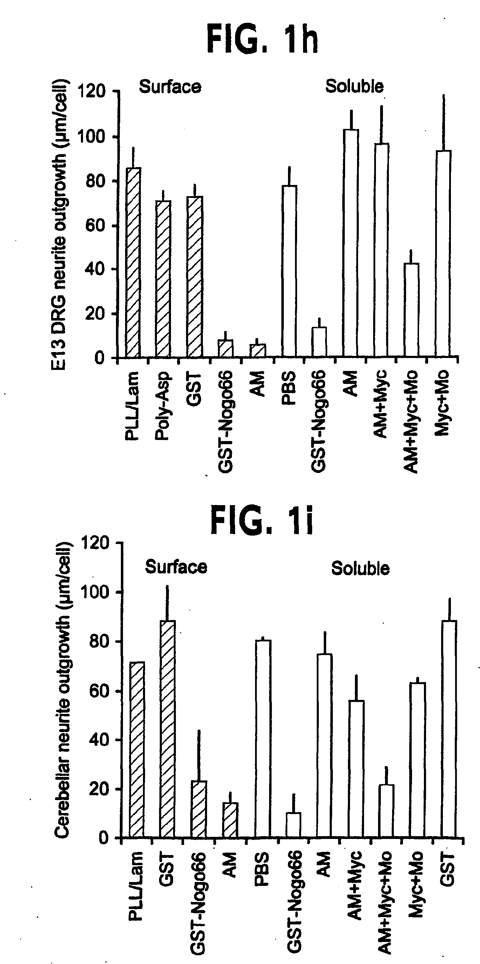

### Figure 1 - Comparison of Nogo domains

(a) is a schematic diagram which summarizes features of the Nogo proteins utilized in this study. (b) is a photograph of NIH-3T3 fibroblasts cultured on surfaces coated with Amino-Nogo, GST-Nogo-66 or no protein and stained for filamentous actin (scale bar, 40 µm). (c) is a photograph of chick E12 dorsal root ganglions cultured on surfaces coated with Amino-Nogo, GST-Nogo-66 or no protein (substrate-bound) or with 100 nM Nogo protein (soluble) (scale bar, 40 µm). (d) is a photograph of a gel and an immunoblot where purified Amino-Nogo-Myc-His protein was subjected to SDS-PAGE and stained with Commassie Brilliant Blue (CBB) or immunoblotted with anti-Myc antibodies (Myc) (molecular weight markers of 200, 116, 97, 65 & 45 kDa are at left). (e) is a graph displaying experimental data where the percentage of 3T3 fibroblasts with an area greater than 1200 µm² (spread) was measured from experiments as in (b) on Nogo-coated surfaces (black) or with soluble 100 nM Nogo preparations (blue) (AM, Amino-Nogo; AM+Myc, Amino-Nogo preincubated with anti-Myc antibody; AM+Myc+Mo, AM+Myc preincubated with anti-mouse IgG antibody; Myc+Mo, anti-Myc antibody plus anti-murine IgG antibody), (f) is a graph displaying experimental data where the percentage of spread COS-7 cells was determined after culture on Nogo-coated surfaces or with soluble 100 nM Nogo preparations. (g) is a graph displaying experimental data where the effects of purified preparations of GST-Nogo-66 or Amino-Nogo on growth cone morphology was assessed in E 12 dorsal root ganglion cultures at the indicated concentrations after thirty minutes. This demonstrates that GST-Nogo-66 is two orders of magnitude more potent than Amino-Nogo in this assay. (h) is a graph displaying experimental data where the neurite outgrowth per cell in E 13 dorsal root ganglion cultures was quantitated from experiments as in (c) on Nogo-coated surfaces or with soluble 100 nM Nogo preparations. (i) is a graph displaying experimental data where the effects of Nogo preparations on neurite outgrowth in cerebellar granule neurons was measured.

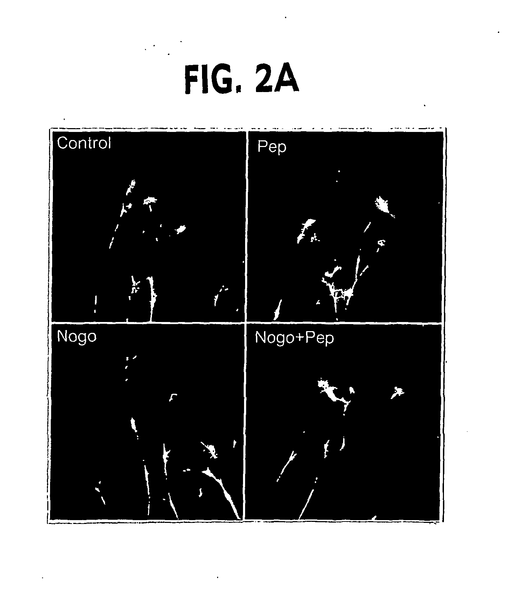

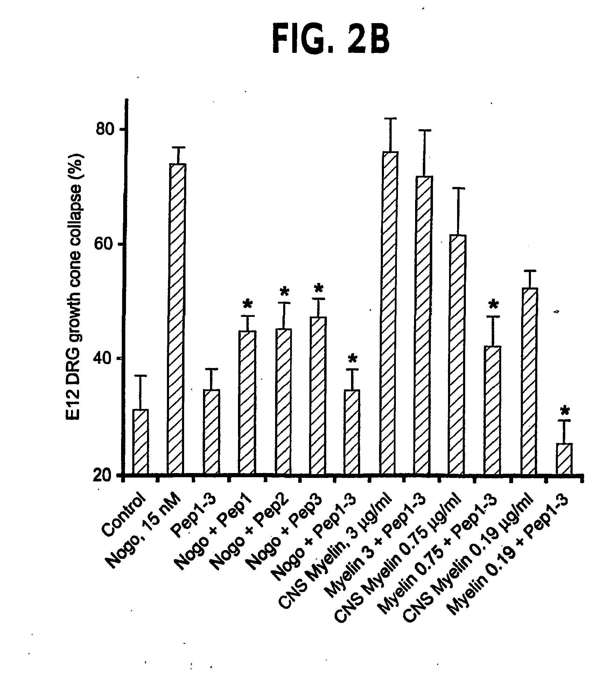

### Figure 2 - Nogo fragments antagonize Nogo and CNS myelin action

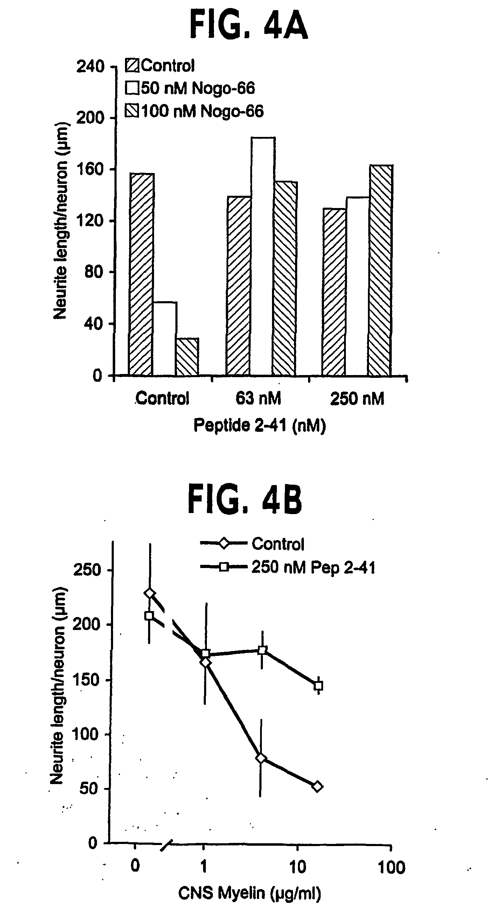

(a) is a photograph of chick E 12 dorsal root ganglion explants that were cultured and growth cone collapse assessed as described in Figure 4. Cultures were exposed to the following preparations for thirty minutes before fixation and staining with rhodamine-phalloidin: buffer only (Control); 15 nM GST-Nogo (Nogo); 1 µM each of Pep1, Pep2 and Pep3 (Pep); 15 nM GST-Nogo plus 1 µM each of Pep1, Pep2 and Pep3 (Nogo + Pep). Note that growth cone collapse by Nogo is blocked by peptide addition. Pep1, residues 1-25 of the extracellular domain; Pep2, 11-35; and Pep3, 21-45. (b) is a graph quantifying the results from growth cone collapse assays as in (a). Individual peptides were included at 4 µM, and the peptide 1-3 mixture was 1 µM of each peptide. CNS myelin was prepared as described and the indicated total myelin protein concentrations were included in the cultures. All results are the means ± s.e.m. calculated from four to seven determinations. Those values significantly different from the corresponding values with the same concentration of Nogo or myelin but without peptide are indicated (asterisk, p < 0.05, Student's two-tailed t test).

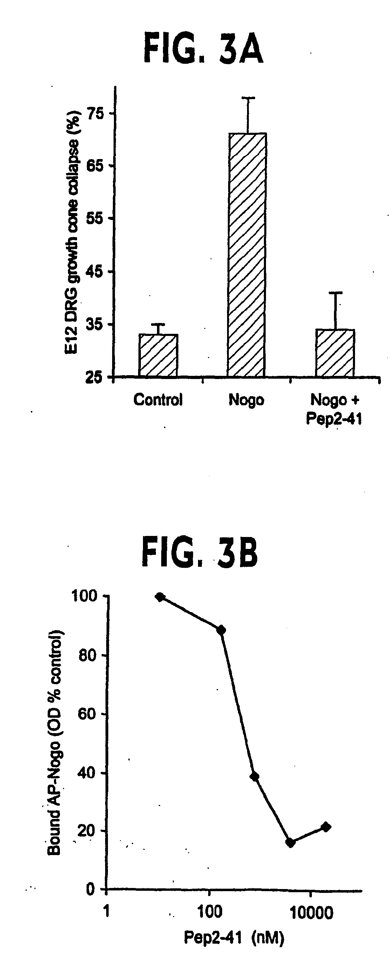

### Figure 3 - Nogo antagonist Pep2-41

(a) is a graph displaying the results of chick E12 dorsal root ganglion growth cone collapse assays. These assays were performed and quantified as in GrandPré et al., (2000) Nature 403, 439-444. Assays were conducted with no addition (Control), 15 nM GST-Nogo (Nogo) or 15 nM GST-Nogo plus 1 µM Pep2-41 (Nogo - Pep). The values are means ± s.e.m. calculated from four determinations. (b) is a graph displacing the results of binding experiments where binding of 10 nM AP-Nogo to chick E12 dorsal root ganglion neurons was measured as described in Figure 4 with the addition of the indicated concentrations of Pep2-41.

### Figure 4 - Nogo Pep2-41 prevents both Nogo & CNS myelin inhibition of neurite outgrowth

This figure is a graph which displays the results of outgrowth assays where neurons were cultured in the presence of the indicated concentrations of Pep2-41, purified GST-Nogo (GST-Nogo-66) protein and crude CNS myelin protein. Chick E13 dorsal root ganglion neurons were cultured under standard conditions. For outgrowth assays, neurons were cultured in the presence of the indicated concentrations of Pep2-41, purified GST-Nogo (GST-Nogo-66) protein and crude CNS myelin protein. This demonstrates that Pep2-41 can reverse the inhibition of neurite outgrowth by either GST-Nogo or total CNS myelin.

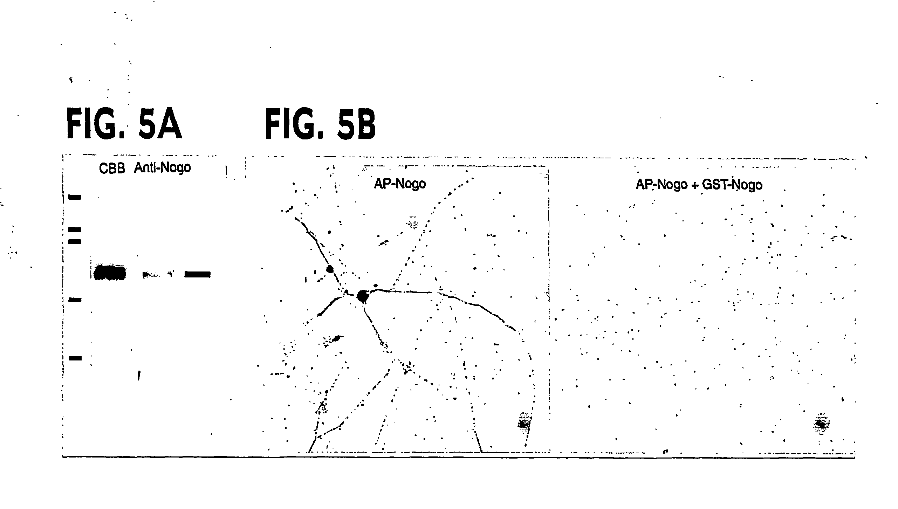

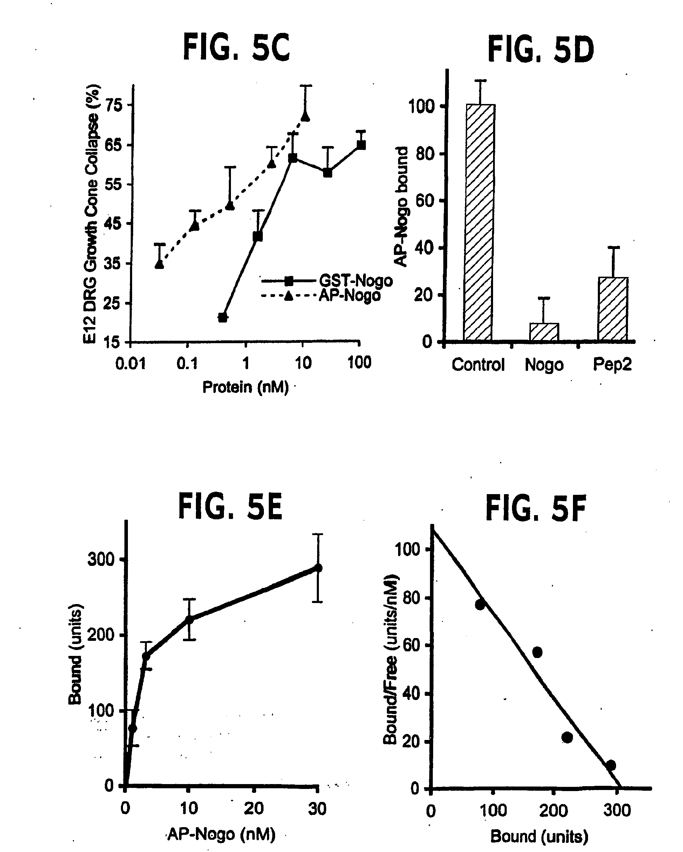

### Figure 5 - Ligand binding assay for axonal Nogo receptors

(a) is a photograph of a gel and an immunoblot where the His-AP-Nogo (66 amino acid) protein was expressed in HEK293T cells, and purified from conditioned medium on a Nickel-containing resin via the His tag. Purified protein was subjected to SDS-PAGE and stained for total protein with CBB or immunoblotted with anti-Nogo antibodies (anti-Nogo). Molecular weight markers of 200, 116, 97, 65 and 45 kDa are shown at left, and the migration of AP-Nogo at right. (b) is a photograph of dissociated chick E12 dorsal root ganglion neurons that were incubated with 10 nM AP-Nogo or 10 nM AP-Nogo + 160 nM GST-Nogo for sixty minutes at 23°C. The cells were washed, fixed and incubated at 60°C in order to inactivate endogenous AP. Bound AP-Nogo was detected by incubation with nitro blue tetrazolium. Note the intense neuronal staining by AP-Nogo that is displaced by unlabeled ligand. (c) is a graph displaying experimental data where the potency of AP-Nogo and GST-Nogo in E12 chick dorsal root ganglion growth cone collapse assays was assessed as described in the Example section. The EC₅₀ of AP-Nogo was determined to be 1 nM or less. The means ± s.e.m. calculated from five to eight determinations are illustrated. (d) is a graph displaying experimental data where the binding of 10 nM AP-Nogo to chick E12 dorsal root ganglion neurons was assessed alone, or in the presence of 100 nM GST-Nogo or in the presence of 4 µM Pep2, which was quantified from experiments as in (b) by the method described in the Example section. The means ± s.e.m. calculated from eight determinations are shown. (e) is a graph displaying experimental data where AP-Nogo binding to dorsal root ganglion neurons was measured as a function of AP-Nogo concentration. This is one of six experiments with similar results. (f) is a graph summarizing the data from (e) replotted for Scatchard analysis. The apparent K_{d} for AP-Nogo binding to E12 chick dorsal root ganglion neurons is 3 nM.

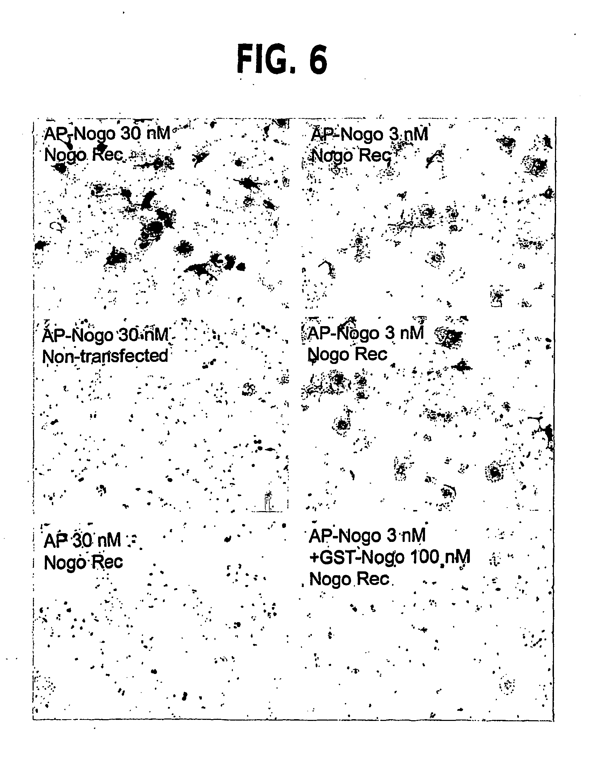

### Figure 6 - Nogo binding to COS-7 expressing the Nogo receptor

This figure is a photograph of COS-7 cells that were transfected with an expression vector encoding the murine Nogo receptor. Two days after transfection, binding of AP-Nogo or AP was assessed as described in the Example section for dorsal root ganglion neurons. Note the selective binding of AP-Nogo to Nogo receptor expressing cells. Binding is greatly reduced in the presence of excess Nogo peptide not fused to AP.

### Figure 7 - Structure of the Nogo receptor

This schematic diagram illustrates the major structural features of the Nogo receptor.

### Figure 8 - Distribution of Nogo receptor mRNA.

This figure is a photograph of Northern blot of Nogo receptor mRNA for polyA+ RNA samples from the indicated murine tissues on the left and for total RNA samples from various rat brain regions on the right. The migration of RNA size markers is shown at left.

### Figure 9 - Nogo-66 Receptor Immunohistology

(a) is a photograph of an immunoblot where membrane fractions (10 µg protein) from the indicated cells or chick tissues were analyzed by anti-Nogo-66 receptor immunoblot (molecular weight markers in kDa are at right). (b) is a photograph of COS-7 cells expressing Myc-Nogo-66 receptor or chick E5 spinal cord explants (eight days *in vitro*) stained with anti-Nogo-66 receptor, anti-Myc or the oligodendrocyte-specific 04 antibody. The bottom three panels show double label immunohistochemistry of the same field (scale bar, 40 µm for the top three panels and 80 µm for the bottom three panels). (c) is a photograph of paraformaldehyde-fixed vibratome sections of adult brain or spinal cord stained with the anti-Nogo-66 receptor preparation. This demonstrates staining of axonal profiles (arrows) in both the pons and spinal cord. Staining is dramatically reduced in the presence of 10 µg/ml GST-Nogo-66 receptor antigen.

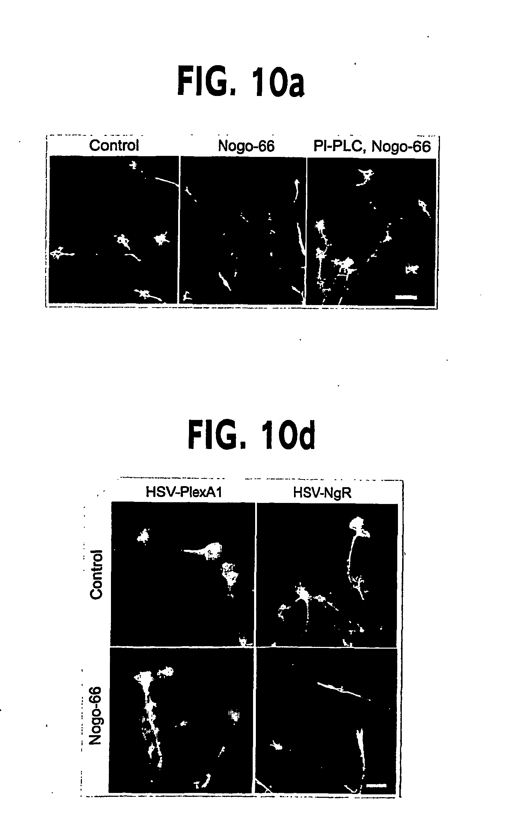

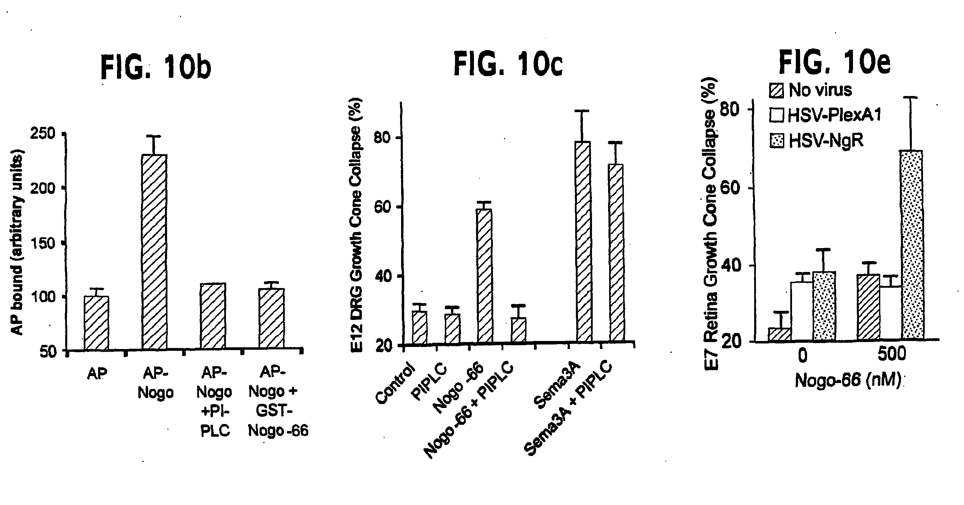

### Figure 10 - Nogo-66 Receptor mediates growth cone collapse by Nogo-66

(a) is a photograph of chick E 12 DRG explants exposed to Nogo-66 following pre-treatment with PI-PLC or buffer. Staining of F-actin in axons is illustrated (scale bar, 40 µm). (b) is a graph summarizing the experimental results of binding of 3 nM AP or AP-Nogo to chick E12 dorsal root ganglion dissociated neurons. Where indicated the cultures were pre-treated with PI-PLC or 150 nM GST-Nogo-66 was included in the incubation with AP-Nogo. (c) is a graph summarizing growth cone collapse measurements from experiments as in (a). Chick E12 DRG cultures were treated with or without PI-PLC prior to exposure to 30 nM GST-Nogo-66 or 100 pM Sema3A.(d) is a photograph of E7 retinal ganglion cell explants infected with a control virus (HSV-PlexinA1) or with HSV-Myc-Nogo-66 receptor and then incubated with or without Nogo-66. Phalloidin staining of axonal growth cones is illustrated (scale bar, 25 µm). (e) is a graph quantitating growth cone collapse in uninfected, or viral infected E7 retinal neurons as in (d).

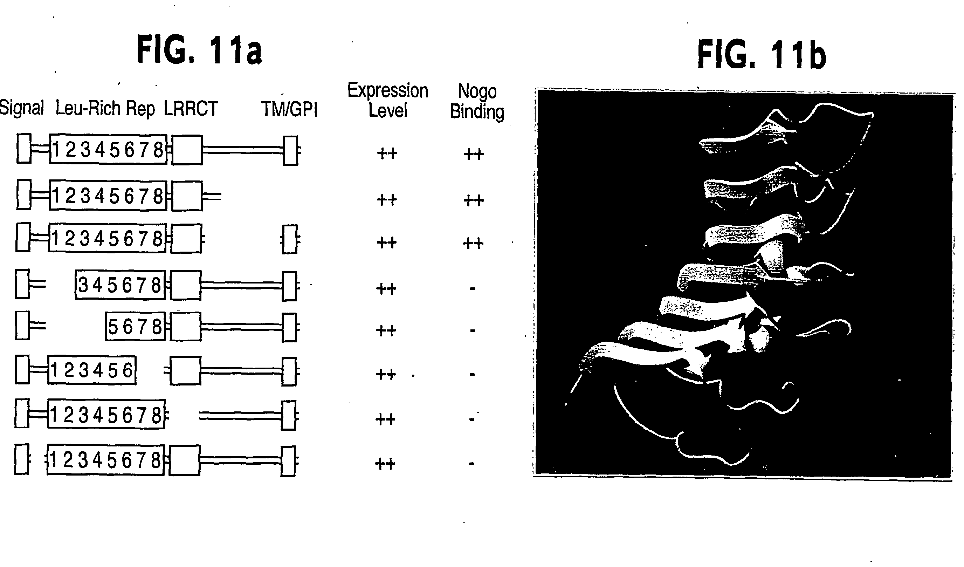

### Figure 11 - Structure-function analysis of Nogo-66 receptor

(a) is a schematic diagram of different Nogo-66 receptor deletion mutants. These mutants were assessed for level of expression by immunoblot and for AP-Nogo binding. Note that the leucine rich repeats and the leucine rich repeat carboxy terminal are required for Nogo binding but the remainder of the protein is not. The second protein was tested after purification and immobilization. (b) is a diagram of the predicted three dimensional structure for the first seven leucine rich repeats of the Nogo-66 receptor. This is derived from computer modeling based on the predicted structure of the related leucine rich repeats of the leutropin receptor (Jiang et al., (1995) Structure 3, 1341-1353). Modeling is performed by Swiss-Model at www.expasy.ch/spdbv. Those regions with beta sheet and alpha helix secondary structure are also indicated.

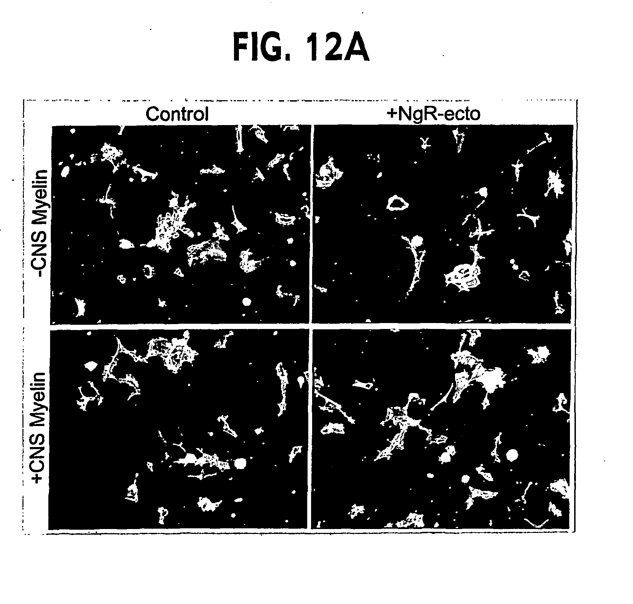

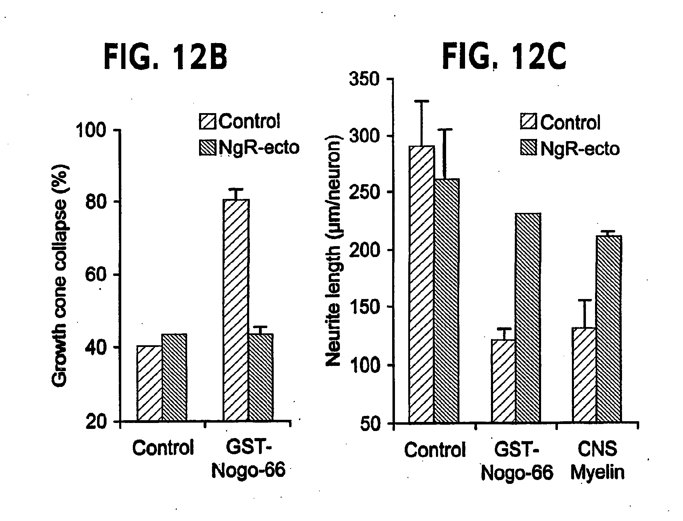

### Figure 12 - Soluble Nogo receptor blocks Nogo-66

Chick E13 DRG neurons cultured under standard conditions. In growth cone collapse assays, conditioned medium from HEK 293T cells secreting the 1-348 amino acid ectodomain fragment of the murine Nogo receptor or control conditioned medium was added together with 100 nM Nogo-66. In the bottom left panel, the data in the graph demonstrates that Nogo-induced collapse is blocked by the soluble receptor fragment. For outgrowth assays, neurons were cultured in the presence of control or Nogo receptor ectodomain conditioned medium together with Nogo-66 protein (50 nM) or central nervous system myelin (15 µg total protein/ml). The top four panels show photographs demonstrating that central nervous system myelin inhibits outgrowth and that this is blocked by the presence the Nogo receptor ectodomain protein. Outgrowth is quantitated in the graph in the bottom right panel.

### DETAILED DESCRIPTION OF THE INVENTION

### I. Definitions

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described.

As used herein, the term "axon" refers to a long cellular protrusion from a neuron, whereby efferent (outgoing) action potentials are conducted from the cell body towards target cells.

As used herein, the term "axonal growth" refers to an extension of the long process or axon, originating at the cell body and preceded by the growth cone.

As used herein, the term "central nervous system disorder" refers to any pathological state associated with abnormal function of the central nervous system (CNS). The term includes, but is not limited to, altered CNS function resulting from physical trauma to cerebral tissue, viral infection, autoimmune mechanism, genetic mutation and neurodegenerative diseases or disorders.

As used herein, the term "chimeric protein" refers to any polypeptide which is not completely homologous at the amino acid level to its wild-type sequence or is encoded by a nucleic acid which is derived from splicing two distinct sources of nucleic acids. The term includes, but is not limited to, fusion proteins and proteins designed to contain one or more amino acid substitutions which distinguishes their amino acid sequence from the wild type sequence.

As used herein, the term "demyelinating disease" refers to a pathological disorder characterized by the degradation of the myelin sheath of the oligodendrocyte cell membrane.

As used herein, the term "growth cone" refers to a specialized region at the tip of a growing neurite that is responsible for sensing the local environment and moving the axon toward its appropriate synaptic target cell.

As used herein, the term "growth cone movement" refers to the extension or collapse of the growth cone toward a neuron's target cell.

As used herein, the term "neurite" refers to a process growing out of a neuron. As it is sometimes difficult to distinguish a dendrite from an axon in culture, the term neurite is used for both.

As used herein, the term "oligodendrocyte" refers to a neuroglial cell of the CNS whose function is to myelinate CNS axons.

As used herein, the term "polypeptide" refers to a peptide which on hydrolysis yields more than two amino acids, called tripeptides, tetrapeptides, etc. according to the number of amino acids contained in the polypeptide. The term "polypeptide" is used synonomously with the term "protein" and "peptide"throughout the specification.

### II. Specific Aspects of this Disclosure

### A. Nogo receptor Protein and Peptide Agents for the Nogo receptor Protein

The present disclosure provides isolated protein, allelic variants of the protein, and conservative amino acid substitutions of the protein. As used herein, the protein or polypeptide refers to a Nogo receptor protein that has the human amino acid sequence depicted in SEQ ID NO: 2 or the murine amino acid sequence depicted in SEQ ID NO: 4. The protein or polypeptide also refers to the peptides identified as Nogo receptor peptide agents that have the amino acid sequences depicted in SEQ ID NO: 8, 10, 12, 14, 16, 18 and 20. The disclosure also includes naturally occurring allelic variants and proteins that have a slightly different amino acid sequence than that specifically recited above. Allelic variants, though possessing a slightly different amino acid sequence than those recited above, will still have the same or similar biological functions associated with the human and murine Nogo receptor proteins and the Nogo receptor peptide agents depicted in SEQ ID NO: 2, 4, 8, 10, 12, 14, 16, 18 and 20.

As used herein, the family of proteins related to the Nogo receptor proteins refers to proteins that have been isolated from organisms in addition to humans and mice. The methods used to identify and isolate other members of the family of proteins related to the Nogo receptor proteins are described below.

The Nogo receptor proteins and peptide agents of the present disclosure are preferably in isolated form. As used herein, a protein or ligand is said to be isolated when physical, mechanical or chemical methods are employed to remove the protein from cellular constituents that are normally associated with the protein. A skilled artisan can readily employ standard purification methods to obtain an isolated protein or ligand.

The proteins of the present disclosure further include conservative variants of the proteins and ligands herein described. As used herein, a conservative variant refers to alterations in the amino acid sequence that do not adversely affect the biological functions of the protein. A substitution, insertion or deletion is said to adversely aspect the protein when the altered sequence prevents or disrupts a biological function associated with the protein. For example, the overall charge, structure or hydrophobic-hydrophilic properties of the protein can be altered without adversely affecting a biological activity. Accordingly, the amino acid sequence can be altered, for example to render the peptide more hydrophobic or hydrophilic, without adversely affecting the biological activities of the protein.

Ordinarily, the allelic variants, the conservative substitution variants, and the members of the protein family, will have an amino acid sequence having at least seventy-five percent amino acid sequence identity with the human and murine sequences set forth in SEQ ID NO: 2,4, 8,10,12,14,16,18 and 20, more preferably at least eighty percent, even more preferably at least ninety percent, and most preferably at least ninety-five percent. Identity or homology with respect to such sequences is defined herein as the percentage of amino acid residues in the candidate sequence that are identical with the known peptides, after aligning the sequences and introducing gaps, if necessary, to achieve the maximum percent homology, and not considering any conservative substitutions as part of the sequence identity. N-terminal, C-terminal or internal extensions, deletions, or insertions into the peptide sequence shall not be construed as affecting homology.

Thus, the proteins and peptides of the present disclosure include molecules comprising the amino acid sequence of SEQ ID NO: 2, 4, 8,10, 12,14,16, 18 or 20; fragments thereof having a consecutive sequence of at least about 3, 4, 5, 6, 10, 15, 20, 25, 30, 35 or more amino acid residues of the Nogo receptor proteins and peptide agents; amino acid sequence variants of such sequences wherein at least one amino acid residue has been inserted N- or C-terminal to, or within, the disclosed sequence; amino acid sequence variants of the disclosed sequences, or their fragment as defined above, that have been substituted by another residue. Contemplated variants further include those containing predetermined mutations by, *e.g*., homologous recombination, site-directed or PCR mutagenesis, and the corresponding proteins of other animal species, including but not limited to rabbit, rat, porcine, bovine, ovine, equine and non-human primate species, the alleles or other naturally occurring variants of the family of proteins; and derivatives wherein the protein has been covalently modified by substitution, chemical, enzymatic, or other appropriate means with a moiety other than a naturally occurring amino acid (for example, a detectable moiety such as an enzyme or radioisotope).

As described below, members of the family of proteins can be used: (1) to identify agents which modulate at least one activity of the protein, (2) in methods of identifying binding partners for the protein, (3) as an antigen to raise polyclonal or monoclonal antibodies, and 4) as a therapeutic agent.

### B. Nucleic Acid Molecules

The present disclosure further provides nucleic acid molecules that encode the proteins and peptides comprising the amino acid sequence of SEQ ID NO: 2, 4, 8, 10, 12,14,16,18 or 20 and the related proteins herein described, preferably in isolated form. As used herein, "nucleic acid" includes genomic **DNA,** cDNA, mRNA and antisense molecules, as well as nucleic acids based on alternative backbones or including alternative bases whether derived from natural sources or synthesized.

Homology or identity is determined by **BLAST** (**B**asic **L**ocal **A**lignment **S**earch **T**ool) analysis using the algorithm employed by the programs **blastp, blastn, blastx, tblastn** and **tblastx** (Karlin et al., (1990) Proc. Natl. Acad. Sci. USA 87, 2264-2268 and Altschul, (1993) J. Mol. Evol. 36, 290-300 ) which are tailored for sequence similarity searching. The approach used by the **BLAST** program is to first consider similar segments between a query sequence and a database sequence, then to evaluate the statistical significance of all matches that are identified and finally to summarize only those matches which satisfy a preselected threshold of significance. For a discussion of basic issues in similarity searching of sequence databases see Altschul et al., (1994) Nature Genetics 6, 119-129. The search parameters for **histogram, descriptions, alignments, expect** (*i.e*., the statistical significance threshold for reporting matches against database sequences), **cutoff, matrix** and **filter** are at the default settings. The default scoring matrix used by **blastp, blastx, tblastn,** and **tblastx** is the **BLOSUM62** matrix (Henikoff et al., (1992) Proc. Natl. Acad. Sci. USA 89, 10915-10919). Four blastn parameters were adjusted as follows: Q=10 (gap creation penalty); R=10 (gap extension penalty); wink=1 (generates word hits at every wink^{th} position along the query); and gapw=16 (sets the window width within which gapped alignments are generated). The equivalent **Blastp** parameter settings were Q=9; R=2; wink=1; and gapw=32. A **Bestfit** comparison between sequences, available in the GCG package version 10.0, uses DNA, parameters GAP=50 (gap creation penalty) and LEN=3 (gap extension penalty) and the equivalent settings in protein comparisons are GAP=8 and LEN=2.

As used herein, "high stringency conditions" means hybridization at 42°C in the presence of 50% formamide, followed by a first wash at 65°C with 2x SSC containing 1% sodium SDS, followed by a second wash at 65°C with 0.1 x SSC.

As used herein, a nucleic acid molecule is said to be "isolated" when the nucleic acid molecule is substantially separated from contaminant nucleic acid encoding other polypeptides from the source of nucleic acid.

The present disclosure further provides fragments of the encoding nucleic acid molecule. As used herein, a fragment of an encoding nucleic acid molecule refers to a portion of the entire protein encoding sequence. The size of the fragment will be determined by the intended use. For example, if the fragment is chosen so as to encode an active portion of the protein, the fragment will need to be large enough to encode the functional region(s) of the protein. If the fragment is to be used as a nucleic acid probe or PCR primer, then the fragment length is chosen so as to obtain a relatively small number of false positives during probing/priming.

Fragments of the encoding nucleic acid molecules of the present disclosure (*i.e*., synthetic oligonucleotides) that are used as probes or specific primers for the polymerase chain reaction (PCR) or to synthesize gene sequences encoding proteins of the disclosure can easily be synthesized by chemical techniques, for example, the phosphotriester method of Matteucci et al., (1981) J. Am. Chem. Soc.103, 3185-3191 or using automated synthesis methods. In addition, larger DNA segments can readily be prepared by well known methods, such as synthesis of a group of oligonucleotides that define various modular segments of the gene, followed by ligation of oligonucleotides to build the complete modified gene.

The encoding nucleic acid molecules of the present disclosure may further be modified so as to contain a detectable label for diagnostic and probe purposes. A variety of such labels are known in the art and can readily be employed with the encoding molecules herein described. Suitable labels include, but are not limited to, biotin, radiolabeled nucleotides and the like. A skilled artisan can employ any of the art known labels to obtain a labeled encoding nucleic acid molecule.

Modifications to the primary structure itself by deletion, addition, or alteration of the amino acids incorporated into the protein sequence during translation can be made without destroying the activity of the protein. Such substitutions or other alterations result in proteins having an amino acid sequence encoded by a nucleic acid falling within the contemplated scope of the present disclosure.

### C. Isolation of Other Related Nucleic Acid Molecules

As described above, the identification of the nucleic acid molecule having SEQ ID NO: 1,3,7,9,11,13,15,17 and 19 allows a skilled artisan to isolate nucleic acid molecules that encode other members of the Nogo receptor protein family in addition to the sequences herein described. Further, the presently disclosed nucleic acid molecules allow a skilled artisan to isolate nucleic acid molecules that encode other members of the family of Nogo receptor proteins and peptide agents.

Essentially, a skilled artisan can readily use the amino acid sequence of SEQ ID NO: 2, 4, 8,10 ,12, 4, 16, 18 and 20 or epitope-containing fragments thereof to generate antibody probes to screen expression libraries prepared from appropriate cells. Typically, polyclonal antiserum from mammals such as rabbits immunized with the purified protein (as described below) or monoclonal antibodies can be used to probe a mammalian cDNA or genomic expression library, such as lambda gtll library, to obtain the appropriate coding sequence for other members of the protein family. The cloned cDNA sequence can be expressed as a fusion protein, expressed directly using its own control sequences, or expressed by constructions using control sequences appropriate to the particular host used for expression of the enzyme.

Alternatively, a portion of a coding sequence herein described can be synthesized and used as a probe to retrieve DNA encoding a member of the protein family from any mammalian organism. Oligomers containing *e.g*., approximately 18-20 nucleotides (encoding about a six to seven amino acid stretch) can be prepared and used to screen genomic DNA or cDNA libraries to obtain hybridization under stringent conditions or conditions of sufficient stringency to eliminate an undue level of false positives.

Additionally, pairs of oligonucleotide primers can be prepared for use in a polymerase chain reaction (PCR) to selectively clone an encoding nucleic acid molecule. A PCR denature/anneal/extend cycle for using such PCR primers is well known in the art and can readily be adapted for use in isolating other encoding nucleic acid molecules.

### D. Recombinant DNA Molecules Containing a Nucleic Acid Molecule

The present disclosure further provides recombinant DNA molecules (rDNA) that contain a coding sequence, e.g. a coding sequence of the present invention. As used herein, a rDNA molecule is a DNA molecule that has been subjected to molecular manipulation. Methods for generating rDNA molecules are well known in the art, for example, see Sambrook et al., (1989) Molecular Cloning - A Laboratory Manual, Cold Spring Harbor Laboratory Press. In the preferred rDNA molecules, a coding DNA sequence is operably linked to expression control sequences and vector sequences.

The choice of vector and expression control sequences to which one of the protein family encoding sequences of the present disclosure is operably linked depends directly, as is well known in the art, on the functional properties desired (*e.g*., protein expression, and the host cell to be transformed). A vector of the present disclosure, e.g. a vector of the present invention, may be at least capable of directing the replication or insertion into the host chromosome, and preferably also expression, of the structural gene included in the rDNA molecule.

Expression control elements that are used for regulating the expression of an operably linked protein encoding sequence are known in the art and include, but are not limited to, inducible promoters, constitutive promoter, secretion signals, and other regulatory elements. Preferably, the inducible promoter is readily controlled, such as being responsive to a nutrient in the host cell's medium.

In one instance, the vector containing a coding nucleic acid molecule will include a prokaryotic replicon, *i.e*., a DNA sequence having the ability to direct autonomous replication and maintenance of the recombinant DNA molecule extra-chromosomally in a prokaryotic host cell, such as a bacterial host cell, transformed therewith. Such replicons are well known in the art. In addition, vectors that include a prokaryotic replicon may also include a gene whose expression confers a detectable marker such as a drug resistance Typical of bacterial drug resistance genes are those that confer resistance to ampicillin or tetracycline.

Vectors that include a prokaryotic replicon can further include a prokaryotic or bacteriophage promoter capable of directing the expression (transcription and translation) of the coding gene sequences in a bacterial host cell, such as *E. coli.* A promoter is an expression control element formed by a DNA sequence that permits binding of RNA polymerase and transcription to occur. Promoter sequences compatible with bacterial hosts are typically provided in plasmid vectors containing convenient restriction sites for insertion of a DNA segment of the present disclosure. Examples of such vector plasmids are pUC8, pUC9, pBR322 and pBR329 (Biorad Laboratories), pPL and pKK223 (Pharmacia). Any suitable prokaryotic host can be used to express a recombinant DNA molecule encoding a protein of the disclosure.

Expression vectors compatible with eukaryotic cells, preferably those compatible with vertebrate cells, can also be used to form rDNA molecules that contain a coding sequence. Eukaryotic cell expression vectors are well known in the art and are available from several commercial sources. Typically, such vectors are provided containing convenient restriction sites for insertion of the desired DNA segment. Examples of such vectors are pSVL and pKSV-10 (Pharmacia), pBPV-1, pML2d (International Biotechnologies), pTDT1 (ATCC 31255) and the like eukaryotic expression vectors.

Eukaryotic cell expression vectors used to construct the rDNA molecules of the present disclosure may further include a selectable marker that is effective in a eukaryotic cell, preferably a drug resistance selection marker. A preferred drug resistance marker is the gene whose expression results in neomycin resistance, *i.e*., the neomycin phosphotransferase (*neo*) gene. (Southern et al., (1982) J. Mol. AnaL Genet. 1, 327-341). Alternatively, the selectable marker can be present on a separate plasmid, the two vectors introduced by co-transfection of the host cell, and transfectants selected by culturing in the appropriate drug for the selectable marker.

### E. Host Cells Containing an Exogenously Supplied Coding Nucleic Acid Molecule

The present disclosure further provides host cells transformed with a nucleic acid molecule that encodes a protein of the present disclosure, e.g. a protein of the present invention. The host cell can be either prokaryotic or eukaryotic. Eukaryotic cells useful for expression of a protein of the disclosure are not limited, so long as the cell line is compatible with cell culture methods and compatible with the propagation of the expression vector and expression of the gene product. Preferred eukaryotic host cells include, but are not limited to, yeast, insect and mammalian cells, preferably vertebrate cells such as those from a mouse, rat, monkey or human cell line. Examples of useful eukaryotic host cells include Chinese hamster ovary (CHO) cells available from the ATCC as CCL61, NIH Swiss mouse embryo cells NIH-3T3 available from the ATCC as CRL1658, baby hamster kidney cells (BHK), and the like eukaryotic tissue culture cell lines.

Transformation of appropriate cell hosts with a rDNA molecule of the present disclosure is accomplished by well known methods that typically depend on the type of vector used and host system employed. With regard to transformation of prokaryotic host cells, electroporation and salt treatment methods can be employed (see, for example, Sambrook et al., (1989) Molecular Cloning - A Laboratory Manual, Cold Spring Harbor Laboratory Press; Cohen et al., (1972) Proc. Natl. Acad. Sci. USA 69, 2110-2114). With regard to transformation of vertebrate cells with vectors containing rDNA, electroporation, cationic lipid or salt treatment methods can be employed (see, for example, Graham et al., (1973) Virology 52, 456-467; Wigler et al., (1979) Proc. Natl. Acad. Sci. USA 76, 1373-1376).

Successfully transformed cells, *i.e*., cells that contain a rDNA molecule of the present disclosure, can be identified by well known techniques including the selection for a selectable marker. For example, cells resulting from the introduction of an rDNA of the present disclosure can be cloned to produce single colonies. Cells from those colonies can be harvested, lysed and their DNA content examined for the presence of the rDNA using a method such as that described by Southern, (1975) J. Mol. BioL 98, 503-517 or the proteins produced from the cell assayed via an immunological method.

### F. Production of Recombinant Proteins using a rDNA Molecule

The present disclosure further provides methods for producing a protein of the disclosure using nucleic acid molecules herein described. In general terms, the production of a recombinant form of a protein typically involves the following steps:

First, a nucleic acid molecule is obtained that encodes a protein of the disclosure, such as the nucleic acid molecule depicted in SEQ ID NO: 1, 3, 7, 9, 11, 13,15, 17 and 19 or nucleotides 166-1584 of SEQ ID NO: 1 and nucleotides 178-1596 of SEQ ID NO: 3. If the encoding sequence is uninterrupted by introns, it is directly suitable for expression in any host.

The nucleic acid molecule is then preferably placed in operable linkage with suitable control sequences, as described above, to form an expression unit containing the protein open reading frame. The expression unit is used to transform a suitable host and the transformed host is cultured under conditions that allow the production of the recombinant protein. Optionally the recombinant protein is isolated from the medium or from the cells; recovery and purification of the protein may not be necessary in some instances where some impurities may be tolerated.

Each of the foregoing steps can be done in a variety of ways. For example, the desired coding sequences may be obtained from genomic fragments and used directly in appropriate hosts. The construction of expression vectors that are operable in a variety of hosts is accomplished using appropriate replicons and control sequences, as set forth above. The control sequences, expression vectors, and transformation methods are dependent on the type of host cell used to express the gene and were discussed in detail earlier. Suitable restriction sites can, if not normally available, be added to the ends of the coding sequence so as to provide an excisable gene to insert into these vectors. A skilled artisan canreadily adapt any host/expression system known in the art for use with the nucleic acid molecules of the disclosure to produce recombinant protein.

### G. Methods to Identity Binding Partners

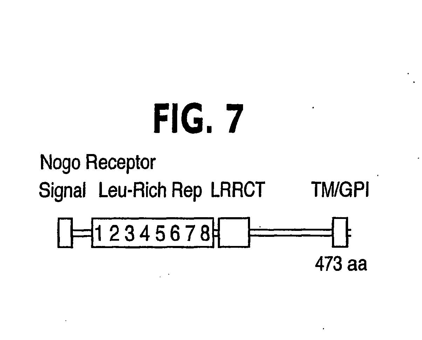

The present disclosure provides methods for use in isolating and identifying binding partners of proteins of the disclosure. In some instances, a protein of the disclosure is mixed with a potential binding partner or an extract or fraction of a cell under conditions that allow the association of potential binding partners with the protein of the disclosure. After mixing, peptides, polypeptides, proteins or other molecules that have become associated with a protein of the disclosure are separated from the mixture. The binding partner bound to the protein of the disclosure can then be removed and further analyzed. To identify and isolate a binding partner, the entire protein, for instance the entire Nogo receptor protein of either SEQ ID NO: 2 or 4 or the entire Nogo protein of SEQ ID NO: 6 can be used. Alternatively, a fragment of the protein can be used. An example of a useful Nogo receptor protein fragment is a soluble Nogo receptor polypeptide that lacks a transmembrane domain (Figure 7).

As used herein, a cellular extract refers to a preparation or fraction which is made from a lysed or disrupted cell. The preferred source of cellular extracts will be cells derived from human brain or spinal cord tissue, for instance, human cerebral tissue. Alternatively, cellular extracts may be prepared from any source of neuronal tissue or available neuronal cell lines, particularly olgiodendrocyte derived cell lines.

A variety of methods can be used to obtain an extract of a cell. Cells can be disrupted using either physical or chemical disruption methods. Examples of physical disruption methods include, but are not limited to, sonication and mechanical shearing. Examples of chemical lysis methods include, but are not limited to, detergent lysis and enzyme lysis. A skilled artisan can readily adapt methods for preparing cellular extracts in order to obtain extracts for use in the present methods.

Once an extract of a cell is prepared, the extract is mixed with the protein of the disclosure under conditions in which association of the protein with the binding partner can occur. A variety of conditions can be used, the most preferred being conditions that closely resemble conditions found in the cytoplasm of a human cell. Features such as osmolarity, pH, temperature, and the concentration of cellular extract used, can be varied to optimize the association of the protein with the binding partner.

After mixing under appropriate conditions, the bound complex is separated from the mixture. A variety of techniques can be utilized to separate the mixture. For example, antibodies specific to a protein of the disclosure can be used to immunoprecipitate the binding partner complex. Alternatively, standard chemical separation techniques such as chromatography and density-sediment centrifugation can be used.

After removal of non-associated cellular constituents found in the extract, the binding partner can be dissociated from the complex using conventional methods. For example, dissociation can be accomplished by altering the salt concentration or pH of the mixture.

To aid ia separating associated binding partner pairs from the mixed extract, the protein of the disclosure can be immobilized on a solid support. For example, the protein can be attached to a nitrocellulose matrix or acrylic beads. Attachment of the protein to a solid support aids in separating peptide-binding partner pairs from other constituents found in the extract. The identified binding partners can be either a single protein or a complex made up of two or more proteins. Alternatively, binding partners may be identified using the Alkaline Phosphatase fusion assay according to the procedures of Flanagan & Vanderhaeghen, (1998) Annu. Rev. Neurosci. 21, 309-345 or Takahashi et al., (1999) Cell 99, 59-69; the Far-Western assay according to the procedures of Takayama et al., (1997) Methods Mol. Biol. 69, 171-184 or Sauder et al., J. Gen. Virol. (1996) 77, 991-996 or identified through the use of epitope tagged proteins or GST fusion proteins.

Alternatively, the nucleic acid molecules of the disclosure can be used in a yeast two-hybrid system. The yeast two-hybrid system has been used to identify other protein partner pairs and can readily be adapted to employ the nucleic acid molecules herein described (see Stratagene Hybrizap® two-bybrid system).

### H. Methods to Identify Agents that Modulate Expression

The present disclosure provides methods for identifying agents that modulate the expression of a nucleic acid encoding the Nogo receptor protein. The present disclosure also provides methods for identifying agents that modulate the expression of a nucleic acid encoding the Nogo protein. Such assays may utilize any available means of monitoring for changes in the expression level of the nucleic acids of the disclosure. As used herein, an agent is said to modulate the expression of a nucleic acid of the disclosure, for instance a nucleic acid encoding the protein having the sequence of SEQ ID NO: 2, 4 or 6, if it is capable of up- or down-regulating expression of the nucleic acid in a cell.

In one assay format, cell lines that contain reporter gene fusions between the open reading frame defined by nucleotides 166-1584 of SEQ ID NO: 1, or nucleotides 178-1596 of SEQ ID NO: 3, or nucleotides 135-3713 of SEQ ID NO: 5, and any assayable fusion partner may be prepared. Numerous assayable fusion partners are known and readily available, including the firefly luciferase gene and the gene encoding chloramphenicol acetyltransferase (Alam et al., (1990) Anal. Biochem. 188, 245-254). Cell lines containing the reporter gene fusions are then exposed to the agent to be tested under appropriate conditions and time. Differential expression of the reporter gene between samples exposed to the agent and control samples identifies agents which modulate the expression of a nucleic acid encoding the protein having the sequence of SEQ ID NO: 2, 4 or 6.

Additional assay formats may be used to monitor the ability of the agent to modulate the expression of a nucleic acid encoding a Nogo receptor protein of the disclosure such as the protein having the amino acid sequence of SEQ ID NO: 2 or 4 or a Nogo protein having the amino acid sequence of SEQ ID NO: 6. For instance, mRNA expression may be monitored directly by hybridization to the nucleic acids of the disclosure. Cell lines are exposed to the agent to be tested under appropriate conditions and time and total RNA or mRNA is isolated by standard procedures such those disclosed in Sambrook et al., (1989) Molecular Cloning - A Laboratory Manual, Cold Spring Harbor Laboratory Press.

Probes to detect differences in RNA expression levels between cells exposed to the agent and control cells may be prepared from the nucleic acids of the disclosure. It is preferable, but not necessary, to design probes which hybridize only with target nucleic acids under conditions of high stringency. Only highly complementary nucleic acid hybrids form under conditions of high stringency. Accordingly, the stringency of the assay conditions determines the amount of complementarity which should exist between two nucleic acid strands in order to form a hybrid. Stringency should be chosen to maximize the difference in stability between the probe:target hybrid and potential probe:non-target hybrids.

Probes may be designed from the nucleic acids of the disclosure through methods known in the art. For instance, the G+C content of the probe and the probe length can affect probe binding to its target sequence. Methods to optimize probe specificity are commonly available in Sambrook et al., (1989) Molecular Cloning - A Laboratory Manual, Cold Spring Harbour Laboratory Press or Ausubel et al., (1995) Current Protocols in Molecular Biology, Greene Publishing.

Hybridization conditions are modified using known methods, such as those described by Sambrook *et al.,* (1989) and Ausubel *et al.,* (1995) as required for each probe. Hybridization of total cellular RNA or RNA enriched for polyA+ RNA can be accomplished in any available format. For instance, total cellular RNA or RNA enriched forpolyA+ RNA can be affixed to a solid support and the solid support exposed to at least one probe comprising at least one, or part of one of the sequences of the disclosure under conditions in which the probe will specifically hybridize. Alternatively, nucleic acid fragments comprising at least one, or part of one of the sequences of the disclosure can be affixed to a solid support, such as a silicon based wafer or a porous glass wafer. The wafer can then be exposed to total cellular RNA or polyA+ RNA from a sample under conditions in which the affixed sequences will specifically hybridize. Such wafers and hybridization methods are widely available, for example, those disclosed by Beattie, (1995) WO9511755. By examining for the ability of a given probe to specifically hybridize to a RNA sample from an untreated cell population and from a cell population exposed to the agent, agents which up or down regulate the Expression of a nucleic acid encoding the Nogo receptor protein having the sequence of SEQ ID NO: 2 or 4 are identified.

Hybridization for qualitative and quantitative analysis ofmRNA may also be carried out by using a RNase Protection Assay (*i.e*., RPA, see Ma et al., Methods (1996) 10, 273-238). Briefly, an expression vehicle comprising cDNA encoding the gene product and a phage specific DNA dependent RNA polymerase promoter (*e.g*., T7, T3 or SP6 RNA polymerase) is linearized at the 3' end of the cDNA molecule, downstream from the phage promoter, wherein such a linearized molecule is subsequently used as a template for synthesis of a labeled antisense transcript of the cDNA by *in vitro* transcription. The labeled transcript is then hybridized to a mixture of isolated RNA *(i.e.,* total or fractionated mRNA) by incubation at 45°C overnight in a buffer comprising 80% formamide, 40 mM Pipes, pH 6.4, 0.4 M NaCl and I mM EDTA. The resulting hybrids are then digested in a buffer comprising 40 µg/ml ribonuclease A and 2 µg/ml ribonuclease. After deactivation and extraction of extraneous proteins, the samples are loaded onto urea-polyacrylamide gels for analysis.

In another assay format, agents which effect the expression of the instant gene products, cells or cell lines would first be identified which express said gene products physiologically. Cells and cell lines so identified would be expected to comprise the necessary cellular machinery such that the fidelity of modulation of the transcriptional apparatus is maintained with regard to exogenous contact of agent with appropriate surface transduction mechanisms and the cytosolic cascades. Further, such cells or cell lines would be transduced or transfected with an expression vehicle (*e.g*., a plasmid or viral vector) construct comprising an operable non-translated 5'-promoter containing end of the structural gene encoding the instant gene products fused to one or more antigenic fragments, which are peculiar to the instant gene products, wherein said fragment are under the transcriptional control of said promoter and are expressed as polypeptides whose molecular weight can be distinguished from the naturally occurring polypeptides or may further comprise an immunologically distinct tag. Such a process is well known in the art (see, Sambrook et al., (1989) Molecular Cloning - A Laboratory Manual, Cold Spring Harbor Laboratory Press).

Cells or cell lines transduced or transfected as outlined above would then be contacted with agents under appropriate conditions; for example, the agent comprises a pharmaceutically acceptable excipient and is contacted with cells in an aqueous physiological buffer such as phosphate buffered saline (PBS) at physiological pH, Eagles balanced salt solution (BSS) at physiological pH, PBS or BSS comprising serum or conditioned media comprising PBS or BSS and serum incubated at 37°C. Said conditions may be modulated as deemed necessary by one of skill in the art. Subsequent to contacting the cells with the agent, said cells will be disrupted and the polypeptides of the disruptate are fractionated such that a polypeptide fraction is pooled and contacted with an antibody to be further processed by immunological assay (*e.g*., ELISA, immunoprecipitation or Western blot). The pool of proteins isolated from the "agent contacted" sample will be compared with a control sample where only the excipient is contacted with the cells and an increase or decrease in the immunologically generated signal from the "agent contacted" sample compared to the cool will be used to distinguish the effectiveness of the agent.

### I. Methods to Identify Agents that Modulate Activity

The present disclosure provides methods for identifying agents that modulate at least one activity of a Nogo receptor protein. The disclosure also provides methods for identifying agents that modulate at least one activity of a Nogo protein. Such methods or assays may utilize any means of monitoring or detecting the desired activity.

In one format, the specific activity of a Nogo receptor protein or Nogo protein, normalized to a standard unit, between a cell population that has been exposed to the agent to be tested compared to an un-exposed control cell population may be assayed. Cell lines or populations are exposed to the agent to be tested under appropriate conditions and time. Cellular lysates may be prepared from the exposed cell line or population and a control, unexposed cell line or population. The cellular lysates are then analyzed with the probe.

Antibody probes can be prepared by immunizing suitable mammalian hosts utilizing appropriate immunization protocols using the Nogo receptor protein, Nogo protein, Nogo receptor peptide agents or antigen-containing fragments of any of the foregoing. To enhance immunogenicity, these proteins or fragments can be conjugated to suitable carriers. Methods for preparing immunogenic conjugates with carriers such as BSA, KLH or other carrier proteins are well known in the art. In some circumstances, direct conjugation using, for example, carbodiimide reagents may be effective; in other instances linking reagents such as those supplied by Pierce Chemical Co. may be desirable to provide accessibility to the hapten. The hapten peptides can be extended at either the amino or carboxy terminus with a cysteine residue or interspersed with cysteine residues, for example, to facilitate linking to a carrier. Administration of the immunogens is conducted generally by injection over a suitable time period and with use of suitable adjuvants, as is generally understood in the art. During the immunization schedule, titers of antibodies are taken to determine adequacy of antibody formation.

While the polyclonal antisera produced in this way may be satisfactory for some applications, for pharmaceutical compositions, use of monoclonal preparations is preferred. Immortalized cell lines which secrete the desired monoclonal antibodies may be prepared using standard methods, see *e.g*., Kohler & Milstein, (1992) Biotechnology 24, 524-526 or modifications which effect immortalization of lymphocytes or spleen cells, as is generally known. The immortalized cell lines secreting the desired antibodies can be screened by immunoassay in which the antigen is the peptide hapten, polypeptide or protein. When the appropriate immortalized cell culture secreting the desired antibody is identified, the cells can be cultured either *in vitro* or by production in ascites fluid.

The desired monoclonal antibodies may be recovered from the culture supernatant or from the ascites supernatant. The intact anti-Nogo or anti-Nogo receptor antibodies or fragments thereof which contain the immunologically significant portion can be used as *e.g*., antagonists of binding between Nogo (ligand) and a Nogo receptor. Use of immunologically reactive fragments, such as the Fab, Fab' of F(ab')₂ fragments is often preferable, especially in a therapeutic context, as these fragments are generally less immunogenic than the whole immunoglobulin.

The antibodies or fragments may also be produced, using current technology, by recombinant means. Antibody regions that bind specifically to the desired regions of the protein can also be produced in the context of chimeras with multiple species origin.

Antibody regions that bind specifically to the desired regions of the protein can also be produced in the context of chimeras with multiple species origin, for instance, humanized antibodies. The antibody can therefore be a humanized antibody or human a antibody, as described in U.S. Patent 5,585,089 or Riechmann et al., (1988) Nature 332, 323-327.

Agents that are assayed in the above method can be randomly selected or rationally selected or designed. As used herein, an agent is said to be randomly selected when the agent is chosen randomly without considering the specific sequences involved in the association of the protein of the disclosure alone or with its associated substrates, binding partners, etc. An example of randomly selected agents is the use a chemical library or a peptide combinatorial library, or a growth broth of an organism.

As used herein, an agent is said to be rationally selected or designed when the agent is chosen on a non-random basis which takes into account the sequence of the target site or its conformation in connection with the agent's action. Agents can be rationally selected or rationally designed by utilizing the peptide sequences that make up these sites. For example, a rationally selected peptide agent can be a peptide whose amino acid sequence is identical to the binding domain (SEQ ID NO: 20) of Nogo which interacts with the Nogo receptor. Alternatively, it can be a fragment of the binding domain, *e.g.,* SEQ ID NO: 8, 10, 12, 14, 16 and 18.

The agents of the present disclosure can be, as examples, Peptides, antibodies, antibody fragments, small molecules, vitamin derivatives, as well as carbohydrates. Peptide agents of the disclosure can be prepared using standard solid phase (or solution phase) peptide synthesis methods, as is known in the art. In addition, the DNA encoding these peptides may be synthesized using commercially available oligonucleotide synthesis instrumentation and produced recombinantly using standard recombinant production systems. The production using solid phase peptide synthesis is necessitated if non-gene-encoded amino acids are to be included.

Another class of agents of the present disclosure are antibodies or fragments thereof that bind to a Nogo protein or Nogo receptor protein. Antibody agents can be obtained by immunization of suitable mammalian subjects with peptides, containing as antigenic regions, those portions of the protein intended to be targeted by the antibodies.

### J. High Throughput Assays

The power of high throughput screening is utilized to the search for new compounds which are capable of interacting with the Nogo receptor protein. For general information on high-throughput screening (*e.g*., Devlin. (1998) High Throughput Screening, Marcel Dekker; U.S. Patent 5,763,263). High throughput assays utilize one or more different assay techniques.

Immunodiagnostics and Immunoassays. These are a group of techniques used for the measurement of specific biochemical substances, commonly at low concentrations in complex mixtures such as biological fluids, that depend upon the specificity and high affinity shown by suitably prepared and selected antibodies for their complementary antigen. A substance to be measures must, of necessity, be antigenic -either an immunogenic macromolecule or a haptenic small molecule. To each sample a known, limited amount of specific antibody is added and the fraction of the antigen combining with it, often expressed as the bound:free ratio, is estimated, using as indicator a form of the antigen labeled with radioisotope (radioimmunoassay), fluorescent molecule (fluoroimmunoassay), stable free radical (spin immunoassay), enzyme (enzyme immunoassay), or other readily distinguishable label.

Antibodies can he labeled in various ways, including: enzyme-linked immunosorbent assay (ELISA); radioimmuno-assay (RLA); fluorescent immunoassay (FIA), chemiluminescent immunoassay (CLLA); and labeling the antibody with colloidal gold particles (immunogold).

Common assay formats include the sandwhich assay, competitive or competition assay, latex agglutination assay, homogeneous assay, microtitre plate format and the microparticle-based assay.

Enzyme-linked immunosorbent assay (ELISA). ELISA is an immunochemical technique that avoids the hazards of radiochemicals and the expense of fluorescence detection systems. Instead, the assay uses enzymes as indicators. ELISA is a form of quantitative immunoassay based on the use of antibodies (or antigens) that are linked to an insoluble carrier surface, which is then used to "capture" the relevant antigen (or antibody) in the test solution. The antigen-antibody complex is then detected by measuring the activity of an appropriate enzyme that had previously been covalently attached to the antigen (or antibody).

For information on ELISA techniques, see, for example, Crowther, (1995) ELISA - Theory and Practice (Methods in Molecular Biology), Humana Press; Challacombe & Kemeny, (1998) ELISA and Other Solid Phase Immunoassays - Theoretical and Practical Aspects, John Wiley; Kemeny, (1991) A Practical Guide to ELISA, Pergamon Press; Ishikawa. (1991) Ultrasensitive and Rapid Enzyme Immunoassay (Laboratory Techniques in Biochemistry and Molecular Biology) Elsevier.

Colorimetric Assays for Enzymes. Colorimetry is any method of quantitative chemical analysis in which the concentration or amount of a compound is determined by comparing the color produced by the reaction of a reagent with both standard and test amounts of the compound, *e.g*., using a colorimeter or a spectrophotometer.

Standard colorimetric assays of beta-galactosidase enzymatic activity are well known to those skilled in the art (see, for example, Norton et al., (1985) Mol. Cell. Biol. 5, 281-290). A colorimetric assay can be performed on whole cell lysates using O-nitrophenyl-beta-D-galactopyranoside (ONPG, Sigma) as the substrate in a standard colorimetric beta-galactosidase assay (Sambrook et al., (1989) Molecular Cloning - A Laboratory Manual, Cold Spring Harbor Laboratory Press. Automated colorimetric assays are also available for the detection of beta-galactosidase activity (see *e.g*., U.S. Patent 5,733,720).

Immunofluorescence Assays. Immunofluorescence or immunofluorescence microscopy is a technique in which an antigen or antibody is made fluorescent by conjugation to a fluorescent dye and then allowed to react with the complementary antibody or antigen in a tissue section or smear. The location of the antigen or antibody can then be determined by observing the fluorescence by microscopy under ultraviolet light.

For general information on immunofluorescent techniques, see, for example, Knapp et al., (1978) Immunofluorescence and Related Staining Techniques, Elsevier, Allan, (1999) Protein Localization by Fluorescent Microscopy - A Practical Approach (The Practical Approach Series) Oxford University Press; Caul, (1993) Immunofluorescence Antigen Detection Techniques in Diagnostic Microbiology, Cambridge University Press. For detailed explanations of immunofluorescent techniques applicable to the present disclosure, see U.S. Patent 5,912,176; U.S. Patent 5,869,264; U.S. Patent 5,866,319; and U.S. Patent 5,861,259.

### K. Uses for Agents that Modulate Activity

As provided in the Examples, the Nogo and Nogo receptor proteins and nucleic acids, such as the proteins having the amino acid sequence of SEQ ID NO: 2,4 or 6, are expressed in myelin derived from axon and dendrites. Agents that modulate or up- or down-regulate the expression of the Nogo or Nogo receptor protein or agents such as agonists or antagonists of at least one activity of the Nogo or Nogo receptor protein may be used to modulate biological and pathologic processes associated with the protein's function and activity. This aspect of the disclosure is particularly useful in the treatment of human subjects.

Pathological processes refer to a category of biological processes which produce a deleterious effect. For example, expression of a protein of the disclosure may be associated with inhibition of axonal regeneration following cranial, cerebral or spinal trauma, stroke or a demyelinating disease. Such demyelinating diseases include, but are not limited to, multiple sclerosis, monophasic demyelination, encephalomyelitis, multifocal leukoencephalopathy, panencephalitis, Marchiafava-Bignami disease, pontine myelinolysis, adrenoleukodystrophy, Pelizaeus-Merzbacher disease, Spongy degeneration, Alexander's disease, Canavan's disease, metachromatic leukodystrophy and Krabbe's disease. As used herein, an agent is said to modulate a pathological process when the agent reduces the degree or severity of the process. For instance, a demyelinating disease may be prevented or disease progression modulated by the administration of agents which reduce, promote or modulate in some way the expression or at least one activity of a protein of the disclosure.

In one example, administration of the Nogo peptide agents depicted in SEQ ID NO: 8, 10, 12, 14, 16, 8 and 20 can be used to treat a demyelinating disease associated with Nogo or the Nogo receptor protein. In another example, cells which express the peptide agents of the disclosure may be transplanted to a site spinal cord injury to facilitate axonal growth throughout the injured site. Such transplanted cells would provide a means for restoring spinal cord function following injury or trauma.

In yet another example, administration of soluble Nogo receptor protein that binds to Nogo can be used to treat a demyelinating disease associated with Nogo or the Nogo receptor protein. This agent can be used to prevent the binding of Nogo to cell bound Nogo receptor and act as an antagonist of Nogo. Soluble receptors have been used to bind cytokines or other ligands to regulate their function (Thomson, (1998) Cytokine Handbook, Academic Press). A soluble receptor occurs in solution, or outside of the membrane. Soluble receptors may occur because the segment of the molecule which spans or associates with the membrane is absent. This segment is commonly referred to in the art as the transmembrane domain of the gene, or membrane binding segment of the protein. Thus, in some instances of the disclosure, a soluble receptor includes a fragment or an analog of a membrane bound receptor. Preferably, the fragment contains at least six, *e.g.,* ten, fifteen, twenty, twenty-five, thirty, forty, fifty, sixty or seventy amino acids, provided it retains its desired activity.

In other instances of the disclosure, the structure of the segment that associates with the membrane is modified (*e.g*., DNA sequence polymorphism or mutation in the gene) so the receptor is not inserted into the membrane, or the receptor is inserted, but is not retained within the membrane. Thus, a soluble receptor, in contrast to the corresponding membrane bound form, differs in one or more segments of the gene or receptor protein that are important to its association with the membrane.

The agents of the present disclosure can be provided alone, or in combination, or in sequential combination with other agents that modulate a particular pathological process. For example, an agent of the present disclosure can be administered in combination with anti-inflammatory agents following stroke as a means for blocking further neuronal damage and inhibition of axonal regeneration. As used herein, two agents are said to be administered in combination when the two agents are administered simultaneously or are administered independently in a fashion such that the agents will act at die same time.

The agents of the present disclosure can be administered via parenteral, subcutaneous, intravenous, intramuscular, intraperitoneal, transdermal, or buccal routes. For example, an agent may be administered locally to a site of injury via microinfusion. Typical sites include, but are not limited to, damaged areas of the spinal cord resulting from injury or damaged sites in the brain resulting from a stroke. Alternatively, or concurrently, administration may be by the oral route. The dosage administered will be dependent upon the age, health, and weight of the recipient, kind of concurrent treatment, if any, frequency of treatment, and the nature of the effect desired.

The present disclosure further provides compositions containing one or more agents which modulate expression or at least one activity of a protein of the disclosure. While individual needs vary, determination of optimal ranges of effective amounts of each component is within the skill of the art. Typical dosages comprise 1 pg/kg to 100 mg/kg body weight. The preferred dosages for systemic administration comprise 100 ng/kg to 100 mg/kg body weight. The preferred dosages for direct administration to a site via microinfusion comprise 1 ng/kg to 1 µg/kg body weight.

In addition to the pharmacologically active agent, the compositions of the present disclosure may contain suitable pharmaceutically acceptable carriers comprising excipients and auxiliaries which facilitate processing of the active compounds into preparations which can be used pharmaceutically for delivery to the site of action. Suitable formulations for parenteral administration include aqueous solutions of the active compounds in water-soluble form, for example, water-soluble salts. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides. Aqueous injection suspensions may contain substances which increase the viscosity of the suspension include, for example, sodium carboxymethyl cellulose, sorbitol and dextran. Optionally, the suspension may also contain stabilizers. Liposomes can also be used to encapsulate the agent for delivery into the cell.

The pharmaceutical formulation for systemic administration according to the disclosure may be formulated for enteral, parenteral or topical administration. Indeed, all three types of formulations may be used simultaneously to achieve systemic administration of the active ingredient. Suitable formulations for oral administration include hard or soft gelatin capsules, pills, tablets, including coated tablets, elixirs, suspensions, syrups or inhalations and controlled release forms thereof.

In practicing the methods of this disclosure, the agents of this disclosure may be used alone or in combination, or in combination with other therapeutic or diagnostic agents, In certain preferred instances, the compounds of this disclosure may be co-administered along with other compounds typically prescribed for these conditions according to generally accepted medical practice, such as anti-inflammatory agents, anticoagulants, antitbrombotics, including platelet aggregation inhibitors, tissue plasminogen activators, urokinase, prourokinase, streptokinase, aspirin and heparin. The compounds of this disclosure can be utilized *in vivo*, ordinarily in mammals, such as humans, sheep, horses, cattle, pigs, dogs, cats, rats and mice, or *in vitro.*

### L. Peptide Mimetics.

This disclosure also includes peptide mimetics which mimic the three-dimensional structure of Nogo and block Nogo binding at the Nogo receptor. Such peptide mimetics may have significant advantages over naturally-occurring peptides, including, for example: more economical production, greater chemical stability, enhanced pharmacological properties (half-life, absorption, potency, efficacy, etc.), altered specificity (*e.g*., a broad-spectrum of biological activities), reduced antigenicity, and others.

In one form, mimetics are peptide-containing molecules that mimic elements of protein secondary structure. (see, for example, Johnson et al., (1993) Peptide Turn Mimetics, in Biotechnology and Pharmacy, Pezzuto et al., (editors) Chapman and Hall). The underlying rationale behind the use of peptide mimetics is that the peptide backbone of proteins exists chiefly to orient amino acid side chains in such a way as to facilitate molecular interactions, such as those of antibody and antigen. A peptide mimetic is expected to permit molecular interactions similar to the natural molecule.

In another form, peptide analogs are commonly used in the pharmaceutical industry as non-peptide drugs with properties analogous to those of the template peptide. These types of non-peptide compounds are also referred to as "peptide mimetics" or "peptidomimetics" (Fauchere, (1986) Adv. Drug Res. 15,29-69; Veber & Freidinger, (1985) Trends Neurosci. 8, 392-396; Evans et al., (1987) J. Med. Chem. 30, 1229-1239, which are incorporated herein by reference) and are usually developed with the aid of computerized molecular modeling.

Peptide mimetics that are structurally similar to therapeutically useful peptides may be used to produce an equivalent therapeutic or prophylactic effect. Generally, peptide mimetics are structurally similar to a paradigm polypeptide (*i.e*., a polypeptide that has a biochemical property or pharmacological activity), such as the extracellular domain of Nogo, but have one or more peptide linkages optionally replaced by a linkage selected from the group consisting of: -CH₂NH-, -CH₂S-, -CH₂-CH₂-, -CH=CH- (cis and trans), -COCH₂-, -CH(OH)CH₂- and -CH2SO-, by methods known in the art and further described in the following references; Weinstein, (1983) Chemistry and Biochemistry of Amino Acids, Peptides and Proteins, Marcel Dekker; Morley, (1980) Trends Pharmacol. Sci. 1, 463-468 (general review); Hudson et al., (1979) Int. J. Pept. Protein Res.14, 177-185 (-CH₂NH-, CH₂CH₂-); Spatola et al., (1986) Life Sci. 38, 1243-1249 (-CH₂,-S); Hann, (1982) J. Chem. Soc. Perkin Trans. 1,307-314 (-CH-CH-, cis and trans); Almquist et al., (1980) J. Med. Chem. 23,1392-1398 (-COCH₂-); Jennings-White et al., (1982) Tetrahedron Lett. 23, 2533 (-COCH2-); Holladay et al, (1983) Tetrahedron Lett. 24, 4401-4404 (-C(OH)CH₂-); and Hruby, (1982) Life Sci. 31, 189-199 (-CH₂S-).

Labeling of peptide mimetics usually involves covalent attachment of one or more labels, directly or through a spacer (*e.g*., an amide group), to non-interfering position(s) on the peptide mimetic that are predicted by quantitative structure-activity data and molecular modeling. Such non-interfering positions generally are positions that do not form direct contacts with the macromolecule(s) (*e.g*., are not contact points in Nogo-Nogo receptor complexes) to which the peptide mimetic binds to produce the therapeutic effect. Derivitization (*e.g.,* labeling) of peptide mimetics, should not substantially interfere with the desired biological or pharmacological activity of the peptide mimetic.

Nogo peptide mimetics can be constructed by structure-based drug design through replacement of amino acids by organic moieties (see, for example, Hughes, (1980) Philos. Trans. R. Soc. Lond. 290, 387-394; Hodgson, (1991) Biotechnol. 9,19-21; Suckling, (1991) Sci. Prog. 75, 323-359).

The use of peptide mimetics can be enhanced through the use of combinatorial chemistry to create drug libraries. The design of peptide mimetics can be aided by identifying amino acid mutations that increase or decrease binding of Nogo at the Nogo receptor. Approaches that can be used include the yeast two hybrid method (see Chien et al., (1991) Proc. Natl. Acad. Sci. USA 88, 9578-9582) and using the phage display method. The two hybrid method detects protein-protein interactions in yeast (Fields et al., (1989) Nature 340,245-246). The phage display method detects the interaction between an immobilized protein and a protein that is expressed on the surface of phages such as lambda and M13 (Amberg et al., (1993) Strategies 6, 2-4; Hogrefe et al., (1993) Gene 128, 119-126). These methods allow positive and negative selection for protein-protein interactions and the identification of the sequences that determine these interactions.

For general information on peptide synthesis and peptide mimetics, see, for example; Jones, (1992) Amino Acid and Peptide Synthesis, Oxford University Press; Jung, (1997) Combinatorial Peptide and Nonpeptide Libraries: A Handbook, John Wiley; Bodanszky et al., (1993) Peptide Chemistry - A Practical Textbook, Springer Verlag.

### M. Transgenic, Animals

The term "animal" as used herein includes all vertebrate animals, except humans. It also includes an individual animal in all stages of development, including embryonic and fetal stages. A "transgenic animal" is an animal containing one or more cells bearing genetic information received, directly or indirectly, by deliberate genetic manipulation at a subcellular level, such as by microinjection or infection with recombinant virus. This introduced DNA molecule may be integrated within a chromosome, or it may be extra-chromosomally replicating DNA. The term "germ cell-line transgenic animal" refers to a transgenic animal in which the genetic information was introduced into a germ line cell, thereby conferring the ability to transfer the information to offspring. If such offspring in fact possess some or all of that information, then they, too, are transgenic animals. Transgenic animals containing mutant, knock-out, modified genes or gene construct to over-express or conditionally express a gene corresponding to the cDNA sequences of SEQ ID NO: 1 or 3 or related sequences are encompassed in this disclosure.

The information may be foreign to the species of animal to which the recipient belongs, foreign only to the particular individual recipient, or genetic information already possessed by the recipient. In the last case, the introduced gene may be differently expressed compared to the native endogenous gene. The genes may be obtained by isolating them from genomic sources, by preparation of cDNA from isolated RNA templates, by directed synthesis, or by some combination thereof.

To be expressed, a gene should be operably linked to a regulatory region. Regulatory regions, such as promoters, may be used to increase, decrease, regulate or designate to certain tissues or to certain stages of development the expression of a gene. The promoter need not be a naturally occurring promoter. The "transgenic non-human animals'' of the disclosure are produced by introducing "transgenes" into the germline of the non-human animal. The methods enabling the introduction of DNA into cells are generally available and well-known in the art. Different methods of introducing transgenes could be used. Generally, the zygote is the beast target for microinjection. In the mouse, the male pronucleus reaches the size of approximately twenty microns in diameter, which allows reproducible injection of one to two picoliters of DNA solution. The use of zygotes as a target for gene transfer has a major advantage. In most cases, the injected DNA will be incorporated into the host gene before the first cleavage (Brinster et al., (1985) Proc. Natl. Acad. Sci. USA 82, 4438-4442). Consequently, nearly all cells of the transgenic non-human animal will carry the incorporated transgene. Generally, this will also result in the efficient transmission of the transgene to offspring of the founder since 50% of the gene cells will harbor the transgene. Microinjection of zygotes is a preferred method for incorporating transgenes in practicing this disclosure.

Retroviral infection can also be used to introduce a transgene into a non-human animal. The developing non-human embryo can be cultured *in vitro* to the blastocyst stage. During this time, blastomeres may be targets for retroviral infection. Efficient infection of the blastomeres is obtained by enzymatic treatment to remove the zona pellucida. The viral vector system used to introduce the transgene is typically a replication-defective retrovirus carrying the transgene (Jahner et al., (1985) Proc. Natl. Acad. Sci. USA 82, 6927-6931; Van der Putten et al., (1985) Proc. Natl. Acad. Sci. USA 82, 6148-6152). Transfection is easily and efficiently obtained by culturing the blastomeres on a monolayer of virus-producing cells (Van der Putten et al., (1985) Proc. Natl. Acad. Sci. USA 82, 6148-6152; Stewart et al., (1987) EMBO J. 6, 383-388). Alternatively, infection can be performed at a later stage. Virus or virus-producing cells can be injected into the blastocoele (Jahner et al., (1982) Nature 298, 623-628). Most of the founder animals will be mosaic for the transgene since incorporation occurs only in a subset of the cells which formed the transgenic non-human animal. Furthermore, the founder animal may contain retroviral insertions of the transgene at a variety of positions in the genome; these generally segregate in the offspring. In addition, it is also possible to introduce transgenes into the germ line, albeit with low efficiency, by intrauterine retroviral infection of the midgestation embryo (Jahner et al., (1982) Nature 298, 623-628).

A third type of target cell for transgene introduction is the embryonal stem cell (ES). ES cells are obtained from pre-implantation embryos cultured *in vitro (*Evans et al., (1981) Nature 292, 154-156; Bradley et al., (1984) Nature 309, 255-256; Gossler et al., (1986) Proc. Natl. Acad. Sci. USA 83, 9065-9069). Transgenes can be efficiently introduced into ES cells by DNA transfection or by retrovirus-mediated transduction. The resulting transformed ES cells can thereafter be combined with blastocysts from a non-human animal. The ES cells colonize the embryo and contribute to the germ line of the resulting chimeric animal.

The methods for evaluating the presence of the introduced DNA as well as its expression are readily available and well-known in the art. Such methods include, but are not limited to DNA (Southern) hybridization to detect the exogenous DNA, polymerase chain reaction (PCR), polyacrylamide gel electrophoresis (PAGE) and Western blots to detect DNA, RNA and protein. The methods include immunological and histochemical techniques to detect expression of a Nogo receptor gene.

As used herein, a "transgene" is a DNA sequence introduced into the germline of a non-human animal by way of human intervention such as by way of the Examples described below. The nucleic acid sequence of the transgene, in this case a form of SEQ ID NO: 1 or 3, may be integrated either at a locus of a genome where that particular nucleic acid sequence is not otherwise normally found or at the normal locus for the transgene. The transgene may consist of nucleic acid sequences derived from the genome of the same species or of a different species than the species of the target animal. For example, axonal regeneration in mice lacking Nogo can be compared with that in mice lacking MAG or both MAG and Nogo. To determine if the effect of the anti-Nogo antibody is due to Nogo blockade, antibody effects can be studied in animals lacking Nogo expression.

As discussed above, a nucleic acid of this disclosure can be transfected into a host cell using a vector. Preferred vectors are plasmids and viral vectors, such as retroviruses. Viral vector may be used to produce a transgenic animal according to the disclosure. Preferably, the viral vectors are replication defective, that is, they are unable to replicate autonomously in the target cell. In general, the genome of the replication defective viral vectors which are used within the scope of the present disclosure lack at least one region which is necessary for the replication of the virus in the infected cell. These regions can either be eliminated (in whole or in part), or be rendered non-functional by any technique known to a person skilled in the art. These techniques include the total removal, substitution (by other sequences, in particular by the inserted nucleic acid), partial deletion or addition of one or more bases to an essential (for replication) region. Such techniques may be performed *in vitro* (on the isolated DNA) or *in situ,* using the techniques of genetic manipulation or by treatment with mutagenic agents.

Preferably, the replication defective virus retains the sequences of its genome which are necessary fur encapsidating the viral particles. The retroviruses are integrating viruses which infect dividing cells. The retrovirus genome includes two LTRs, an encapsidation sequence and three coding regions (*gag, pol* and *env*). The construction of recombinant retroviral vectors has been described (see, for example, Bernstein et al., (1985) Genet. Eng. 7, 235; McCormick, (1985) Biotechnol. 3, 689-691). In recombinant retroviral vectors, the *gag, pol* and *env* genes are generally deleted, in whole or in part, and replaced with a heterologous nucleic acid sequence of interest. These vectors can be constructed from different types of retrovirus, such as, HIV, MoMuLV (murine Moloney leukemia virus), MSV (marine Moloney sarcoma virus), HaSV (Harvey sarcoma virus); SNV (spleen necrosis virus); RSV (Rous sarcoma, virus) and Friend virus.

In general, in order to construct recombinant retroviruses containing a nucleic acid sequence, a plasmid is constructed which contains the LTRs, the eucapsidation sequence and the coding sequence. This construct is used to transfect a packaging cell line, which cell line is able to supply in trans the retroviral functions which are deficient in the plasmid. In general, the packaging cell lines are thus able to express the *gag, pol* and *env* genes. Such packaging cell lines have been described in the prior art, in particular the cell line PA317 (U.S. Patent 4,861,719); the PsiCRIP cell line (WO9002806) and the GP+envAm-12 cell line (WO8907150). In addition, the recombinant retroviral vectors can contain modifications within the LTRs for suppressing transcriptional activity as well as extensive encapsidation sequences which may include a part of the *gag* gene (Bender et al., (1987) J. Virol. 61, 1639-1646). Recombinant retroviral vectors are purified by standard techniques known to those having ordinary skill in the art.

In one aspect the nucleic acid encodes antisense RNA molecules. In this instance, the nucleic acid is operably linked to suitable regulators regions (discussed above) enabling expression of the nucleic acid sequence, and is introduced into a cell utilizing, preferably, recombinant vector constructs, which will express the antisense nucleic acid once the vector is introduced into the cell. Examples of suitable vectors includes plasmids, adenoviruses, adeno-associated viruses (see, for example, U.S. Patent 4,797,368, U.S. Patent 5,139,941), retroviruses (see above), and herpes viruses. For delivery of a therapeutic gene the vector is preferably an adeno-associated virus.

Adenoviruses are eukaryotic DNA viruses that can be modified to efficiently deliver a nucleic acid of the disclosure to a variety of cell types. Various serotypes of adenovirus exist. Of these serotypes, preference is given, within the scope of the present invention, to using type two or type five human adenoviruses (Ad 2 or Ad 5) or adenovirus of animal origin (see W09426914). Those adenoviruses of animal origin which can be used within the scope of the present disclosure include adenoviruses of canine, bovine, murine, ovine, porcine, avian, and simian origin.

The replication defective recombinant adenoviruses according to the disclosure can be prepared by any technique known to the person skilled in the art. In particular, they can be prepared by homologous recombination between an adenovirus and a plasmid which carries, inter alia, the DNA, sequence of interest. The homologous recombination is effected following cotransfection of the said adenovirus and plasmid into an appropriate cell line. The cell line which is employed should preferably (i) be transformable by the said elements, and (ii) contain the sequences which are able to complement the part of the genome of the replication defective adenovirus, preferably in integrated form in order to avoid the risks of recombination. Recombinant adenoviruses are recovered and purified using standard molecular biological techniques, which are well known to one of ordinary skill in the art.

A number of recombinant or transgenic mice have been produced, including those which express an activated oncogene sequence (U.S. Patent 4,736,866); express Simian SV 40 T-antigen (U.S. Patent 5,728,915); lack the expression of interferon regulatory factor 1 (IRF-1) (U.S. Patent 5,731,490); exhibit dopaminergic dysfunction (U.S. Patent 5,723,719); express at least one human gene which participates in blood pressure control (U.S. Patent 5,731,489); display greater similarity to the conditions existing in naturally occurring Alzheimer's disease (U.S. Patent 5,720,936); have a reduced capacity to mediate cellular adhesion (U.S. Patent 5,602,307); possess a bovine growth hormone gene (Clutter et al., (1996) Genetics 143, 1753-1760) or are capable of generating a fully human antibody response (Zou et al., (1993) Science 262, 1271-1274).

While mice and rats remain the animals of choice for most transgenic experimentation, in some instances it is preferable or even necessary to use alternative animal species. Transgenic procedures have been successfully utilized in a variety of non-murine animals, including sheep, goats, chickens, hamsters, rabbits, cows and guinea pigs (see Aigner et al., (1999) Biochem. Biophys. Res. Common. 257, 843-850; Castro et al., (1999) Genet. Anal. 15, 179-187; Brink et al., (2000) Theriogenology 53,139-148; Colman, (1999) Genet. Anal. 15, 167-173; Eyestone, (1999) Theriogenology 51, 509-517; Baguisi et al., (1999) Nat. Biotechnol. 17,456-461; Prather et al., (1999) Theriogenology 51, 487-498; Pain et al., (1999) Cells Tissues Organs 165, 212-219; Fernandez et al., (1999) Indian J. Exp. Biol 37, 1085-1092; U.S. Patent 5,908,969; U.S. Patent 5,792,902; U.S. Patent 5,892,070; U.S. Patent 6,025,540).

### N. Diagnostic Methods

One means of diagnosing a demyelinating disease using the nucleic acid molecules or proteins of this disclosure involves obtaining a tissue sample from living subjects. Obtaining tissue samples from living sources is problematic for tissues such as those of the central nervous system, In patients suffering from a demyelinating disease, tissue samples for diagnostic methods may be obtained by less invasive procedures. For example, samples may be obtained from whole blood and serum.

The use of molecular biological tools has become routine in forensic technology. For example, nucleic acid probes may be used to determine the expression of a nucleic acid molecule comprising all or at least part of the sequences of SEQ ID NO:1 in forensic pathology specimens. Further, nucleic acid assays may be carried out by any means of conducting a transcriptional profiling analysis. In addition to nucleic acid analysis, forensic methods of the disclosure may target the protein encoded by SEQ ID NO: 1 to determine up- or down-regulation of the genes (Shiverick et al., (1975) Biochim. Biophys. Acta 393,124-133).

Methods of the disclosure may involves treatment of tissues with collagenases or other proteases to make the tissue amenable to cell lysis (Semenov et al., (1987) Biull. Eksp. Biol. Med. 104,113-116). Further, it is possible to obtain biopsy samples from different regions of the brain for analysis.

Assays to detect nucleic acid or protein molecules of the disclosure may be in any available format. Typical assays for nucleic acid molecules include hybridization or PCR based formats. Typical assays for the detection of proteins, polypeptides or peptides of the disclosure include the use of antibody probes in any available format such as *in situ* binding assays, etc. See Harlow & Lane, (1988) Antibodies-A Laboratory Manual, Cold Spring Harbor Laboratory Press. In preferred instances, assays are carried out with appropriate controls.

Without further description, it is believed that one of ordinary skill in the art can, using the preceding description and the following illustrative examples, make and utilize the compounds of the present disclosure, including the molecules of the present invention, and practice the disclosed and claimed methods. The following working examples relate to preferred aspects of the present disclosure, and are not to be construed as limiting in any way the remainder of the disclosure.

### EXAMPLES

### Example 1- Identification of Nogo as a Member of the Reticulon Family of Proteins

Adult mammalian axon regeneration is generally successful in the periphery but dismally poor in the CNS. However, many classes ofCNS axons can extend for long distances in peripheral nerve grafts (Benfy & Aguayo (1982) Nature 296,150-152). Comparison of CNS and peripheral nervous system (PNS) myelin has revealed that CNS white matter is selectively inhibitory for axonal outgrowth (Schwab & Thoenen (1985) J. Neurosci. 5,2415-2423). Several components of CNS white matter, NI35, NI250 (Nogo) and MAG, with inhibitory activity for axon extension have been described (Wang et al., (1999) Transduction of inhibitory signals by the axonal growth cone, in Neurobiology of Spinal Cord Injury, Kalb & Strittmatter (editors) Humana Press; Caroni & Schwab, (1988) J. Cell BioL 106,1281-1288; Spillmann et al., (1998) J. Biol. Chem. 73, 19283-19293; McKerracher et al., (1994) Neuron 13, 805-811; Mukhopadhyay et al., (1994) Neuron 13, 757-767.) The IN-1 antibody raised against NI35 and N1250 (Nogo) has been reported to allow moderate degrees of axonal regeneration and functional recovery after spinal cord injury (Bregman et al., (1995) Nature 378, 498-501; Thallmair et al., (1998) Nature Neurosci 1, 24-31). The present disclosure identifies Nogo as a member of the Reticulon protein family.

Nogo is expressed by oligodendrocytes but not by Schwann cells, and associates primarily with the endoplasmic reticulum. The 66 amino acid humenal-extracellular domain of Nogo (SEQ ID NO: 20) inhibits axonal extension and collapses dorsal root ganglion growth cones. Other Reticulon proteins are not expressed by oligodendrocytes, and the 66 amino acid lumenal-extracellular domain from other Reticulon proteins does not inhibit axonal regeneration. These data provide a molecular basis to assess the contribution of Nogo to the failure of axonal regeneration in the adult CNS.

For expression and protein purification of recombinant Nogo-A, the full length sequence (KIAA0886) was generously provided by the Kazusa DNA Research Institute. The full length coding sequence was amplified by the polymerase chain reaction (PCR) and ligated into the pCDNA3.1-MycHis vector (Invitrogen) to generate a plasmid encoding Nogo-A fused at the carboxyl terminus to the Myc epitope (Nogo-A-Myc). Alternatively, the coding sequence was amplified using primers that encode an in-frame Myc epitope immediately amino terminal to the first residue and a stop codon at the carboxyl terminus (Myc-Nogo-A). The Nogo-C-MycHis and Rtn1C-MycHis expression vectors were derived in the same fashion except that an adult rat brain cDNA library was used as template for a PCR reaction with primers was based on the Nogo-C or Rtn1C sequences (Van de Velde et al., (1994) J. Cell. Sci. 107, 2403-2416). These plasmids were transfected into COS-7 or HEK293T by the Lipofectamine (Gibco-BRL) or the FuGENE 6 (Boerhinger Mannheim) method.

A portion of Nogo-A encoding the 66 amino acid lumenal-extracellular fragment of Nogo-A was amplified by PCR and ligated into the pGEX-2T plasmid to yield a prokaryotic expression vector for the GST-Nogo fusion protein. Similar regions of Rtn1, Rtn2 and Rtn3 were amplified by nested PCR using an adult rat brain cDNA library as template and ligated to pGEX-2T. *E. coli* transformed with these plasmids were induced with IPTG. Soluble, native GST fusion proteins were purified using a glutathione-resin and contained approximately 75% GST and 25% full length GST-Nogo or GST-Rtn protein. The majority of the GST-Nogo protein was not extractable from under non-denaturing conditions, but an 8 M urea extract dialyzed against PBS contained over 98% pure GST-Nogo.

Myc immunoreactivity is detectable with an apparent size in the 225 kDa range under reducing conditions (data not shown). Thus, the cDNA directs the expression of a protein with appropriate electrophoretic mobility and the amino acid sequence to be Nogo which was termed human Nogo-A (hNogo-A).

The conserved carboxyl tail of the Rtn family proteins contains two hydrophobic domains separated by a 66 amino acid residue hydrophilic segment. None of the sequences contain a signal peptide. The predicted topology for these proteins is for the amino and carboxyl termini to reside in the cytosol, and for the conserved region to associate with the lipid bilayer. For Rtn1-A, there is experimental evidence demonstrating that the polypeptide behaves as an integral membrane protein, and that the hydrophobic segments of the conserved domain are responsible for this behavior (Van de Velde et al., (1994) J. Cell. Sci. 107, 2403-2416). Myc-tagged Nogo is also associated with particulate fractions and is extracted by detergent but not high ionic strength (data not shown).

When overexpressed in kidney cells, the Rtn1 protein is localized primarily to endoplasmic reticulum (ER) in a finely granulated pattern, hence the Reticulon name (Van de Velde et al., (1994) J. Cell. Sci. 107, 2403-2416). There is a di-lysine ER retention motif at the carboxyl terminus ofNogo and most Rtn proteins (Van de Velde et al., (1994) J. Cell. Sci. 107, 2403-2416; Jackson et al., (1991) EMBO J. 9, 3153-3162). In neurons, Rtn1 is expressed throughout processes and is concentrated in growth cones (Senden et al., (1996) Eur. J. Cell. Biol. 69, 197-213). Its localization in transfected kidney cells has led to the suggestion that Rtn1 might regulate protein sorting or other aspects of ER function (Van de Velde et al., (1994) J. Cell. Sci. 107, 2403-2416). Both the A and C splice forms of Nogo exhibit a reticular distribution when expressed in COS-7 cells, similar to that of Rtn1-C.

### Example 2 - Polyclonal Antibodies against Nogo

The predicted intra-membrane topology of the two hydrophobic domains of Nogo indicates that the 66 amino acid residues between these segments is localized to the lumenal/extracellular face of the membrane. To explore this further, an antiserum directed against the 66 amino acid domain was generated.

For antibody production and immunohistology, anti-Myc immunoblots and immunohistology with the 9E10 antibody were obtained as described in Takahashi et al., (1998) Nature Neurosci., 1, 487-493 & Takahashi et al., (1999) Cell, 99, 59-69. The GST-Nogo fusion protein was employed as an immunogen to generate an anti-Nogo rabbit antiserum. Antibody was affinity-purified and utilized at 3 µg/ml for immunohistology and 1 µg/ml for immunoblots. To assess the specificity of the antiserum, staining was conducted in the presence of GST-Nogo protein at 0.1 mg/ml. For live cell staining, cells were incubated in primary antibody dilutions at 4°C for one hour in Hanks balanced salt solution with 0.05% BSA and 20 mM Na-Hepes (pH 7.3). After fixation, bound antibody was detected by incubation with fluorescently labeled secondary antibodies.

The antibody detects a low level of surface expression of this epitope, while the Myc epitope at the carboxyl terminus of expressed Nogo is not detected unless cells are permeablized. This surface staining was attributed to a minority of No go protein associated with the plasma membrane rather than the ER membrane. This data supports a topographic model wherein the amino and carboxyl termini of the protein reside in the cytoplasm and 66 amino acid of the protein protrude on the lumenal-extracellular side of the ER or plasma membrane.

### Example 3 - Nogo Expression in the Central Nervous System

If Nogo is a major contributor to the axon outgrowth inhibitory characteristics of CNS myelin as compared to PNS myelin (Caroni & Schwab, (1988) J. Cell Biol. 106, 1281-1288; Spillmann et al., (1998) J. Biol. Chem. 73, 19283-19293; Bregman et al., (1995) Nature 378,498-501), then Nogo should be expressed in adult CNS myelin but not PNS myelin. Northern blot analysis of Nogo expression was performed using probes derived from the 5' Nogo-A/B-specific region and from the 3' Nogo common region of the cDNA. A single band of about 4.1 kilobase was detected with the 5' probe in adult rat optic nerve total RNA samples, but not sciatic nerve samples. The results indicate that the Nogo-A clone is a full length cDNA, and are consistent with a role for Nogo as a CNS-myelin-specific axon outgrowth inhibitor. Northern blot analysis with a 3' probe reveals that optic nerve expresses high levels of the Nogo-A mRNA and much lower levels of Nogo-B and Nogo-C. Whole brain expresses both Nogo-A and Nogo-C, but a number of peripheral tissues (including sciatic nerve) express little or no Nogo. Nogo-C/Rtn4-C expression has been demonstrated in skeletal muscle and adipocytes, as well as in brain (Morris et al., (1991) Biochim. Biophys. Acta 1450, 68-76). Within the Rtn family, optic nerve expression appears to be selective for Nogo, with no detectable expression of Rtn 1 or Rtn 3. Rtn 2 has not been examined.

*In situ* hybridization reveals Nogo mRNA in cells with the morphology of oligodendrocytes in adult rat optic nerve and pyramidal tract. Within the brain, Nogo expression is also detected in certain neuronal populations. In contrast to Nogo, Rtn1 and Rtn3 are not expressed in optic nerve but mRNA is detected in certain neuronal populations. Nogo protein localization was analyzed in spinal cord cultures treated with PDGF and low serum to induce oligodendrocyte differentiation, using the anti-Nogo antibody and the oligodendrocyte-specific 04 monoclonal antibody. In living cells, both the lumenal-extracellular 66 amino acid loop of Nogo and the 04 antigen are detected on the surface of oligodendrocytes. Approximately half of O4-positive cells in these cultures exhibit Nogo surface staining.

### Example 4 - Nogo-Mediated Growth Cone Collapse

For all experiments involving cell culture, the following methods were employed. The culture of embryonic chick E10 and E12 dorsal root ganglion explants and dissociated neurons utilized methods described for E7 dorsal root ganglion cultures (Takahashi et al., (1998) Nature Neurosci. 1, 487-493; Takahashi et al., (1999) Cell 99, 59-69; Goshima et al., (1995) Nature 376, 509-514; Jin & Strittmatter, (1997) J. Neurosci. 7, 6256-6263). NGF-differentiated PC12 cells were cultured as described (Strittmatter et al., (1994) J. Neurosci. 14, 2327-2338). Embryonic spinal cord explants (rat E10 or chick E5) were cultured for 7-14 days in the presence of PDGF-AA to induce differentiation of some cells into mature oligodendrocytes (Vartanian et al., (1999) Proc. Natal. Acad. Sci. USA 96, 731-735). The procedure for growth cone collapse assays is identical to that for analysis of Sema3A-induced growth cone collapse (Takahashi et al., (1998) Nature Neurosci. 1, 487-493; Takahashi et al., (1999) Cell 99, 59-69; Goshima et al., (1995) Nature 376, 509-514; Jin & Strittmatter, (1997) J. Neurosci. 17, 6256-6263). The method for analysis of total neurite outgrowth has also been described (Goshima et al., (1995) Nature 376, 509-514; Jin & Strittmatter, (1997) J. Neurosci. 17, 6256-6263; Strittmatter et al., (1994) J. Neurosci. 14, 2327-2338). In outgrowth assays, proteins and peptides were added one hour after plating to minimize any effect on the total number of adherent cells. To test the effect of substrate-bound GST or GST-Nogo, the protein solutions were dried on poly-L-lysine coated glass, washed and then coated with laminin. For E12 cultures, the neuronal identity of cells was verified by staining with anti-neurofilament antibodies (2H3, Develomental Studies Hybridoma Bank) and neurites were traced by observation of rhodamine-phalloidin staining of F-actin in processes.

The expression of recombinant Nogo in HEK293T cells allows a rigorous test of whether this protein has axon outgrowth inhibiting effects. Washed membrane fractions from vector- or hNogo-A-Myc-transfected HEK293T cells were added to chick E12 dorsal root ganglion explant cultures. Growth cone morphology was assessed after a thirty minute incubation at 37°C by fixation and rhodamine-phalloidin staining.

The control HEK membranes have no detectable effect on growth cone morphology. The Nogo-A-containing membrane fractions induced collapse of a majority of dorsal root ganglion growth cones. This growth cone collapse indicates an axon outgrowth inhibiting activity, and Nogo inhibition of axon extension is also demonstrable (see below). The Nogo-C form also exhibits collapse activity, indicating that the shared carboxyl terminus of the protein including the hydrophobic segments and the 66 amino acid lumenal-extracellular domain contains functionally important residues. Additional inhibitory activity in the amino terminal region of Nogo-A is not excluded by these studies. The sensitivity of more immature explant cultures from E10 chick embryos or from E 15 rat embryos (data not shown) is substantially less. The developmental regulation of sensitivity is consistent with experiments using partially purified Nogo (Bandilow et al., (1997) Eur. J. Neurosci. 9, 2743-2752).

Within the growth cone collapsing Nogo-C protein, the hydrophilic 66 lumenal-extracellular domain seems more likely to interact with the surface of dorsal root ganglion neurons than do the membrane-embedded hydrophobic domains. To test this hypothesis, the 66 ammo acid region of hNogo was expressed in and purified from *E. coli.* A majority of the GST-Nogo fusion protein accumulates in inclusion bodies, but can be recovered by urea extraction. This restricted region of Nogo possesses potent (EC50 = 50 nM) growth cone collapsing activity for chick E12 dorsal root ganglion neurons (data not shown). The urea-extracted protein preparation is likely to present only a small fraction of the Nogo sequence in an active conformation. Therefore, 10% of GST-Nogo that is soluble in *E. coli* was purified using a glutathione-Sepharose resin. This preparation is even more potent than the urea-extracted protein as a collapsing factor, acutely altering growth cone morphology at concentrations as low as I nM.

The nanomolar potency is on a par with most known physiologic regulators of axon guidance. Axon outgrowth from dorsal root ganglion neurons and NGF-differentiated PCR12 cells is also blocked by this soluble GST-Nogo protein in nM concentrations (data not shown). When GST-Nogo is bound to substrate surfaces, axonal outgrowth from dorsal root ganglion neurons or PC12 cells is reduced to undetectable levels. These are selective effects on axon outgrowth rather than cell survival since GST-Nogo does not reduce the number of neurofilament-positive adherent cells (137 ± 24% of GST-treated cultures) nor significantly alter the number of apoptotic nuclei identified by DAP1 staining (4.0 ± 1.7% in control cultures and 5.2 ± 1.1% in GST-Nogo-treated specimens).

Oligodendrocytes appear to express Nogo selectively amongst the Rtn proteins. To explore the selectivity of Nogo s role in the inhibition of axonal regeneration, the axon outgrowth inhibiting activity of other Rtn proteins was considered. The predicted lumenal-extracellular 66 amino acid fragments of Rtn1, Rtn2 and Rtn3 were expressed as GST fusion proteins and purified in native form. At concentrations in which the Nogo fragment collapses a majority of E 12 dorsal root ganglion growth cones, the other Rtn proteins do not alter growth cone morphology (data not shown). Thus, the axon regeneration inhibiting activity is specific for Nogo in the Rtn family.

### Example 5 - Nogo receptor Peptide Agents

To further define the active domain ofNogo, 25 amino acid residue peptides corresponding to segments of the 66 amino acid sequence were synthesized. The peptide corresponding to residues 31-55 of the extracellular fragment of Nogo exhibits growth cone collapsing (Figure 2) and outgrowth inhibiting (data not shown) activities at concentrations of 4 µM. While this sequence may provide the core of the inhibitory domain, the 66 amino acid fragment is clearly required for full potency. Interestingly, this is the region within the 66 amino acid domain sharing the least similarity to other Rtn proteins, consistent with the other family members being inactive as axon regeneration inhibitors. Indeed, the Rtn1 31-55 amino acid lumenal-extracellular peptide exerts no growth cone collapse activity (data not shown).

The aforementioned experimental data identifies Nogo as an oligodendrocyte-specific member of the Rtn family and demonstrates that a discrete domain of Nogo can inhibit axon outgrowth. Other Rtn proteins do not possess this activity. The expression of Nogo in oligodendrocytes but not Schwann cells therefore contributes to the failure of axonal regeneration in the adult mammalian CNS as compared to the adult PNS. The relative contribution of Nogo as compared to other CNS myelin components to the non-permissive nature of CNS white matter can now be characterized at a molecular level.

While the current experimental data is consistent with a role for Nogo in blocking adult CNS axonal regeneration after pathologic injury, this may also be related to the physiologic role of Nogo in non-pathologic states. Based on localization studies, other Rtn proteins are thought to play a role in ER function (Van de Velde et al., (1994) J. Cell. Sci. 107, 2403-2416). A majority of Nogo is distributed in a reticular pattern in COS-7 cells and only a minority seems to be accessible at the cell surface.

### Example 6 - Inhibition of Nogo Activity

The previous examples have shown that a 66 amino acid region near the carboxyl terminus of Nogo inhibits axon outgrowth and is expressed at the cell surface. Shorter twenty-five amino acid segments of this domain are either inert as outgrowth inhibitors or of much lower potency (GrandPré et al., (2000) Nature 403, 439-444). The 31-55 region from this 66 amino acid segment has weak growth cone collapse and axon outgrowth inhibiting activity. To block Nogo action *in vivo,* a competitive antagonist of Nogo which binds to the same receptor site but does not exert a biological effect in its own right would be highly desirable. Various fragments of the 66 amino acid region were tested as blockers of Nogo-mediated axon growth inhibition. Two assays have been used for this purpose. The first is the growth cone collapse assay and the second is a binding assay.

In the growth cone collapse assay, the response to Nogo was measured in the presence of various potential antagonistic peptides. Three of the twenty-five amino acid peptides (1-25, 11-35 and 21-45) from the 66 amino acid region possess blocking activity at µM concentrations (Figure 2). The combination of all three peptides does not alter growth cone morphology under basal conditions but totally prevents collapse by 15 nM GST-Nogo. The same mixture of peptides is also capable of blocking low dose CNS myelin induced growth cone collapse. This blockade supports the hypothesis that Nogo is a primary inhibitory component of CNS myelin. Furthermore, the blockade has properties expected for competitive antagonism, being ineffective at high doses of CNS myelin.

To develop an antagonist with higher specificity and potency, a longer fragment of Nogo has been tested. Preferentially, such a peptide itself has no axon outgrowth inhibiting activity on its own while competitively blocking Nogo action. The 2-41 fragment of Nogo is acetylated at the carboxy terminus and amidated at the amino terminous and is the highest potency blocker of Nogo defined to date. Pep2-41 abolishes GST-Nogo-induced growth cone collapse and possesses an apparent Ki of 150 nM in the binding assay (Figure 3). The 2-41 fragment also blocks the ability of both purified Nogo-66 protein and crude CNS myelin to inhibit neurite outgrowth in cultured neurons (Figure 4).

### Example 7 - Identification of the Nogo receptor

A Nogo binding assay was developed which utilizes a method widely used in examining semaphorin and ephrin axonal guidance function (Flanagan & Vanderhaeghen, (1998) Annu. Rev. Neurosci. 21, 309-345; Takahashi et al., (1999) Cell 99, 59-69). It involves fusing a secreted placental alkaline phosphatase (AP) moiety to the ligand in question to provide a biologically active receptor binding agent which can be detected with an extremely sensitive colorimetric assay. For Nogo, an expression vector was created encoding a signal peptide, a His6 tag for purification, AP and the 66 amino acid active domain of Nogo. The fusion protein can be purified from the conditioned medium of transfected cells in milligram amounts (Figure 5). This protein is biologically active as a growth cone collapsing agent, with an EC₅₀ of 1 nM. AP-Nogo is actually slightly more potent than GST-Nogo perhaps because the protein is synthesized in eukaryotic rather than a prokaryotic cell. Initial studies have revealed saturable, high affinity sites on axons. Binding is blocked by GST-Nogo and by the antagonistic 25 amino acid peptides, consistent with competitive binding to a neuronal receptor site. Since the apparent K_{d} (3 nM) for these sites in close to the EC₅₀ of AP-Nogo in the collapse assay, the sites are likely to be physiologically relevant Nogo receptors.

This assay was utilized for expression cloning of a Nogo receptor. Pools of a mouse adult brain cDNA expression library representing 250,000 independent clones were transfected into non-neuronal COS-7 cells. Non-transfected COS-7 cells do not bind AP-Nogo, but transfection with two pools of 5,000 clones exhibited a few cells with strong AP-Nogo binding. Single cDNA clones encoding a Nogo biding site were isolated by sib-selection from each of the two positive pools. The two independently isolated clones are identical to one another except for a 100 bp extension of the 5' untranslated region in one clone. Transfection of these clones into COS-7 cells yields a binding site with an affinity for AP-Nogo identical to that observed in E13 dorsal root ganglion neurons; the K_{d} for binding is about 3 nM (Figure 6). AP alone does not bind with any detectable affinity to these transfected cells, indicating that the affinity is due to the 66 amino acid derived from Nogo. Furthermore, GST-Nogo displaces AP-Nogo from these sites.

This cDNA encodes a novel 473 amino acid protein. There is no reported cDNA with significant homology in GenBank. The predicted protein contains a signal peptide followed by eight leucine-rich repeat regions, a unique domain and a predicted GPI anchorage site (Figure 7). A human homologue of the murine cDNA was identified that shares 89% amino acid identity. The existence of this cDNA was predicted from the murine cDNA structure and analysis of human genomic sequence deposited in GenBank as part of the Human Sequencing Project. The exons of the human cDNA are distributed over 35 kilobases and the cDNA was not previously recognized in the genomic sequence. The protein structure is consistent with a cell surface protein capable of binding Nogo. The GPI-linked nature of the protein suggests that there may be a second receptor subunit that spans the plasma membrane and mediates Nogo signal transduction.

### Example 8 - Tissue distribution of Nogo receptor

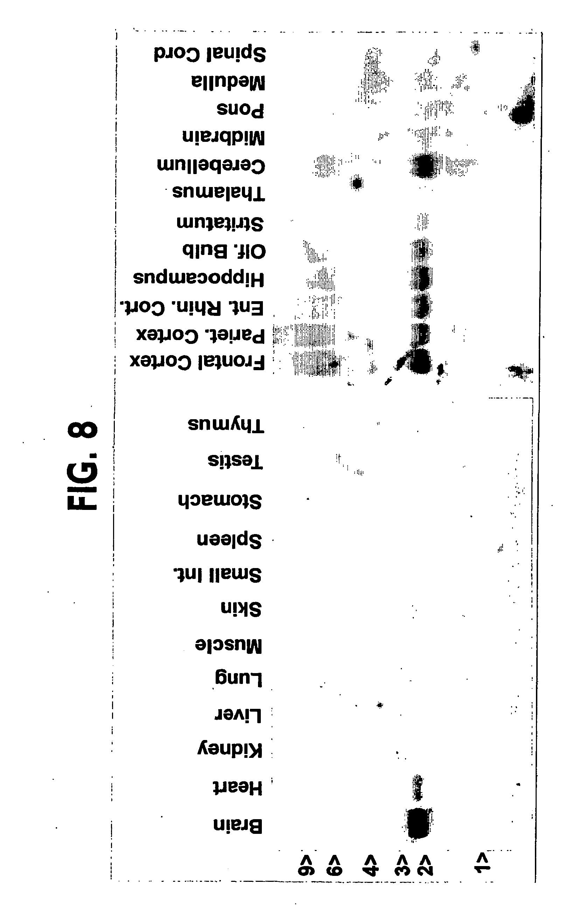

The distribution of the mRNA for this Nogo receptor is consistent with a role for the protein in regulating axonal regeneration and plasticity in the adult CNS. Northern analysis shows a single band of 2.3 kilobases in the adult brain, indicating that the isolated Nogo receptor clone is full length (Figure 8). Low levels of this mRNA are observed in heart and kidney but not in other peripheral tissues. In the brain, expression is widespread and those areas richest in gray matter express the highest levels of the mRNA.

### Example 9 - Biological effects of different Nogo domains

Assays of Nogo-A function have included growth cone collapse, neurite outgrowth, and fibroblast spreading with substrate-bound and soluble protein preparations (Caroni & Schwab, (1988) J. Cell Biol. 106, 1281-1288; GrandPré et al., (2000) Nature 403, 439-444; Chen et al., (2000) Nature 403, 434-439; Prinjha et al., (2000) Nature 403, 483-484). In assays of 3T3 fibroblast morphology, substrate-bound Nogo-66 does not inhibit spreading (Figure 1b,e). Since NI250 preparations and full length Nogo-A are non-permissive for 3T3 spreading, it was necessary to consider whether different domains of Nogo might subserve this *in vitro* activity. To facilitate a comparison of different Nogo-A domains, the acidic amino terminal 1040 amino acid fragment (Amino-Nogo) was expressed as a Myc-his tagged protein in HEK293T cells (Figure 1d). The Nogo protein is present in cytosolic fractions. Surfaces coated with purified Amino-Nogo protein fail to support 3T3 fibroblast spreading (Figure 1b,e). Similar results were observed for a kidney-derived cell line, COS-7 (Figure 1f). Therefore, the amino terminal domain appears to account for the effects of full-length Nogo-A on fibroblasts. The Nogo-66 domain is specific for neurons; it does not affect non-neuronal cells.

Dorsal root ganglion cultures were also exposed to Amino-Nogo protein (Figure 1c,g-i). As for 3T3 fibroblasts, the fibroblast-like cells in the dorsal root ganglion culture do not spread on this substrate. Furthermore, axonal outgrowth is reduced to low levels on Amino-Nogo coated surfaces. Thus, while the Nogo-66 effects are neural-specific, the inhibitory action of the Amino-Nogo domain is more generalized. When presented in soluble form at 100 nM, the Nogo-66 polypeptide collapses chick E12 dorsal root ganglion growth cones and nearly abolishes axonal extension, as described previously (GrandPré et al., (2000) Nature 403, 439-444). In marked contrast, the soluble Amino-Nogo protein appears inactive, and does not significantly modulate dorsal root ganglion growth cone morphology or dorsal root ganglion axonal extension or non-neuronal cell spreading (Figure 1c,g-i).

In the experiments of Walsh and colleague (Prinjha et al., (2000) Nature 403, 483-484), cerebellar granule neurons were studied and soluble Amino-Nogo was presented as an Fc fusion protein, presumably in dimeric form. Therefore, it was necessary to consider whether these differences might explain the inactivity of soluble Amino-Nogo. Mouse P4 cerebellar granule neurons respond to Nogo preparations is a fashion indistinguishable from chick E13 dorsal root ganglion neurons (Figure 1i). Amino-Nogo dimerized with anti-Myc antibody inhibits 3T3 and COS-7 spreading (Figure 1e,f) and tends to reduce cerebellar axon outgrowth (Figure 1i). When further aggregated by the addition of anti-Mouse IgG antibody. Amino-Nogo significantly reduces both dorsal root ganglion and cerebellar axon outgrowth (Figure 1h,i). While the Amino-Nogo protein is quite acidic, electrostatic charge alone does not account for its inhibitory effects since poly-Asp does not alter cell spreading or axonal outgrowth (Figure 1e,f,h). Thus, the Nogo-66 domain is a potent and neuron-specific inhibitor, while the intracellular Amino-Nogo domain inhibits multiple cell types and appears to function only in an aggregated state.

### Example 10 - Localization of Nogo receptor

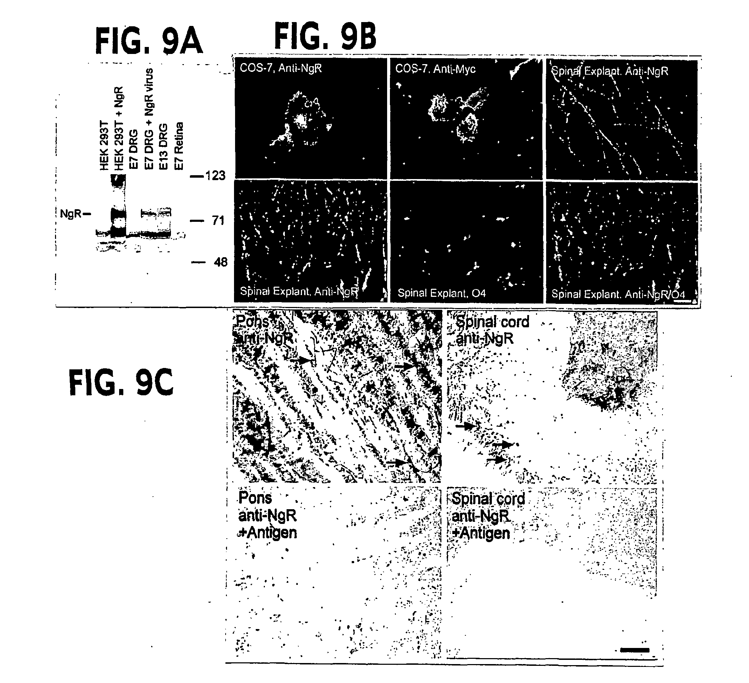

To further characterize the expression of the Nogo-66 receptor protein an antiserum to a GST-Nogo receptor fusion protein was developed. This antiserum detects an 85 kDa protein selectively in Nogo-66 receptor-expressing HEK293T cells (Figure 9a), and specifically stains COS-7 cells expressing Nogo-66 receptor (Figure 9b). Immunohistologic staining of chick embryonic spinal cord cultures localizes the protein to axons, consistent with mediation of Nogo-66-induced axon outgrowth inhibition. Nogo-66 receptor expression is not found in the O4-positive oligodendrocytes that express Nogo-66. Immunoreactive 85 kDa protein is expressed in Nogo-66-responsive neuronal preparations from chick E13 dorsal root ganglion, but to a much lesser degree in weakly responsive tissue from chick E7 dorsal root ganglion and chick E7 retina (Figure 9a). Overall, the pattern of Nogo-66 expression is consistent with the protein mediating Nogo-66 axon inhibition.

This antibody is also effective in localizing the Nogo-66 receptor protein in tissue sections (Figure 9c). While it is clear from *in situ* hybridization studies that the protein is expressed in multiple classes of neurons, immunohistology reveals the protein at high levels in CNS while matter in profiles consistent with axons. Protein is detectable at lower levels in neuronal soma and neuropil. This provides further support for the proposed function of this protein in mediating interactions with oligodendrocytes.

### Example 11 Nogo receptor mediates Nogo-66 responses

The Nogo-66 receptor protein is necessary for Nogo-66 action and not simply a binding site with a function unrelated to inhibition of axonal outgrowth. A first prediction is that phosphoinositol specific-Phospholipase C (PI-PLC) treatment to remove glycophosphatidylinositol (GPI) -linked proteins from the neuronal surface will render neurons insensitive to Nogo-66. This prediction holds true for chick E13 dorsal root ganglion neurons, PI-PLC treatment abolishes both AP-Nogo binding and GST-Nogo-66-induced growth cone collapse (Figure 10a-c). As a control, Sema3A responses in the parallel cultures are not altered by PI-PLC treatment. Of course, PI-PLC treatment is expected to remove a number of proteins from the axonal surface so this result leaves open the possibility that other GPI-linked proteins are mediating the Nogo-66 response in untreated cultures.

To demonstrate that the Nogo-66 receptor is capable of mediating Nogo-66 inhibition of axon outgrowth, the protein was expressed in neurons lacking a Nogo-66 response. Both dorsal root ganglion and retinal neurons from E7 chick embryos were examined. The Nogo responses in the dorsal root ganglion neurons from this developmental stage arc weak but slight responses can be detected in some cultures (data not shown). E7 retinal ganglion cell growth cones are uniformly insensitive to Nogo-66-induced growth cone collapse (Figure 10e), do not bind AP-Nogo (data not shown) and do not exhibit 85 kDa anti-Nogo-66 receptor immunoreactive protein (Figure 9a). Expression ofNgR in these neurons by infection with recombinant HSV preparations renders the retinal ganglion cell axonal growth cones sensitive to Nogo-66-induced collapse. Infection with a control PlexinA1-expressing control HSV preparation does not alter Nogo responses. Taken together, these data indicate that the Nogo receptor identified here participates in Nogo-66 inhibition of axon regeneration.

### Example 12 - Structural analysis of Nogo-66 receptor

The Nogo-66 receptor structure was examined to determine which regions mediate Nogo-66 binding. The protein is simply divided into the leucine rich repeat and the non-leucine rich repeat region. Deletion analysis clearly shows that the leucine rich repeats are required for Nogo-66 binding but the remainder of the protein is not necessary (Figure 11). Within the leucine rich repeat domain, two domains have been separately deleted. This is predicted to maintain the overall leucine rich repeat domain structure, and a similar approach has been utilized for the leutropin receptor. It is apparent that the Nogo-66 binding requires all eight leucine rich repeats, and suggests that a significant segment of the planar surface created by the linear beta sheets of the leucine rich repeats. The leucine rich repeat-amino terminous and leucine rich repeat-carboxy terminous conserved cysteine rich regions at each end of the leucine rich repeats are also required for Nogo-66 binding, presumably these are necessary to generate appropriate leucine rich repeat conformation.

### Example 13 - Blockade of Nogo by soluble Nogo receptor ectodomain protein

One method for blocking a signal transduction cascade initiated by Nogo-66 binding to the Nogo receptor is to provide excess soluble ectodomain of the receptor. A secreted fragment of the Nogo receptor protein has been produced in HEK293T cells. The cDNA encoding amino acid residues 1-348 of the murine Nogo receptor were ligated into a eukaryotic expression vector and that DNA was transfected into HEK293T cells. Conditioned medium from these cells contains high levels of this Nogo receptor fragment (NgR-ecto), as demonstrated by immunoblots with an anti-NgR antibody. The conditioned medium contains approximately 1 mg of NgR-ecto protein per liter. In the AP-Nogo binding assay to COS-7 cells expressing full length Nogo receptor or to dorsal root ganglion neurons, the addition of NgR-ecto conditioned medium reduces the binding of 0.5 nM AP-Nogo-66 by 80%. Complex formation between soluble NgR-ecto and Nogo-66 prevents binding to cell surface receptors.

For some receptor systems, such soluble receptor ligand complexes can block signaling by creating an ineffective interaction. For example, the soluble ectodomain of Trk serves to block neurotrophin signaling and has been extensively used for this purpose (Shelton et al., (1995) J. Neurosci. 15, 477-491). Alternatively, the Nogo-66/NgR-ecto soluble complex may bind to and stimulate the presumed second transmembrane Nogo receptors subunit. There is precedence for this type of effect from studies of GDNF family receptors (Cacalano et al., (1998) Neuron 21, 53-62). The Nogo-66/NgR-ecto complex does not cause growth cone collapse in those neurons (chick E7 retinal ganglion cells) which lack the Nogo-66 receptor but containing other components of the Nogo signaling pathway. This indicates that NgR-ecto functions as a blocker of Nogo-66 signaling.

In direct tests, the NgR-ecto proteins protects axons from the inhibitory effects of Nogo-66. NgR-ecto prevents Nogo-66-induced growth cone collapse and blocks Nogo-66-induced inhibition of neurite outgrowth from chick E13 DRG neurons (Figure 12). Furthermore, the presence of NgR-ecto protein blocks the ability of CNS myelin to inhibit axonal outgrowth in vitro (Figure 12). These data demonstrate that a NgR-ecto protein can promote axonal regeneration *in vivo.*

Although the present invention has been described in detail with reference to examples above, it is understood that various modifications can be made without departing from the spirit of the invention. Accordingly, the invention is limited only by the following claims. The results of part of the experiments disclosed herein have been published (GrandPré et al., (2000) Nature 403, 439-444) after the filing date of U.S. Provisional Application 60/175,707 from which this application claims priority.

## Claims

1. An isolated nucleic acid molecule selected from the group consisting of:
(a) an isolated nucleic acid molecule that encodes a fragment of SEQ ID NO:2;
(b) an isolated nucleic acid molecule which hybridises to a nucleic acid molecule comprising the complement of SEQ ID NO:1 under high stringency conditions; and
(c) an isolated nucleic acid molecule with at least seventy-five percent sequence homology to SEQ ID NO:1;
wherein said isolated nucleic acid molecule encodes a polypeptide which blocks the inhibition of axonal growth that is mediated by the NOGO-receptor of SEQ ID NO:2.

2. The isolated nucleic acid molecule of Claim 1 wherein said nucleic acid molecule is operably linked to one or more expression control elements.

3. A vector comprising the isolated nucleic acid molecule of Claim 1 or Claim 2.

4. A host cell transformed to contain the isolated nucleic acid molecule of Claim 1 or Claim 2, or a host cell containing the vector of Claim 3.

5. The host cell of Claim 4, wherein said host cell is a eukaryotic cell.

6. A method for producing a polypeptide comprising the step of culturing the host cell of Claim 4 or Claim 5 under conditions in which the polypeptide encoded by said nucleic acid molecule is expressed.

7. An isolated polypeptide produced by the method of Claim 6.

8. An isolated polypeptide selected from the group consisting of:
(a) an isolated polypeptide comprising a fragment of at least 30 amino acids of SEQ ID NO:2; and
(b) an isolated polypeptide with at least seventy-five percent amino acid sequence homology to SEQ ID NO:2;
wherein said polypeptide blocks the inhibition of axonal growth that is mediated by the NOGO-receptor of SEQ ID NO:2.

9. The isolated polypeptide of Claim 8, wherein said polypeptide is an isolated polypeptide with at least eighty percent amino acid sequence identity to SEQ ID NO:2.

10. The isolated polypeptide of Claim 8, wherein said polypeptide is an isolated polypeptide with at least ninety percent amino acid sequence identity to SEQ ID NO:2.

11. The isolated polypeptide of Claim 8, wherein said polypeptide is an isolated polypeptide with at least ninety-five percent amino acid sequence identity to SEQ ID NO:2.

12. A chimeric polypeptide comprising the polypeptide of any one of Claims 7 to 11, wherein said polypeptide blocks the inhibition of axonal growth that is mediated by the NOGO-receptor of SEQ ID NO:2.

13. An antibody or antigen-binding fragment thereof that specifically binds to a polypeptide of any one of Claims 7 to 11 wherein said antibody or antigen-binding fragment inhibits the binding between the NOGO-receptor of SEQ ID NO:2 and the NOGO polypeptide of SEQ ID NO:6 and wherein said antibody or antigen-binding fragment blocks the inhibition of axonal growth that is mediated by the NOGO-receptor of SEQ ID NO:2.

14. The antibody or antigen-binding fragment of Claim 13, wherein said antibody or antigen-binding fragment is a monoclonal antibody.

15. A composition comprising the polypeptide of any one of Claims 7 to 12, or an antibody or antigen-binding fragment of Claim 13 or Claim 14, and a pharmaceutically acceptable carrier.

16. The composition of Claim 15, wherein the composition is formulated for parenteral, subcutaneous, intravenous, intramuscular, intraperitoneal, transdermal, buccal or oral administration, or for administration via microinfusion.

17. Use of the polypeptide of any one of Claims 7 to 12, or the antibody or antigen-binding fragment of Claim 13 or Claim 14, for the preparation of a medicament for promoting axonal growth.

18. Use of the polypeptide of any one of Claims 7 to 12, or the antibody or antigen-binding fragment of Claim 13 or Claim 14, for the preparation of a medicament for promoting axonal regeneration.

19. Use of the nucleic acid molecule of Claim 1 or Claim 2, the polypeptide of any one of Claims 7 to 12, or the antibody or antigen-binding fragment of Claim 13 or Claim 14, for the preparation of a medicament for the treatment of a central nervous system (CNS) disease, disorder, or injury, in a mammal.

20. The use of Claim 19, wherein said CNS disease, disorder, or injury is selected from the group consisting of cranial or cerebral trauma, spinal cord injury, stroke or a demyelinating disease.

21. The use of Claim 20, wherein said demyelinating disease is selected from the group consisting of multiple sclerosis, monophasic demyelination, encephalomyelitis, multifocal leukoencephalopathy, panencephalitis, Marchiafava-Bignami disease, pontine myelinolysis, adrenoleukodystrophy, Pelizaeus-Merzbacher disease, Spongy degeneration, Alexander's disease, Canavan's disease, metachromatic leukodystrophy, and Krabbe's disease.

22. An *in vitro* method of promoting axonal growth comprising contacting a neuron with the polypeptide of any one of Claims 7 to 12 or the antibody or antigen-binding fragment of Claim 13 or Claim 14.

23. An *in vitro* method of promoting axonal regeneration comprising contacting a neuron with the polypeptide of any one of Claims 7 to 12 or the antibody or antigen-binding fragment of Claim 13 or Claim 14.

## Patentansprüche

1. Isoliertes Nukleinsäuremolekül, das aus der Gruppe ausgewählt ist, bestehend aus:
(a) einem isolierten Nukleinsäuremolekül, das ein Fragment von SEQ ID NO:2 kodiert;
(b) einem isolierten Nukleinsäuremolekül, das mit einem Nukleinsäuremolekül, das das Komplement von SEQ ID NO:1 umfasst, unter Bedingungen hoher Stringenz hybridisiert; und
(c) einem isolierten Nukleinsäuremolekül mit mindestens 75 % Sequenzhomologie zu SEQ ID NO:1,
wobei das isolierte Nukleinsäuremolekül ein Polypeptid kodiert, das die Hemmung des axonalen Wachstums blockiert, das durch den NOGO-Rezeptor von SEQ ID NO:2 vermittelt wird.

2. Das isolierte Nukleinsäuremolekül nach Anspruch 1, wobei das Nukleinsäuremolekül mit einem oder mehreren Expressionskontrollelementen operabel verknüpft ist.

3. Vektor, umfassend das isolierte Nukleinsäuremolekül nach Anspruch 1 oder Anspruch 2.

4. Wirtszelle, die transformiert ist, um das isolierte Nukleinsäuremolekül nach Anspruch 1 oder Anspruch 2 zu enthalten, oder eine Wirtszelle, die den Vektor nach Anspruch 3 enthält.

5. Die Wirtszelle nach Anspruch 4, wobei die Wirtszelle eine eukaryotische Zelle ist.

6. Verfahren zur Herstellung eines Polypeptids, umfassend den Schritt des Kultivierens der Wirtszelle nach Anspruch 4 oder Anspruch 5 unter Bedingungen, wobei das von dem Nukleinsäuremolekül kodierte Polypeptid exprimiert wird.

7. Durch das Verfahren nach Anspruch 6 hergestelltes isoliertes Polypeptid.

8. Isoliertes Polypeptid, das aus der Gruppe ausgewählt ist, bestehend aus:
(a) einem isolierten Polypeptid, umfassend ein Fragment von mindestens 30 Aminosäuren von SEQ ID NO:2; und
(b) einem isolierten Polypeptid mit mindestens 75 % Aminosäure-Sequenzhomologie zu SEQ ID NO:2;
wobei das Polypeptid die Hemmung des axonalen Wachstums blockiert, das durch den NOGO-Rezeptor von SEQ ID NO:2 vermittelt wird.

9. Das isolierte Polypeptid nach Anspruch 8, wobei das Polypeptid ein isoliertes Polypeptid mit mindestens 80 % Aminosäure-Sequenzidentität zu SEQ ID NO:2 ist.

10. Das isolierte Polypeptid nach Anspruch 8, wobei das Polypeptid ein isoliertes Polypeptid mit mindestens 90 % Aminosäure-Sequenzidentität zu SEQ ID NO:2 ist.

11. Das isolierte Polypeptid nach Anspruch 8, wobei das Polypeptid ein isoliertes Polypeptid mit mindestens 95 % Aminosäure-Sequenzidentität zu SEQ ID NO:2 ist.

12. Chimäres Polypeptid, umfassend das Polypeptid nach einem der Ansprüche 7 bis 11,
wobei das Polypeptid die Hemmung des axonalen Wachstums blockiert, das durch den NOGO-Rezeptor von SEQ ID NO:2 vermittelt wird.

13. Antikörper oder Antigen-bindendes Fragment davon, das spezifisch an ein Polypeptid nach einem der Ansprüche 7 bis 11 bindet, wobei der Antikörper oder das Antigen-bindende Fragment das Binden zwischen dem NOGO-Rezeptor von SEQ ID NO:2 und dem NOGO-Polypeptid von SEQ ID NO:6 hemmt und wobei der Antikörper oder das Antigen-bindende Fragment die Hemmung des axonalen Wachstums blockiert, das durch den NOGO-Rezeptor von SEQ ID NO:2 vermittelt wird.

14. Der Antikörper oder das Antigen-bindende Fragment nach Anspruch 13, wobei der Antikörper oder das Antigen-bindende Fragment ein monoklonaler Antikörper ist.

15. Zusammensetzung, umfassend das Polypeptid nach einem der Ansprüche 7 bis 12, oder einen Antikörper oder ein Antigen-bindendes Fragment nach Anspruch 13 oder Anspruch 14 und einen pharmazeutisch verträglichen Träger.

16. Die Zusammensetzung nach Anspruch 15, wobei die Zusammensetzung zur parenteralen, subkutanen, intravenösen, intramuskulären, intraperitonealen, transdermalen, bukkalen oder oralen Verabreichung oder zur Verabreichung über Mikroinfusion formuliert ist.

17. Verwendung des Polypeptids nach einem der Ansprüche 7 bis 12 oder des Antikörpers oder Antigen-bindenden Fragments nach Anspruch 13 oder Anspruch 14 zur Herstellung eines Medikaments zur Beschleunigung des axonalen Wachstums.

18. Verwendung des Polypeptids nach einem der Ansprüche 7 bis 12 oder des Antikörpers oder Antigen-bindenden Fragments nach Anspruch 13 oder Anspruch 14 zur Herstellung eines Medikaments zur Beschleunigung der axonalen Regenerierung.

19. Verwendung des Nukleinsäuremoleküls nach Anspruch 1 oder Anspruch 2, des Polypeptids nach einem der Ansprüche 7 bis 12 oder des Antikörpers oder Antigen-bindenden Fragments nach Anspruch 13 oder Anspruch 14 zur Herstellung eines Medikaments zur Behandlung einer Zentralnervensystem(ZNS)-Erkrankung, -Störung oder -Verletzung in einem Säuger.

20. Die Verwendung nach Anspruch 19, wobei die ZNS-Erkrankung, -Störung oder -Verletzung aus der Gruppe ausgewählt ist, bestehend aus kranialem oder zerebralem Trauma, Rückenmarksverletzung, Schlaganfall oder einer demyelinierenden Erkrankung.

21. Die Verwendung nach Anspruch 20, wobei die demyelinierende Erkrankung aus der Gruppe ausgewählt ist, bestehend aus multipler Sklerose, monophasischer Demyelinierung, Enzephalomyelitits, multifokaler Leukoenzephalopathie, Panenzephalitis, Marchiafava-Bignami-Krankheit, pontiner Myelinolyse, Adrenoleukodystrophie, Pelizaeus-Merzbacher-Krankheit, schwammförmiger Degenerierung, Alexander-Krankheit, Cananvan-Krankheit, metachromatischer Leukodystrophie und Krabbe-Krankheit.

22. In-vitro-Verfahren zur Beschleunigung des axonalen Wachstums, umfassend das Kontaktieren eines Neurons mit dem Polypeptid nach einem der Ansprüche 7 bis 12 oder dem Antikörper oder Antigen-bindenden Fragment nach Anspruch 13 oder Anspruch 14.

23. In-vitro-Verfahren zur Beschleunigung der axonalen Regenerierung, umfassend das Kontaktieren eines Neurons mit dem Polypeptid nach einem der Ansprüche 7 bis 12 oder dem Antikörper oder Antigen-bindenden Fragment nach Anspruch 13 oder Anspruch 14.

## Revendications

1. Molécule d'acide nucléique isolée choisie dans le groupe constitué par :
(a) une molécule d'acide nucléique isolée qui code pour un fragment de SEQ ID NO : 2 ;
(b) une molécule d'acide nucléique isolée qui s'hybride à une molécule d'acide nucléique comprenant le complémentaire de SEQ ID NO : 1 dans des conditions de stringence élevée ; et
(c) une molécule d'acide nucléique isolée présentant au moins soixante-quinze pour cent d'homologie de séquence avec SEQ ID NO : 1 ;
où ladite molécule d'acide nucléique isolée code pour un polypeptide qui bloque l'inhibition de la croissance axonale qui est médiée par le récepteur NOGO de SEQ ID NO : 2.

2. Molécule d'acide nucléique isolée selon la revendication 1, où ladite molécule d'acide nucléique est liée de manière fonctionnelle à un ou plusieurs éléments de contrôle de l'expression.

3. Vecteur comprenant la molécule d'acide nucléique isolée selon la revendication 1 ou la revendication 2.

4. Cellule hôte transformée pour contenir la molécule d'acide nucléique isolée selon la revendication 1 ou la revendication 2, ou cellule hôte contenant le vecteur selon la revendication 3.

5. Cellule hôte selon la revendication 4, où ladite cellule hôte est une cellule eucaryote.

6. Procédé de production d'un polypeptide comprenant l'étape consistant à cultiver la cellule hôte selon la revendication 4 ou la revendication 5 dans des conditions dans lesquelles le polypeptide codé par ladite molécule d'acide nucléique est exprimé.

7. Polypeptide isolé produit par le procédé selon la revendication 6.

8. Polypeptide isolé choisi dans le groupe constitué par :
(a) un polypeptide isolé comprenant un fragment d'au moins 30 acides aminés de SEQ ID NO : 2 ; et
(b) un polypeptide isolé présentant au moins soixante-quinze pour cent d'homologie de séquence d'acides aminés avec SEQ ID NO : 2 ;
où ledit polypeptide bloque l'inhibition de la croissance axonale qui est médiée par le récepteur NOGO de SEQ ID NO : 2.

9. Polypeptide isolé selon la revendication 8, où ledit polypeptide est un polypeptide isolé présentant au moins quatre-vingts pour cent d'identité de séquence d'acides aminés avec SEQ ID : 2.

10. Polypeptide isolé selon la revendication 8, où ledit polypeptide est un polypeptide isolé présentant au moins quatre-vingt-dix pour cent d'identité de séquence d'acides aminés avec SEQ ID : 2.

11. Polypeptide isolé selon la revendication 8, où ledit polypeptide est un polypeptide isolé présentant au moins quatre-vingt-quinze pour cent d'identité de séquence d'acides aminés avec SEQ ID : 2.

12. Polypeptide chimérique comprenant le polypeptide selon l'une quelconque des revendications 7 à 11, où ledit polypeptide bloque l'inhibition de la croissance axonale qui est médiée par le récepteur NOGO de SEQ ID NO : 2.

13. Anticorps ou fragment de liaison à l'antigène de celui-ci qui se lie spécifiquement à un polypeptide selon l'une quelconque des revendications 7 à 11, où ledit anticorps
ou fragment de liaison à l'antigène inhibe la liaison entre le récepteur NOGO de SEQ ID NO : 2 et le polypeptide NOGO de SEQ ID NO : 6 et où ledit anticorps ou fragment de liaison à l'antigène bloque l'inhibition de la croissance axonale qui est médiée par le récepteur NOGO de SEQ ID NO : 2.

14. Anticorps ou fragment de liaison à l'antigène selon la revendication 13, où ledit anticorps ou fragment de liaison de l'antigène est un anticorps monoclonal.

15. Composition comprenant le polypeptide selon l'une quelconque des revendications 7 à 12, ou un anticorps ou un fragment de liaison à l'antigène selon la revendication 13 ou la revendication 14, et un support pharmaceutiquement acceptable.

16. Composition selon la revendication 15, où la composition est formulée pour une administration parentérale, sous-cutanée, intraveineuse, intramusculaire, intrapéritonéale, transdermique, buccale ou orale, ou pour une administration par micro-perfusion.

17. Utilisation du polypeptide selon l'une quelconque des revendications 7 à 12, ou de l'anticorps ou du fragment de liaison à l'antigène selon la revendication 13 ou la revendication 14, pour la préparation d'un médicament destiné à favoriser la croissance axonale.

18. Utilisation du polypeptide selon l'une quelconque des revendications 7 à 12, ou de l'anticorps ou du fragment de liaison à l'antigène selon la revendication 13 ou la revendication 14, pour la préparation d'un médicament destiné à favoriser la régénérescence axonale.

19. Utilisation de la molécule d'acide nucléique selon la revendication 1 ou la revendication 2, du polypeptide selon l'une quelconque des revendications 7 à 12, ou de l'anticorps ou du fragment de liaison à l'antigène selon la revendication 13 ou la revendication 14, pour la préparation d'un médicament destiné au traitement d'une maladie, d'un trouble ou d'une lésion du système nerveux central (SNC), chez un mammifère.

20. Utilisation selon la revendication 19, où la maladie, le trouble ou la lésion du SNC est choisi(e) dans le groupe constitué par un traumatisme crânien ou cérébral, une lésion de la moelle épinière, un accident vasculaire cérébral ou une maladie démyélinisante.

21. Utilisation selon la revendication 20, où ladite maladie démyélinisante est choisie dans le groupe constitué par la sclérose en plaques, la démyélinisation monophasique, l'encéphalomyélite, la leucoencéphalopathie multifocale, la panencéphalite, la maladie de Marchiafava-Bignami, la myélinolyse pontine, l'adrénoleucodystrophie, la maladie de Pelizaeus-Merzbacher, la dégénérescence spongieuse, la maladie d'Alexander, la maladie de Canavan, la leucodystrophie métachromatique et la maladie de Krabbe.

22. Procédé *in vitro* favorisant la croissance axonale comprenant la mise en contact d'un neurone avec le polypeptide selon l'une quelconque des revendications 7 à 12 ou l'anticorps ou le fragment de liaison à l'antigène selon la revendication 13 ou la revendication 14.

23. Procédé *in vitro* favorisant la régénérescence axonale comprenant la mise en contact d'un neurone avec le polypeptide selon l'une quelconque des revendications 7 à 12 ou l'anticorps ou le fragment de liaison à l'antigène selon la revendication 13 ou la revendication 14.
